(19) Europäisches Patentamt / European Patent Office / Office européen des brevets

(11) **EP 3 422 431 B1**

(12) **EUROPEAN PATENT SPECIFICATION**


(45) Date of publication and mention of the grant of the patent:
**03.11.2021 Bulletin 2021/44**

(21) Application number: **17756627.0**

(22) Date of filing: **24.02.2017**

(51) Int Cl.:
***H01L 51/50*** *(2006.01)* ***C09K 11/06*** *(2006.01)*
***C07C 13/62*** *(2006.01)* ***C07D 403/00*** *(2006.01)*
***C07D 251/24*** *(2006.01)* ***C07C 255/50*** *(2006.01)*
***C07C 255/52*** *(2006.01)* ***C07D 209/70*** *(2006.01)*
***C07D 209/86*** *(2006.01)* ***C07D 209/94*** *(2006.01)*
***C07D 307/91*** *(2006.01)* ***C07D 333/76*** *(2006.01)*
***C07D 403/10*** *(2006.01)*

(86) International application number:
**PCT/JP2017/007024**

(87) International publication number:
**WO 2017/146192 (31.08.2017 Gazette 2017/35)**


(54) **ORGANIC ELECTROLUMINESCENT ELEMENT AND ELECTRONIC DEVICE**

ORGANISCHES ELEKTROLUMINESZENTES ELEMENT UND ELEKTRONISCHE VORRICHTUNG

ÉLÉMENT ÉLECTROLUMINESCENT ORGANIQUE ET DISPOSITIF ÉLECTRONIQUE


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.02.2016 JP 2016033672**
**03.10.2016 JP 2016196045**
**09.11.2016 JP 2016219254**

(43) Date of publication of application:
**02.01.2019 Bulletin 2019/01**



(73) Proprietor: **Idemitsu Kosan Co., Ltd**
**Tokyo 100-8321 (JP)**

(72) Inventors:
- **SAITO, Masatoshi**
**Sodegaura-shi**
**Chiba 299-0293 (JP)**
- **YOSHIDA, Kei**
**Sodegaura-shi**
**Chiba 299-0293 (JP)**
- **KAWAMURA, Yuichiro**
**Sodegaura-shi**
**Chiba 299-0293 (JP)**
- **OGIWARA, Toshinari**
**Sodegaura-shi**
**Chiba 299-0293 (JP)**
- **YOSHIZAKI, Kei**
**Sodegaura-shi**
**Chiba 299-0293 (JP)**

(74) Representative: **Gille Hrabal Partnerschaftsgesellschaft mbB**
**Patentanwälte**
**Brucknerstraße 20**
**40593 Düsseldorf (DE)**



(56) References cited:
**EP-A1- 2 530 071** **EP-A1- 3 276 697**
**WO-A1-2014/094963** **WO-A1-2015/022988**
**WO-A1-2016/158540** **JP-A- 2002 008 867**
**JP-A- 2008 530 063** **JP-A- 2011 231 086**
**JP-A- 2013 503 152** **JP-A- 2014 232 861**
**JP-A- 2015 144 224** **JP-A- 2015 173 263**
**JP-A- 2016 006 033** **US-A1- 2013 264 550**

- **HAJIME NAKANOTANI ET AL: "High-efficiency organic light-emitting diodes with fluorescent emitters", NATURE COMMUNICATIONS, vol. 5, no. 1, 30 May 2014 (2014-05-30), XP055560358, DOI: 10.1038/ncomms5016**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).


EP 3 422 431 B1

**Description**

TECHNICAL FIELD

The present invention relates to an organic electroluminescence device and an electronic device.

BACKGROUND ART

When a voltage is applied to an organic electroluminescence device (hereinafter, occasionally referred to as "organic EL device"), holes and electrons are injected into an emitting layer respectively from an anode and a cathode. The injected holes and electrons are recombined to generate excitons in the emitting layer. According to the electron spin statistics theory, singlet excitons are generated at a ratio of 25% and triplet excitons are generated at a ratio of 75%.

A fluorescent organic EL device, which uses emission caused by singlet excitons, has been applied to a full-color display of a mobile phone, TV and the like, but is inferred to exhibit an internal quantum efficiency of 25% at a maximum. A fluorescent EL device is required to use triplet excitons in addition to singlet excitons to promote a further efficient emission from the organic EL device.

In view of the above, a highly efficient fluorescent organic EL device using delayed fluorescence has been studied.

For instance, a thermally activated delayed fluorescence (TADF) mechanism has been studied. The TADF mechanism uses such a phenomenon that inverse intersystem crossing from triplet excitons to singlet excitons thermally occurs when a material having a small energy gap (ΔST) between singlet energy level and triplet energy level is used. Delayed fluorescence (thermally activated delayed fluorescence) is explained in "Yuki Hando-tai no Debaisu Bussei (Device Physics of Organic Semiconductors)" (edited by ADACHI, Chihaya, published by Kodansha, issued on April 1, 2012, on pages 261-268).An organic EL device using the TADF mechanism is disclosed in, for instance, non-Patent Literature 1.

An organic EL device disclosed in Non-Patent Literature 1 includes an emitting layer containing a TADF compound as an assist dopant, a perylene derivative (TBPe; 2,5,8,11-tetra-tert-butylperylene) as a luminescent material, and DPEPO (bis-(2-(diphenylphosphino)phenyl)ether oxide) as a host material. This emitting layer provides a blue emission.

CITATION LIST

NON-PATENT LITERATURE(S)

Non-Patent Literature 1

Hajime Nakanotani et al, "High-efficiency organic light-emitting diodes with fluorescent emitters", NATURE COMMUNICATIONS, 5, 4016, 2014 (2014-05-30), XP055560358, (DOI: 10.1038/ncomms5016) discloses inter alia a TADF-based blue OLED using 2,5,8,11-tetra-tert-butylperylene (TBPe) as fluorescent emitter dopant.

PATENT LITERATURE(S)

US 2013/264550 A1 discloses inter alia OLEDs using the compound

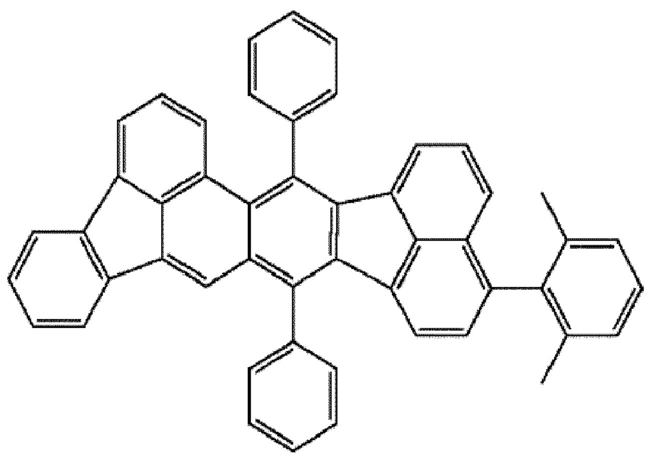

as an emitting dopant.

EP 2530071 A1 discloses fused polycyclic compounds suitable for use mainly as a component for a blue-light-emitting device.

## SUMMARY OF THE INVENTION

### PROBLEMS TO BE SOLVED BY THE INVENTION

The organic electroluminescence device disclosed in Non-Patent Literature 1 provides a blue emission using the TADF compound as the host material and the perylene derivative (the compound TBPe) as the luminescent material, but has an insufficient luminous efficiency. Accordingly, an organic electroluminescence device configured to emit light in a blue wavelength region at a high efficiency has been desired.

An object of the invention is to provide an organic electroluminescence device configured to emit light in a blue wavelength region at a high efficiency. Another object of the invention is to provide an electronic device including the organic electroluminescence device.

### MEANS FOR SOLVING THE PROBLEMS

According to an aspect of the invention, an organic electroluminescence device is provided as defined in any of the claims 1 to 14, including an anode, an emitting layer and a cathode, in which the emitting layer contains a first compound and a second compound, the first compound is a delayed fluorescent compound, wherein

the first compound is a compound represented by a formula,

[Formula 26]

$$Cz-(L)_c-Az$$

where: Az is a cyclic structure selected from the group consisting of a substituted or unsubstituted pyridine ring, a substituted or unsubstituted pyrimidine ring, a substituted or unsubstituted triazine ring, and a substituted or unsubstituted pyrazine ring;

c is an integer of 0 to 5;

L is a linking group selected from the group consisting of a substituted or unsubstituted aromatic hydrocarbon group having 6 to 30 ring carbon atoms, and a substituted or unsubstituted heterocyclic group having 5 to 30 ring atoms;

when c is 0, Cz is bonded to Az by a single bond;

when c is an integer of 2 to 5, a plurality of L are bonded to each other to form a ring, or are not bonded;

a plurality of L are mutually the same or different; and

Cz is represented by a formula (27),

[Formula 27]

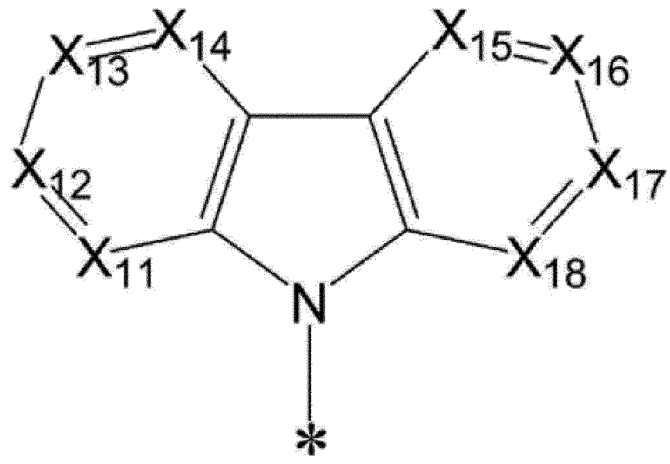

where:

$X_{11}$ to $X_{18}$ are each independently a nitrogen atom or C-Rx;
Rx is each independently a hydrogen atom or a substituent;
Rx serving as the substituent is a group selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group;
a plurality of Rx are mutually the same or different;
when a plurality of ones of $X_{11}$ to $X_{18}$ are C-Rx and Rx are substituents, Rx are not bonded to each other to form a ring; and
* represents a bonding position to a carbon atom in a structure of the linking group represented by L, or a bonding position to a carbon atom in the cyclic structure represented by Az,
the second compound is a fluorescent compound, wherein the second compound is a hydrocarbon compound consisting of a carbon atom(s) and a hydrogen atom(s) having a mother skeleton including fused rings, the number of the fused rings ranging from 5 to 15, and extending while being bent in one direction, said fused rings comprise a five-membered ring,
an emission peak wavelength of a solution of the second compound in toluene is in a range from 430 nm to 480 nm, a molar absorbance coefficient of the second compound at an absorption peak closest to a long-wavelength side is in a range from 40000 L/(mol·cm) to 1000000 L/(mol·cm), and a Stokes shift of the second compound is in a range from 1 nm to 20 nm.

According to another aspect of the invention, an electronic device including the above organic electroluminescence device is provided as defined in claim 15.

According to the above aspects of the invention, an organic electroluminescence device configured to emit light in a blue wavelength region at a high efficiency and an electronic device including the organic electroluminescence device can be provided.

BRIEF DESCRIPTION OF DRAWINGS

Fig. 1 schematically shows an exemplary arrangement of an organic electroluminescence device according to an exemplary embodiment.
Fig. 2 schematically shows a device for measuring transient PL.
Fig. 3 shows examples of a transient PL decay curve.
Fig. 4 shows a relationship between energy levels of a first compound and a second compound and an energy

transfer between the first compound and the second compound in an emitting layer.
Fig. 5 shows a relationship between energy levels of the first compound, the second compound and a third compound and an energy transfer between the first, second and third compounds in the emitting layer.

## DESCRIPTION OF EMBODIMENTS

### First Exemplary Embodiment

### Arrangement(s) of Organic EL Device

An organic EL device in a first exemplary embodiment includes a pair of electrodes and an organic layer between the pair of electrodes. The organic layer includes at least one layer formed of an organic compound. Alternatively, the organic layer may include a plurality of layers formed of the organic compound. The organic layer may further contain an inorganic compound. In the organic EL device in the exemplary embodiment, at least one layer of the organic layer(s) is an emitting layer. Specifically, for instance, the organic layer may consist of a single emitting layer, or alternatively, may further include a layer usable in a typical organic EL device. The layer usable in the organic EL device is not limited to, but, for instance, at least one layer selected from the group consisting of a hole injecting layer, a hole transporting layer, an electron injecting layer, an electron transporting layer and a blocking layer.
Typical device arrangements of the organic EL device include the following arrangements (a) to (f) and the like:

(a) anode/ emitting layer/ cathode;
(b) anode / hole injecting·transporting layer / emitting layer / cathode;
(c) anode / emitting layer / electron injecting·transporting layer / cathode;
(d) anode / hole injecting· transporting layer / emitting layer / electron injecting· transporting layer / cathode;
(e) anode / hole injecting· transporting layer / emitting layer / blocking layer / electron injecting· transporting layer / cathode; and
(f) anode / hole injecting· transporting layer / blocking layer / emitting layer / electron injecting· transporting layer / cathode.

The arrangement (d) is suitably used among the above arrangements. However, the arrangement of the invention is not limited to the above arrangements. The "emitting layer" refers to an organic layer having an emitting function. The term "hole injecting· transporting layer" means at least one of a hole injecting layer or a hole transporting layer. The term "electron injecting· transporting layer" means at least one of an electron injecting layer or an electron transporting layer. When the organic EL device includes the hole injecting layer and the hole transporting layer, the hole injecting layer is suitably provided between the hole transporting layer and the anode. When the organic EL device includes the electron injecting layer and the electron transporting layer, the electron injecting layer is suitably provided between the electron transporting layer and the cathode. The hole injecting layer, the hole transporting layer, the electron transporting layer and the electron injecting layer may each consist of a single layer or a plurality of layers.
Fig. 1 schematically shows an arrangement of an exemplary organic EL device according to the exemplary embodiment.
An organic EL device 1 includes a light-transmissive substrate 2, an anode 3, a cathode 4, and an organic layer 10 provided between the anode 3 and the cathode 4. The organic layer 10 includes: a hole injecting layer 6, a hole transporting layer 7, an emitting layer 5, an electron transporting layer 8, and an electron injecting layer 9. The organic layer 10 includes the hole injecting layer 6, the hole transporting layer 7, the emitting layer 5, the electron transporting layer 8, and the electron injecting layer 9 which are laminated on the anode 3 in this order.

### Emitting Layer

The emitting layer 5 of the organic EL device 1 contains a first compound and a second compound as defined in the claims. The emitting layer 5 may contain a metal complex, but preferably contains no metal complex. The emitting layer 5 preferably does not contain a phosphorescent metal complex.
The first compound is also preferably a host material (occasionally referred to as a matrix material). The second compound is also preferably a dopant material (occasionally referred to as a guest material, emitter, or luminescent material).

### Second Compound

The second compound is a fluorescent compound. Further, the second compound in the exemplary embodiment

is a fluorescent compound having characteristics of a predetermined emission peak wavelength, a predetermined molar absorbance coefficient, and a predetermined Stokes shift in combination.

Emission Peak Wavelength

A solution of the second compound in toluene has an emission peak wavelength in a range from 430 nm to 480 nm.

The solution of the second compound in toluene preferably has the emission peak wavelength in a range from 435 nm to 465 nm. Herein, the emission peak wavelength of the solution of a measurement target compound in toluene (also referred to as a toluene solution of a measurement target compound) means a peak wavelength of an emission spectrum having the maximum luminous intensity among emission spectra measured using the toluene solution in which the measurement compound is dissolved at a concentration of 5 μmol/L.

The second compound preferably emits a blue fluorescence.

The second compound is preferably a material with a high emission quantum efficiency.

Molar Absorbance Coefficient

A molar absorbance coefficient of the second compound at an absorption peak closest to the long-wavelength side is in a range from 40000 L/(mol·cm) to 1000000 L/(mol·cm), preferably from 40000 L/(mol·cm) to 250000 L/(mol·cm), more preferably from 40000 L/(mol·cm) to 150000 L/(mol·cm).

Herein, the absorption peak closest to the long-wavelength side means an absorption peak appearing closest to the long-wavelength side among absorption peaks appearing in a range from 350 nm 500 nm and each having an absorption intensity of at least one-tenth of the maximum absorption peak.

Stokes Shift

The second compound has a Stokes shift in a range from 1 nm to 20 nm.

The Stokes shift of the second compound is preferably in a range from 4 nm to 18 nm, more preferably in a range from 5 nm to 17 nm. Herein, the Stokes shift (ss) is calculated by subtracting an absorption peak wavelength, where the molar absorbance coefficient of the absorption spectrum is obtained, from the emission peak wavelength of the emission spectrum.

In the exemplary embodiment, as the second compound has the smaller Stokes and the larger molar absorbance coefficient, energy loss at energy transfer from the first compound to the second compound is reducible. As a result, a luminous efficiency of the organic EL device is expected to be improvable.

The second compound is a fluorescent compound having the characteristics of the above-described emission peak wavelength, molar absorbance coefficient, and Stokes shift in combination.

Since fluorescence of a compound is substantially determined depending on a mother skeleton of the compound, selection of the mother skeleton is important. For the selection of the mother skeleton, for instance, excited singlet energy, transition probability, luminous efficiency, effective sectional area and the like need to be considered. For instance, the second compound of the exemplary embodiment requires a mother skeleton having contradictory characteristics that the mother skeleton has an effective sectional area whereas the second compound exhibits a large excited singlet energy. The second compound is a compound having a mother skeleton including fused rings (the number of the fused rings ranging from 5 to 15) and extending while being bent in one direction, said fused rings comprise a five-membered ring. The number of the fused rings is more preferably from 7 to 13. The rings forming the fused rings in the second compound are exemplified by a five-membered ring and a six-membered ring.

For instance, the fused rings shown below include six six-membered rings and two five-membered rings, in which the number of the fused rings is eight.

[Formula 1]

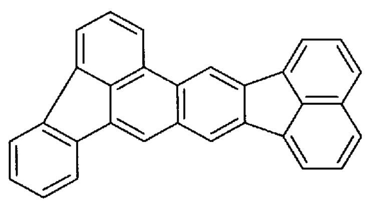

For instance, a porphyrin compound having 4 to 16 rings has a molar absorbance coefficient in a range from 200000 L/(mol·cm) to 500000 L/(mol·cm) (Bulletin of the Chemical Society of Japan, Vol.1978 (1978), No.2, pages 212 to 216), but has a fused ring structure to provide a small excited singlet energy. Accordingly, the porphyrin compound emits light at the emission peak wavelength of 480 nm or more, in other words, does not emit a blue light. Consequently, the porphyrin compound is not usable as the second compound. It is only required to select, as a substituent for the mother skeleton, a substituent suitable for adjusting characteristics of the compound to characteristics suitable for a practical use.

The second compound is a hydrocarbon compound consisting of a carbon atom(s) and a hydrogen atom(s) as defined in the claims.

It is also preferable that the second compound in the exemplary embodiment is a substituted or unsubstituted aromatic hydrocarbon compound.

It is also preferable that the second compound in the exemplary embodiment is a substituted or unsubstituted fused aromatic hydrocarbon compound.

It is also preferable that the second compound in the exemplary embodiment is a substituted or unsubstituted aromatic compound including 5 to 15 fused rings.

It is also preferable that the second compound in the exemplary embodiment includes 7 to 13 fused rings.

In the exemplary embodiment, the emitting layer may include a plurality of kinds of the second compounds.

Examples of the second compound of the exemplary embodiment are shown below. The second compound according to the invention is not limited to these specific examples.

[Formula 2]

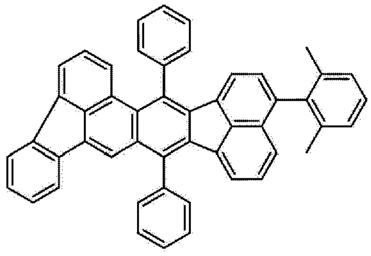
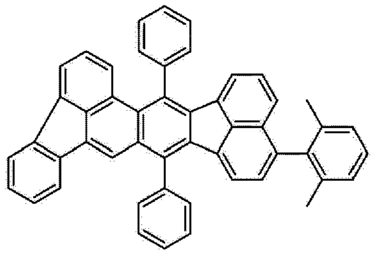

[Formula 3]

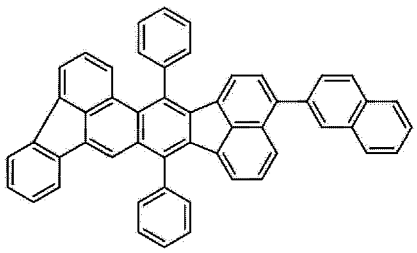
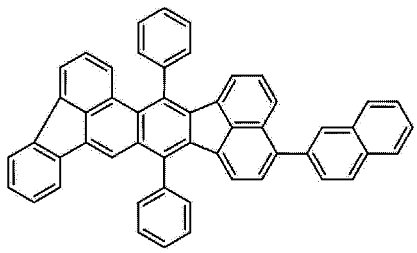

[Formula 4]

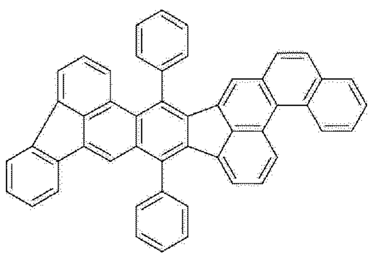
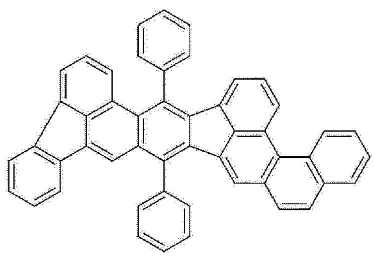

[Formula 7]

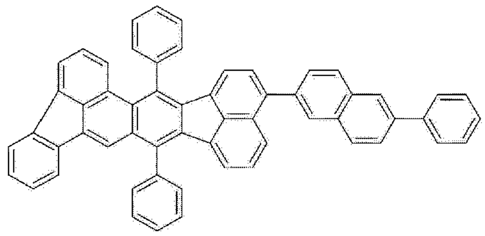

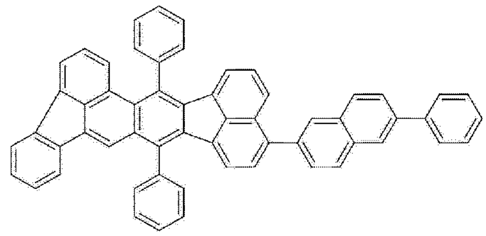

[Formula 9]

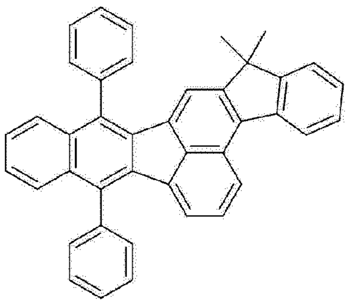

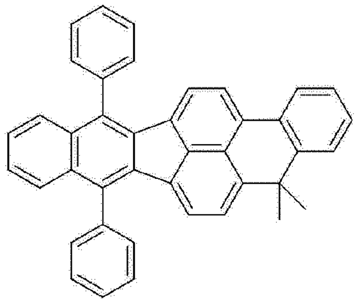

First Compound

The first compound is a delayed fluorescent compound as defined in the claims.

The first compound is a compound represented by a formula (11) below.

[Formula 26]

$$Cz-(L)_c-Az \quad (11)$$

In the formula (11), Az is a cyclic structure selected from the group consisting of a substituted or unsubstituted pyridine ring, a substituted or unsubstituted pyrimidine ring, a substituted or unsubstituted triazine ring, and a substituted or unsubstituted pyrazine ring. c is an integer of 0 to 5. L is a linking group selected from the group consisting of a substituted or unsubstituted aromatic hydrocarbon group having 6 to 30 ring carbon atoms, and a substituted or unsubstituted heterocyclic group having 5 to 30 ring atoms. When c is 0, Cz is bonded to Az by a single bond. When c is an integer of 2 to 5, a plurality of L are bonded to each other to form a ring, or are not bonded. The plurality of L are mutually the same or different. Cz is represented by a formula (12) below.

[Formula 27]

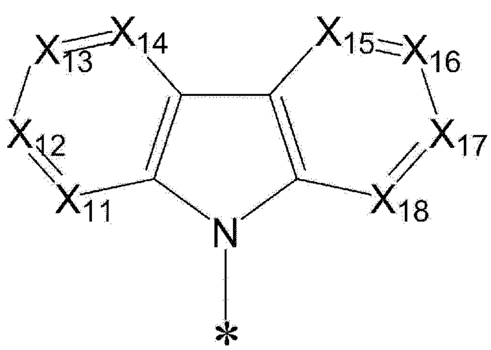

(12)

In the formula (12): $X_{11}$ to $X_{18}$ are each independently a nitrogen atom or C-Rx. Rx is each independently a hydrogen atom or a substituent. Rx serving as the substituent is a group selected from the group consisting of a substituted

or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group. A plurality of Rx are mutually the same or different. When a plurality of ones of $X_{11}$ to $X_{18}$ are C-Rx and Rx are substituents, Rx are bonded to each other to form a ring, or are not bonded. * represents a bonding position to a carbon atom in a structure of the linking group represented by L, or a bonding position to a carbon atom in the cyclic structure represented by Az.

$X_{11}$ to $X_{18}$ are also preferably C-Rx.

In the formula (11), c is preferably 0 or 1.

The compound represented by the formula (11) is also preferably a compound represented by a formula (11A) below.

[Formula 28]

Cz —L— Az (11A)

Az, Cz and L in the formula (11A) respectively represent the same as Az, Cz and L in the formula (11).

L in the formula (11A) is preferably a linking group selected from the group consisting of a substituted or unsubstituted aromatic hydrocarbon group having 6 to 30 ring carbon atoms.

The compound represented by the formula (11A) is also preferably a compound represented by a formula (11B) below.

[Formula 29]

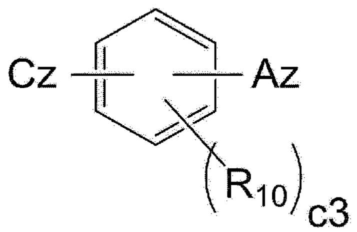

(11B)

In the formula (11A): Az and Cz respectively represent the same as Az and Cz in the formula (11); c3 is 4; $R_{10}$ is a hydrogen atom or a substituent; $R_{10}$ as the substituent is a substituent selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group; and a plurality of $R_{10}$ are mutually the same or different.

The compound represented by the formula (11A) is also preferably a compound represented by a formula (11C) below.

[Formula 30]

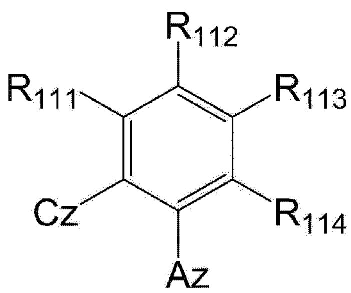

(11C)

In the formula (11C): Az and Cz respectively represents the same as Az and Cz in the formula (11); $R_{111}$ to $R_{114}$ are each independently a hydrogen atom or a substituent; and $R_{111}$ to $R_{114}$ as the substituent are each independently a substituent selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group.

Cz is also preferably represented by a formula (12a), (12b) or (12c).

[Formula 31]

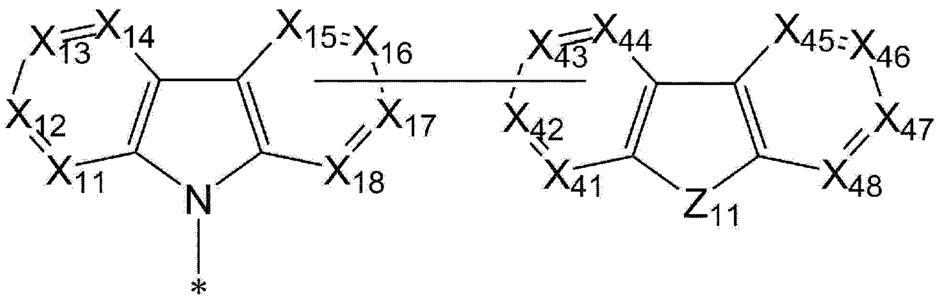

(12a)

[Formula 32]

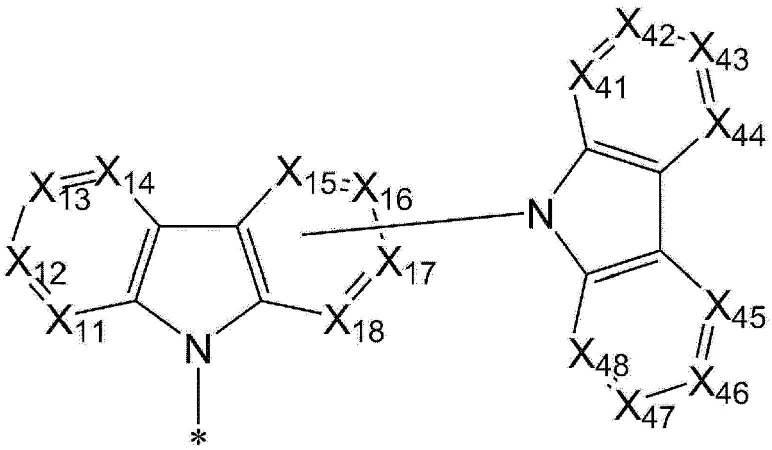

(12b)

[Formula 33]

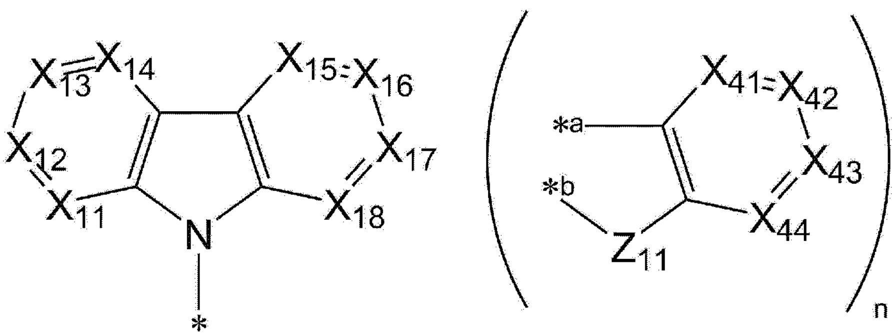

(12c)

In the formulae (12a), (12b) and (12c), $X_{11}$ to $X_{18}$ and $X_{41}$ to $X_{48}$ are each independently a nitrogen atom or C-Rx.

In the formula (12a), at least one of $X_{15}$ to $X_{18}$ is a carbon atom bonded to one of $X_{41}$ to $X_{44}$, and at least one

of $X_{41}$ to $X_{44}$ is a carbon atom bonded to one of $X_{15}$ to $X_{18}$.

In the formula (12b), at least one of $X_{15}$ to $X_{18}$ is a carbon atom bonded to a nitrogen atom in a five-membered ring of a nitrogen-containing fused ring including $X_{41}$ to $X_{48}$.

In the formula (12c), *a and *b each represent a bonding position to one of X11 to X18. At least one of $X_{15}$ to $X_{18}$ is the bonding position represented by *a. At least one of $X_{15}$ to $X_{18}$ is the bonding position represented by *b.

n is an integer of 1 to 4.

Rx is each independently a hydrogen atom or a substituent; and Rx as the substituent are each independently a substituent selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group.

A plurality of Rx are mutually the same or different.

When a plurality of ones of $X_{11}$ to $X_{18}$ are C-Rx and Rx are substituents, Rx are bonded to each other to form a ring, or are not bonded.

When a plurality of ones of $X_{41}$ to $X_{48}$ are C-Rx and Rx are substituents, Rx are bonded to each other to form a ring, or are not bonded.

$Z_{11}$ is any one selected from the group consisting of an oxygen atom, a sulfur atom, $NR_{40}$ and $C(R_{41})_2$.

$R_{40}$ and $R_{41}$ are each independently a hydrogen atom or a substituent; and $R_{40}$ and $R_{41}$ as the substituent are each independently a substituent selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group.

A plurality of $R_{40}$ are mutually the same or different.

A plurality of $R_{41}$ are mutually the same or different.

When the plurality of $R_{41}$ are substituents, the plurality of $R_{41}$ are bonded to each other to form a ring, or are not bonded.

**[0072]** * represents a bonding position to a carbon atom of the cyclic structure represented by Az.

Z11 is preferably $NR_{40}$.

When $Z_{11}$ is $NR_{40}$, $R_{40}$ is preferably a substituted or unsubstituted aryl group having 6 to 30 ring carbon atoms.

$X_{41}$ to $X_{48}$ are preferably C-Rx, provided that at least one of $X_{41}$ to $X_{48}$ is a carbon atom bonded to the cyclic structure represented by the formula (12).

Cz is also preferably represented by the formula (12c) in which n is 1.

Cz is also preferably represented by a formula (12c-1) below. A group represented by the formula (12c-1) is an example of the group represented by the formula (12c), in which $X_{16}$ is the bonding position represented by *a and $X_{17}$ is the bonding position represented by *b.

[Formula 34]

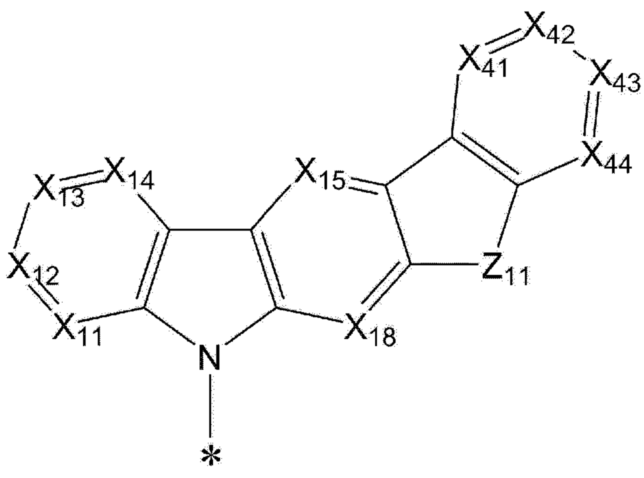

(12c-1)

In the formula (12c-1), $X_{11}$ to $X_{15}$, $X_{18}$ and $X_{41}$ to $X_{44}$ are each independently a nitrogen atom or C-Rx.

Rx is each independently a hydrogen atom or a substituent.

Rx as the substituent is a substituent selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group.

A plurality of Rx are mutually the same or different.

When a plurality of ones of $X_{11}$ to $X_{15}$ and $X_{18}$ are C-Rx and Rx are substituents, Rx are bonded to each other to form a ring, or are not bonded.

When a plurality of ones of $X_{41}$ to $X_{44}$ are C-Rx and Rx are substituents, Rx are bonded to each other to form a ring, or are not bonded.

$Z_{11}$ is any one selected from the group consisting of an oxygen atom, a sulfur atom, $NR_{40}$ and $C(R_{41})_2$. $R_{40}$ and $R_{41}$ are each independently a hydrogen atom or a substituent; and $R_{40}$ and $R_{41}$ as the substituent are each independently a substituent selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group.

A plurality of $R_{40}$ are mutually the same or different.

A plurality of $R_{41}$ are mutually the same or different.

When the plurality of $R_{41}$ are substituents, the plurality of $R_{41}$ are bonded to each other to form a ring, or are not bonded.

**[0088]** * represents a bonding position to a carbon atom of the cyclic structure represented by Az.

When an index n in the formula (12c) is 2, Cz is exemplarily represented by a formula (12c-2) below. Specifically, when n is 2, two structures enclosed by brackets with the index n are fused to the cyclic structure represented by the formula (12).Cz represented by the formula (12c-2) is an example of the group represented by the formula (12c), in which $X_{12}$ is the bonding position represented by *b, $X_{13}$ is the bonding position represented by *a, $X_{16}$ is the bonding position represented by *a and $X_{17}$ is the bonding position represented by *b.

[Formula 35]

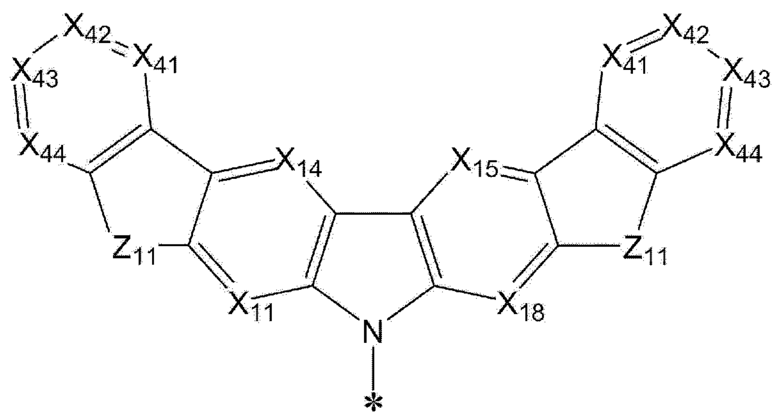

(12c-2)

In the formula (12c-2), $X_{11}$, $X_{14}$, X15, X18, X41 to X44, $Z_{11}$, and * respectively represent the same as $X_{11}$, X14, X15, X18, X41 to X44, $Z_{11}$, and * in the formula (12c-1). A plurality of $R_{41}$ are mutually the same or different. A plurality of $R_{42}$ are mutually the same or different. A plurality of $R_{43}$ are mutually the same or different. A plurality of $R_{44}$ are mutually the same or different. A plurality of $Z_{11}$ are mutually the same or different.

Az is preferably a cyclic structure selected from the group consisting of a substituted or unsubstituted pyrimidine ring and a substituted or unsubstituted triazine ring.

Az is more preferably a cyclic structure selected from the group consisting of a substituted pyrimidine ring and a substituted triazine ring, in which a substituent of each of the substituted pyrimidine ring and the substituted triazine ring is a group selected from the group consisting of a substituted or unsubstituted aryl group having 6 to 30 ring carbon atoms and a substituted or unsubstituted heteroaryl group having 5 to 30 ring atoms. Az is further preferably a cyclic

structure selected from the group consisting of a substituted pyrimidine ring and a substituted triazine ring, in which a substituent of each of the substituted pyrimidine ring and the substituted triazine ring is a substituted or unsubstituted aryl group having 6 to 30 ring carbon atoms.

When the pyrimidine ring and the triazine ring as Az have a substituted or unsubstituted aryl group as a substituent, the aryl group preferably has 6 to 20 ring carbon atoms, more preferably 6 to 14 ring carbon atoms, further preferably 6 to 12 ring carbon atoms.

When Az has a substituted or unsubstituted aryl group as the substituent, the substituent is preferably a substituent selected from the group consisting of a substituted or unsubstituted phenyl group, a substituted or unsubstituted biphenyl group, a substituted or unsubstituted naphthyl group, a substituted or unsubstituted phenanthryl group, a substituted or unsubstituted terphenyl group, and a substituted or unsubstituted fluorenyl group, more preferably a substituent selected from the group consisting of a substituted or unsubstituted phenyl group, a substituted or unsubstituted biphenyl group, and a substituted or unsubstituted naphthyl group.

When Az has a substituted or unsubstituted heteroaryl group as the substituent, the substituent is preferably a substituent selected from the group consisting of a substituted or unsubstituted carbazolyl group, a substituted or unsubstituted dibenzofuranyl group, and a substituted or unsubstituted dibenzothienyl group.

Rx is each independently a hydrogen atom or a substituent. Rx serving as the substituent are preferably each independently a substituent selected from the group consisting of a substituted or unsubstituted aryl group having 6 to 30 ring carbon atoms and a substituted or unsubstituted heteroaryl group having 5 to 30 ring atoms.

When Rx as the substituent is a substituted or unsubstituted aryl group having 6 to 30 ring carbon atoms, Rx as the substituent is preferably a substituent selected from the group consisting of a substituted or unsubstituted phenyl group, a substituted or unsubstituted biphenyl group, a substituted or unsubstituted naphthyl group, a substituted or unsubstituted phenanthryl group, a substituted or unsubstituted terphenyl group, and a substituted or unsubstituted fluorenyl group, more preferably a substituent selected from the group consisting of a substituted or unsubstituted phenyl group, a substituted or unsubstituted biphenyl group, and a substituted or unsubstituted naphthyl group.

When Rx as the substituent is a substituted or unsubstituted heteroaryl group having 5 to 30 ring atoms, Rx as the substituent is preferably a substituent selected from the group consisting of a substituted or unsubstituted carbazolyl group, a substituted or unsubstituted dibenzofuranyl group, and a substituted or unsubstituted dibenzothienyl group.

$R_{40}$ and $R_{41}$ serving as the substituent are preferably each independently a substituent selected from the group consisting of a substituted or unsubstituted aryl group having 6 to 30 ring carbon atoms, a substituted or unsubstituted heteroaryl group having 5 to 30 ring atoms, and a substituted or unsubstituted alkyl group having 1 to 30 carbon atoms.

Delayed Fluorescence

Delayed fluorescence (thermally activated delayed fluorescence) is explained in "Yuki Hando-tai no Debaisu Bussei (Device Physics of Organic Semiconductors)" (edited by ADACHI, Chihaya, published by Kodansha, on pages 261-268). This document describes that, when an energy gap $\Delta E_{13}$ between a singlet state and a triplet state of a fluorescent material can be decreased, in spite of a typical low transition probability, inverse energy transfer from the triplet state to the singlet state occurs at a high efficiency to express thermally stimulated delayed fluorescence (TADF). Further, Fig. 10.38 in this literature illustrates an occurrence mechanism of the delayed fluorescence. The first compound of the exemplary embodiment is a compound exhibiting thermally activated delayed fluorescence caused by this mechanism.

Occurrence of delayed fluorescence emission can be determined by transient PL (Photo Luminescence) measurement.

The behavior of delayed fluorescence can be analyzed based on the decay curve obtained by the transient PL measurement. The transient PL measurement is a process where a sample is irradiated with a pulse laser to be excited, and a decay behavior (transient characteristics) of PL emission after the irradiation is stopped is measured.PL emission using a TADF material is divided into an emission component from singlet excitons generated by the first PL excitation and an emission component from singlet excitons generated via triplet excitons. The lifetime of the singlet excitons generated by the initial PL excitation is in a nanosecond order and considerably short. Emission from these singlet excitons thus decays immediately after the irradiation with the pulse laser.

On the other hand, since delayed fluorescence provides emission from singlet excitons generated through long-life triplet excitons, emission is gradually reduced. There is thus a large difference in time between emission from the singlet excitons generated by the initial PL excitation and emission from the singlet excitons generated via triplet excitons. Therefore, a luminous intensity resulting from the delayed fluorescence can be obtained.

Fig. 2 schematically shows an exemplary device for measuring transient PL.

A transient PL measuring device 100 of the first exemplary embodiment includes: a pulse laser 101 capable of emitting light with a predetermined wavelength; a sample chamber 102 configured to house a measurement sample; a spectrometer 103 configured to disperse light emitted from the measurement sample; a streak camera 104 configured

to form a two-dimensional image; and a personal computer 105 configured to analyze the two-dimensional image imported thereinto. It should be noted that transient PL may be measured by a device different from one described in the first exemplary embodiment.

The sample to be housed in the sample chamber 102 is prepared by forming a thin film, which is made of a matrix material doped with a doping material at a concentration of 12 mass%, on a quartz substrate.

The thus-obtained thin film sample is housed in the sample chamber 102, and is irradiated with a pulse laser emitted from the pulse laser 101 to excite the doping material. The emitted excitation light is taken in a 90-degree direction with respect to the irradiation direction of the excitation light, and is dispersed by the spectrometer 103. A two-dimensional image of the light is formed through the streak camera 104. In the thus-obtained two-dimensional image, an ordinate axis corresponds to time, an abscissa axis corresponds to wavelength, and a bright spot corresponds to luminous intensity. The two-dimensional image is taken at a predetermined time axis, thereby obtaining an emission spectrum with an ordinate axis representing luminous intensity and an abscissa axis representing wavelength. Further, the two-dimensional image is taken at a wavelength axis, thereby obtaining a decay curve (transient PL) with an ordinate axis representing the logarithm of luminous intensity and an abscissa axis representing time.

For instance, using a reference compound H1 below as the matrix material and a reference compound D1 as the doping material, a thin film sample A was prepared as described above and the transitional PL was measured.

[Formula 46]

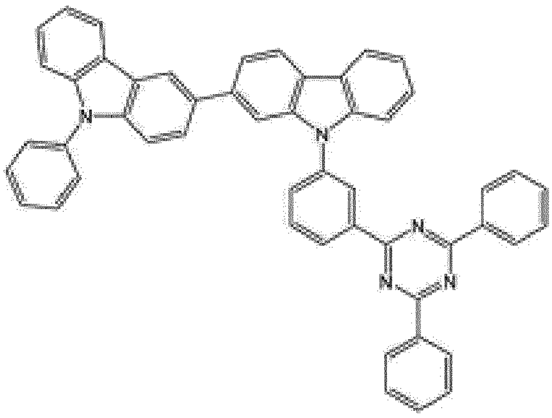

Reference Compound H1

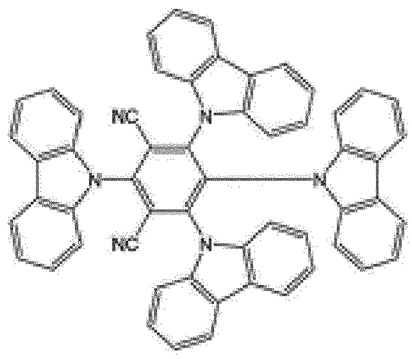

Reference Compound D1

Respective decay curves of the above thin film sample A and a thin film sample B were analyzed. The thin film sample B was prepared as described above, using a reference compound H2 below as the matrix material and the reference compound D1 as the doping material.

Fig. 3 shows a decay curve obtained from transient PL measured using each of the thin film samples A and B.

[Formula 47]

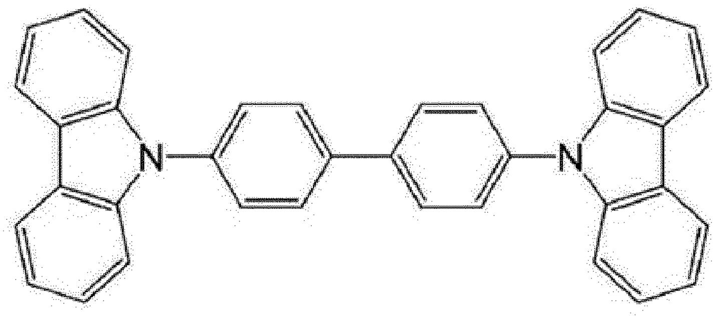

Reference Compound H2

As described above, an emission decay curve with an ordinate axis representing luminous intensity and an abscissa axis representing time can be obtained by the transient PL measurement. Based on the emission decay curve, a fluorescence intensity ratio between fluorescence emitted from a singlet state generated by photo-excitation and delayed fluorescence emitted from a singlet state generated by inverse energy transfer via a triplet state can be estimated. In a delayed fluorescent material, a ratio of the intensity of the slowly decaying delayed fluorescence to the intensity of the promptly decaying fluorescence is relatively large.

In the first exemplary embodiment, the luminescence amount of the delayed fluorescence can be obtained using the device shown in Fig. 2. Emission from the first compound includes: Prompt emission observed immediately when the excited state is achieved by exciting the first compound with a pulse beam (i.e., a beam emitted from a pulse laser) having an absorbable wavelength; and Delayed emission observed not immediately when but after the excited state is achieved. In the first exemplary embodiment, the amount of Delayed emission is preferably 5% or more relative to the amount of Prompt emission. Specifically, when the amount of Prompt emission is denoted by $X_P$ and the amount of Delayed emission is denoted by $X_D$, a value of $X_D/X_P$ is preferably 0.05 or more.

The amount of Prompt emission and the amount of Delayed emission can be obtained in the same method as a method described in "Nature 492, 234-238, 2012." The amount of Prompt emission and the amount of Delayed emission may be calculated using a device different from one described in the above Reference Literature.

For instance, a sample usable for measuring the delayed fluorescence may be prepared by co-depositing the first compound and a compound TH-2 below on a quartz substrate at a ratio of the first compound being 12 mass% to form a 100-nm-thick thin film.

[Formula 48]

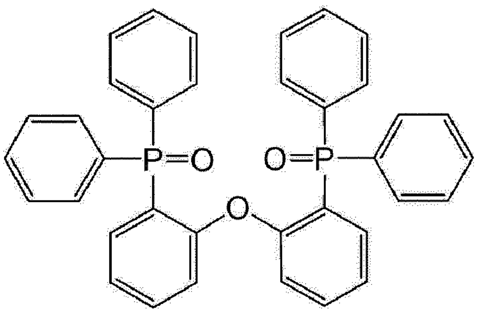

TH-2

Method of Preparing First Compound

The first compound can be exemplarily prepared by a method described in Chemical Communications p.10385-10387 (2013) and NATURE Photonics p.326-332 (2014).Moreover, the first compound can be exemplarily prepared by a method described in International Publication Nos. WO2013/180241, WO2014/092083, WO2014/104346 and the like. Furthermore, the first compound can be exemplarily prepared with use of a known alternative reaction and a starting material depending on a target object with reference to a reaction described later in Examples.

Specific examples of the first compound of the first exemplary embodiment are shown below. The first compound according to the invention is not limited to these specific examples.

[Formula 49]

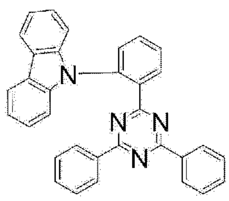
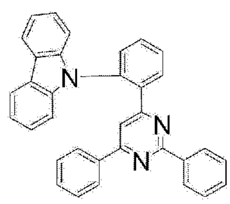
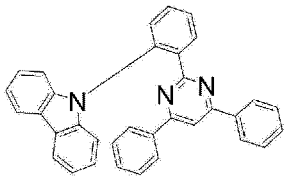
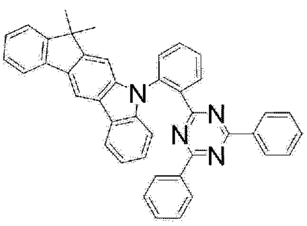
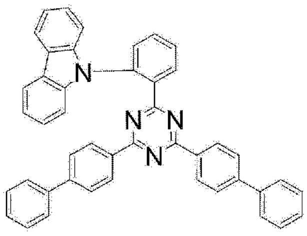
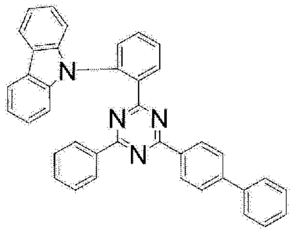
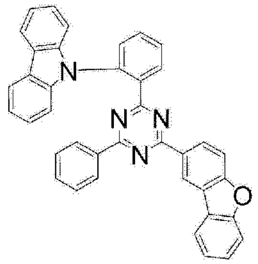

[Formula 50]

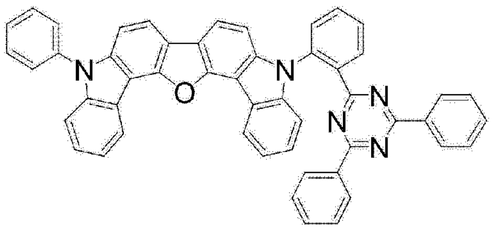
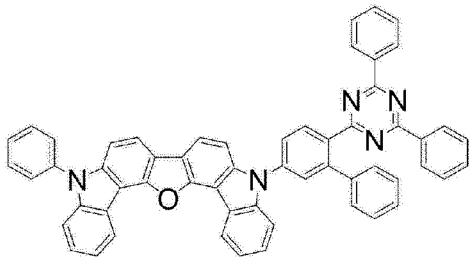
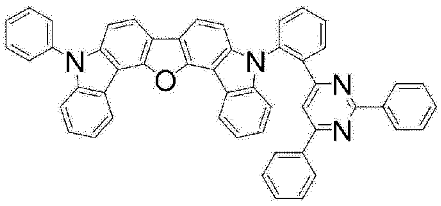
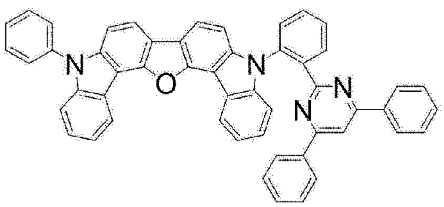

[Formula 51]

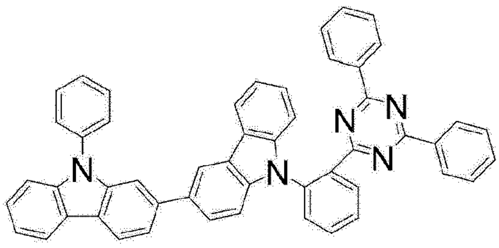

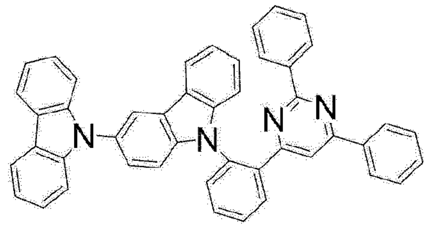

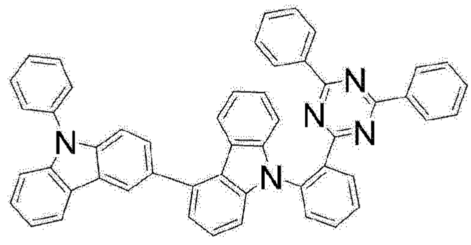

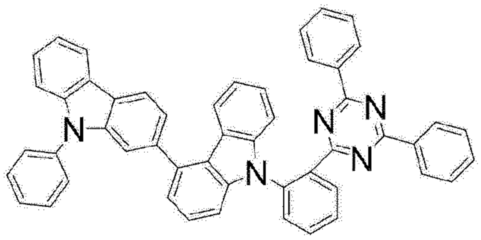

[Formula 52]

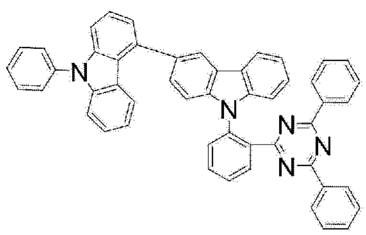

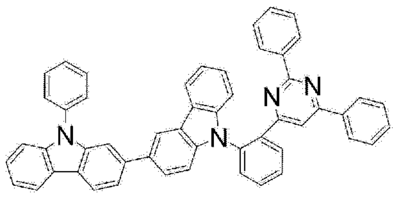

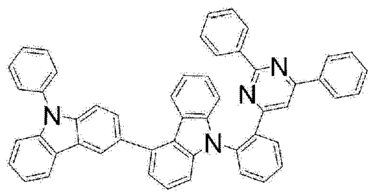

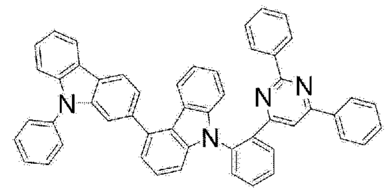

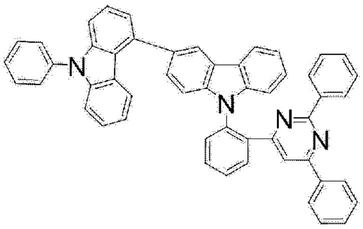

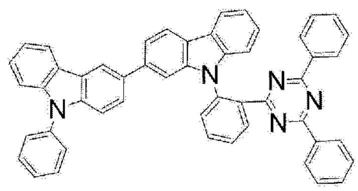

[Formula 53]

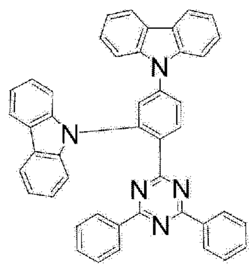 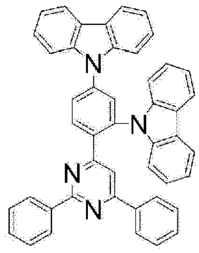 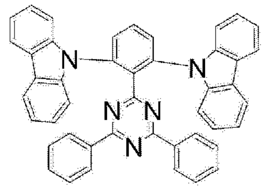 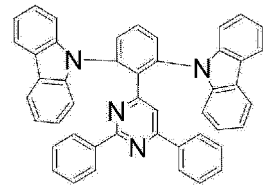

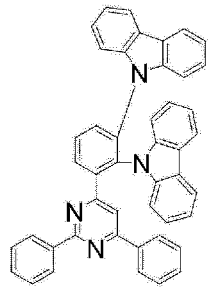 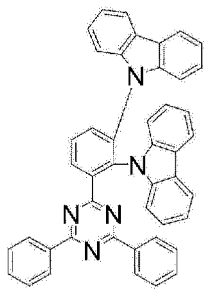 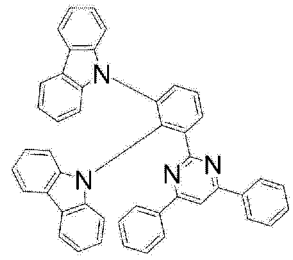 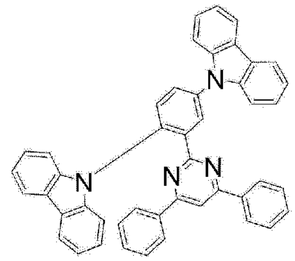

[Formula 54]

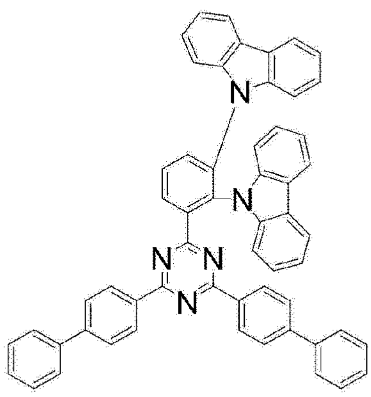 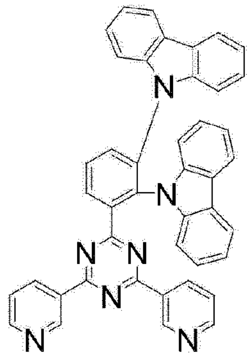 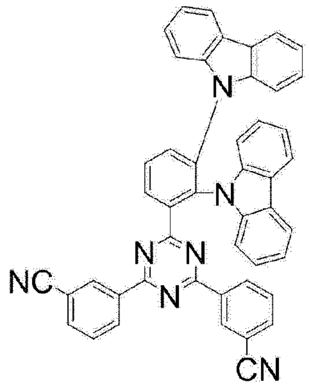

[Formula 55]

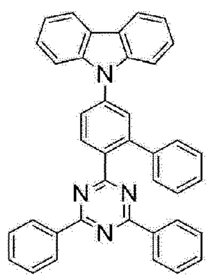 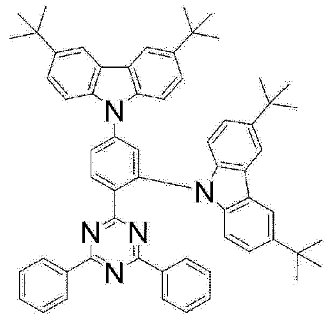 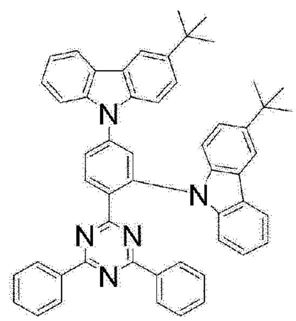

[Formula 56]

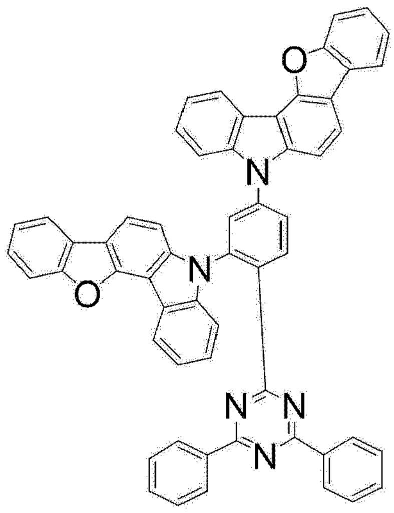

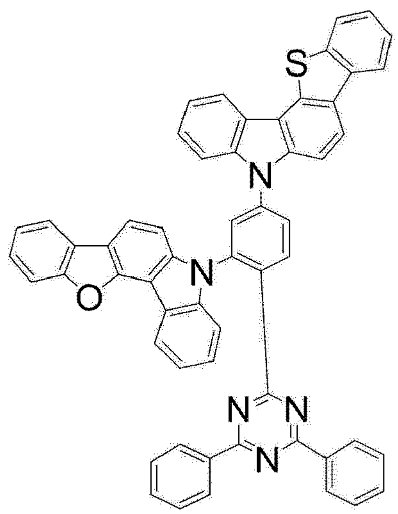

[Formula 57]

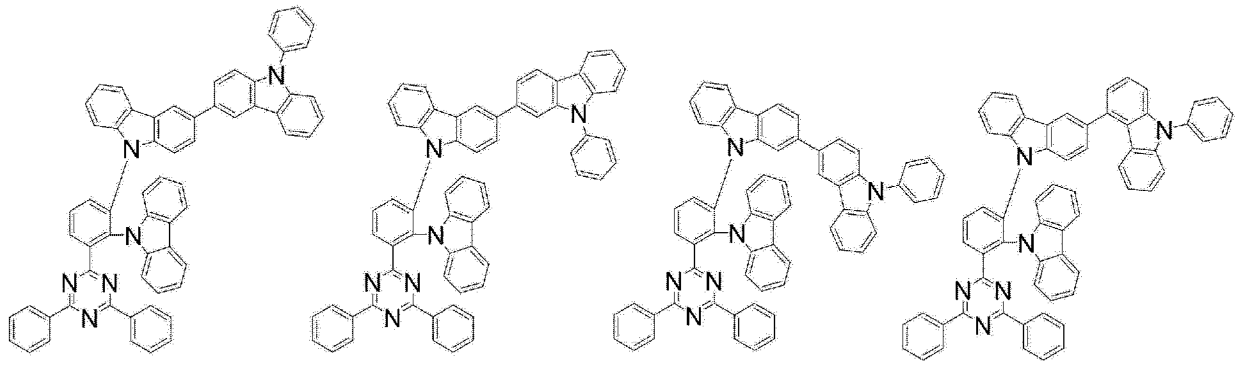

[Formula 58]

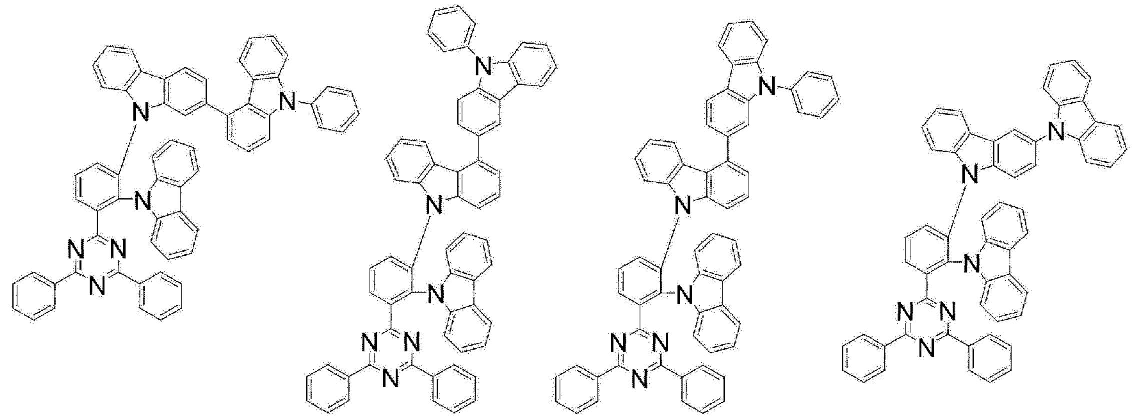

[Formula 59]

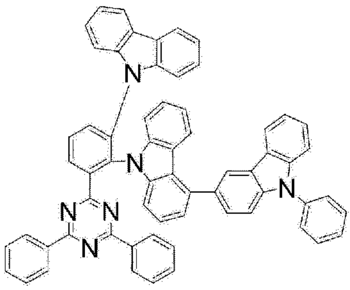
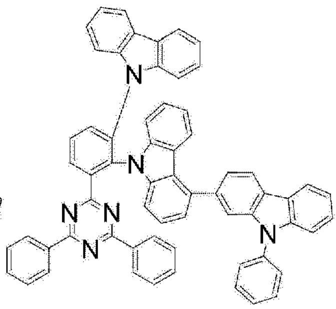
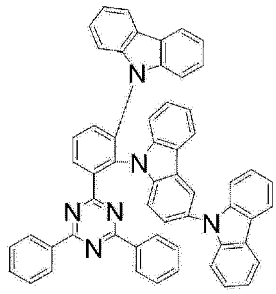

[Formula 60]

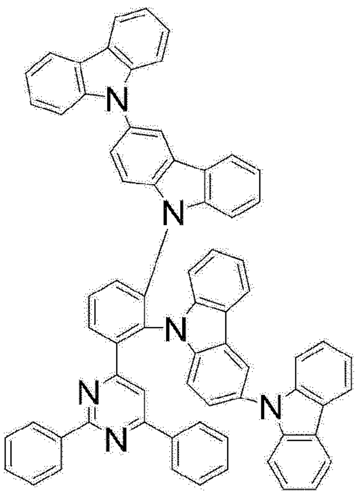
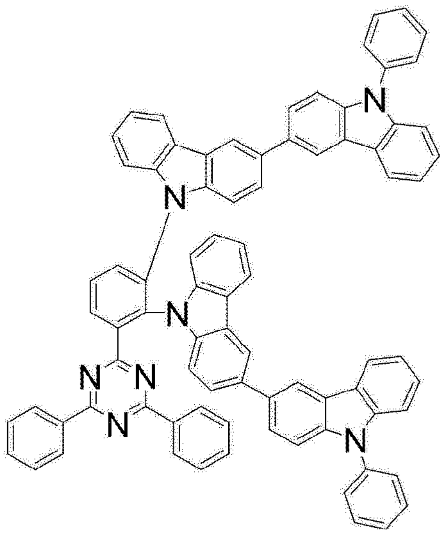

[Formula 61]

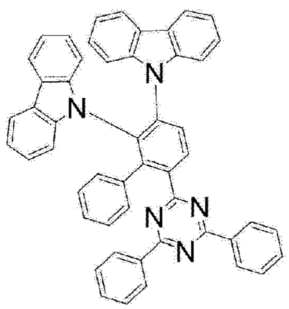
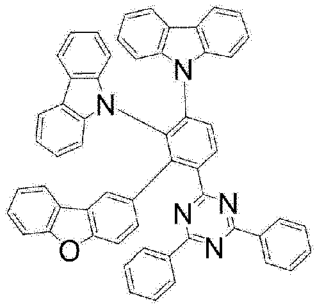
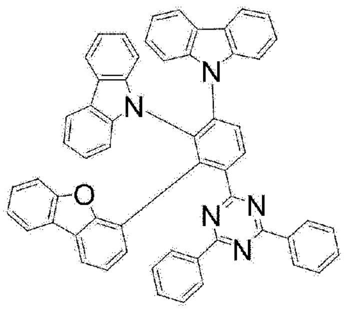

[Formula 62]

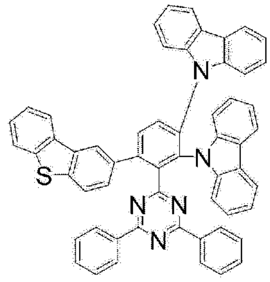
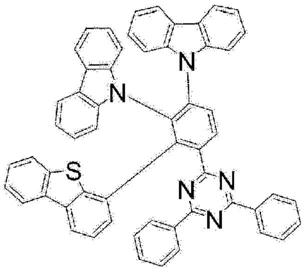
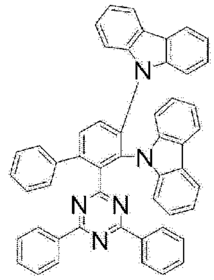
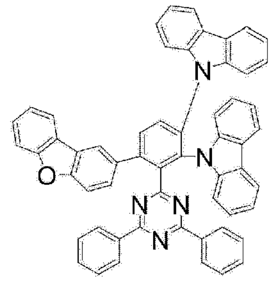

[Formula 63]

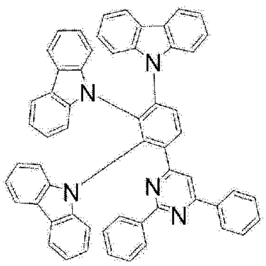
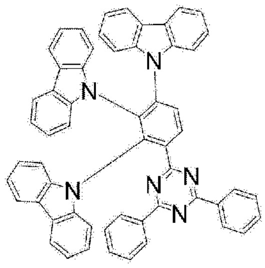
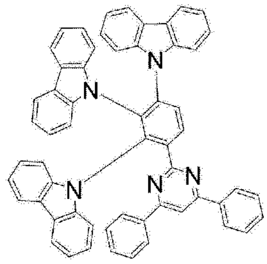
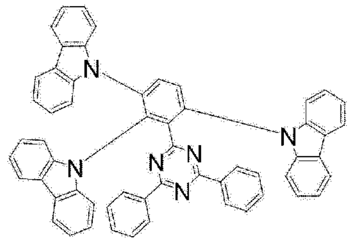
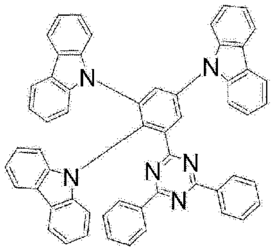
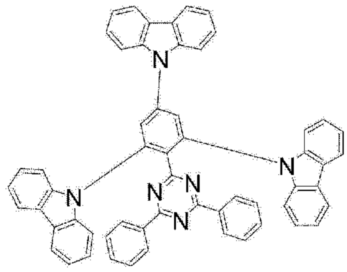
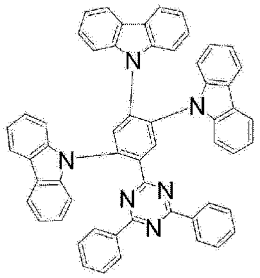

[Formula 64]

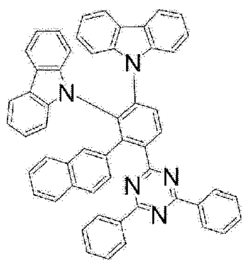
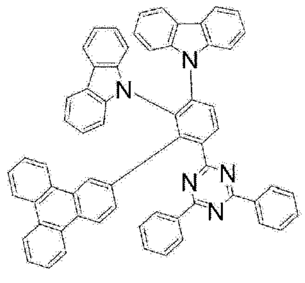
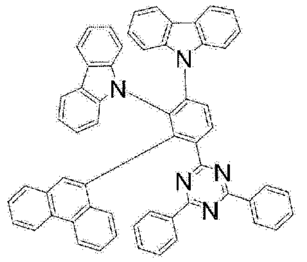
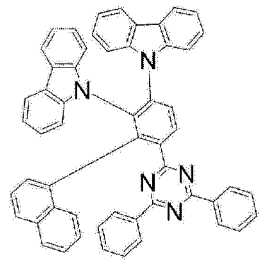
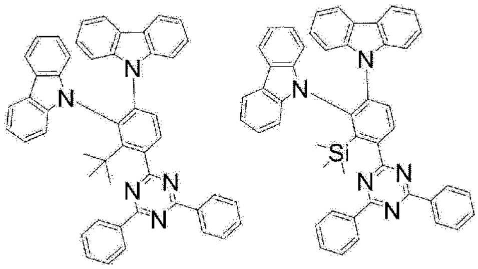
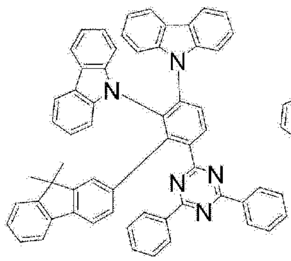
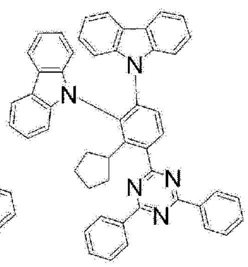

[Formula 65]

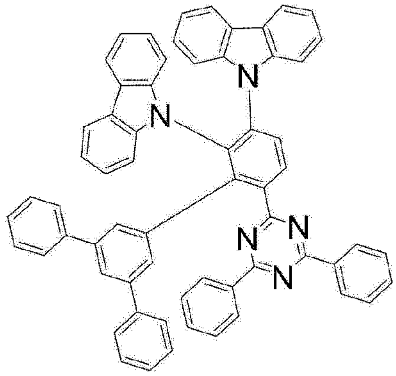

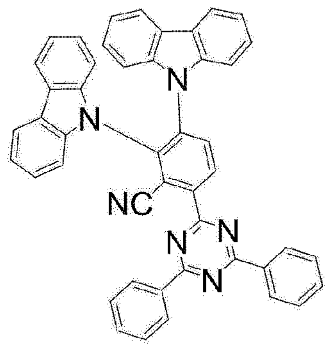

[Formula 66]

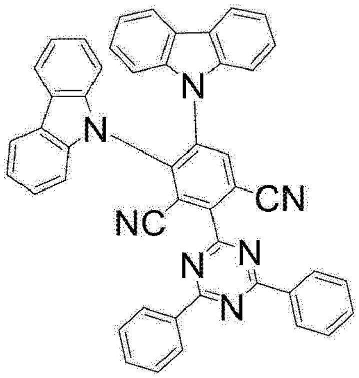

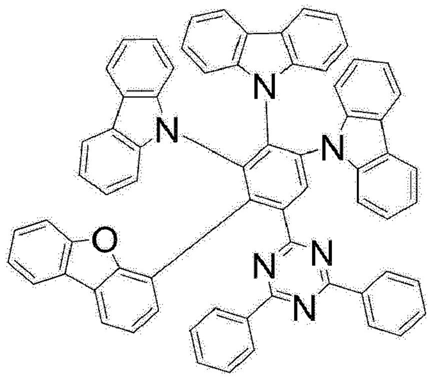

[Formula 67]

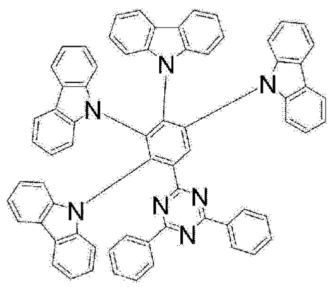

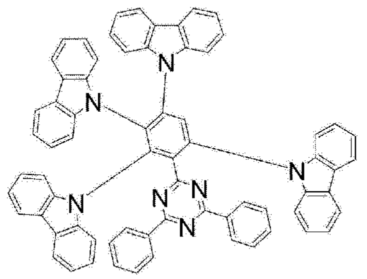

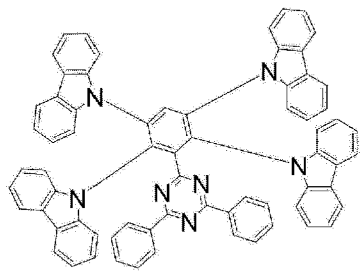

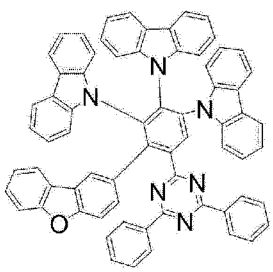

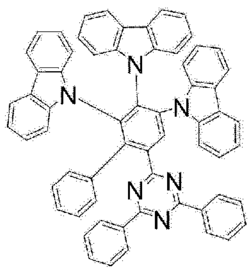

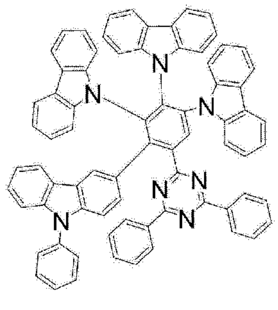

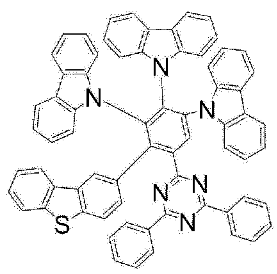

[Formula

68]

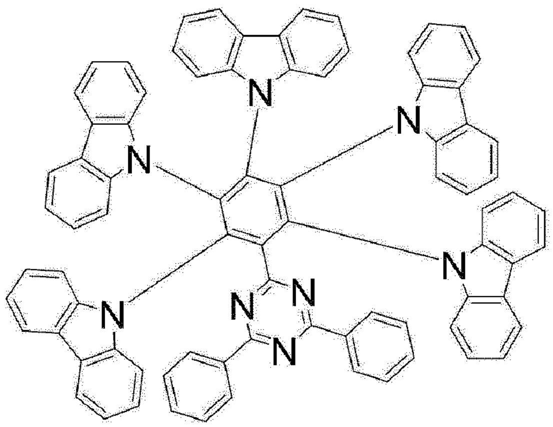

[Formula 70]

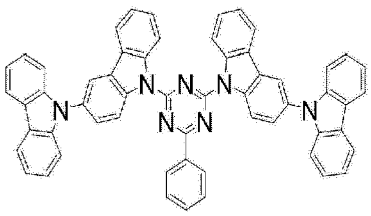

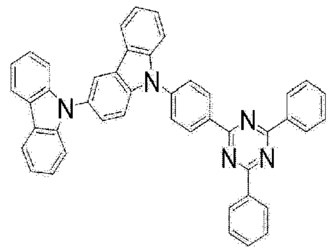

[Formula 71]

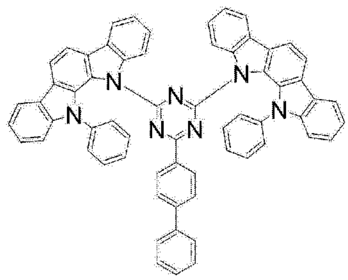

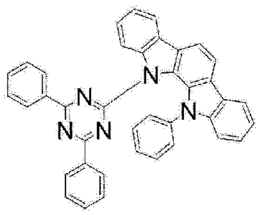

[Formula 74]

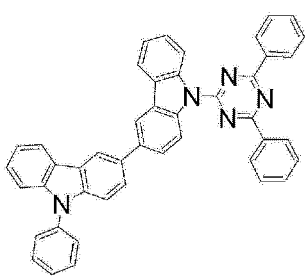

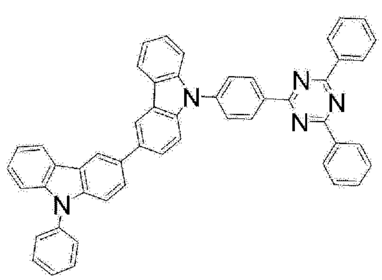

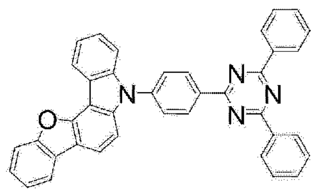

[Formula 75]

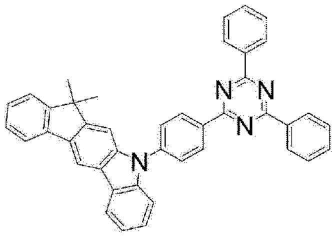

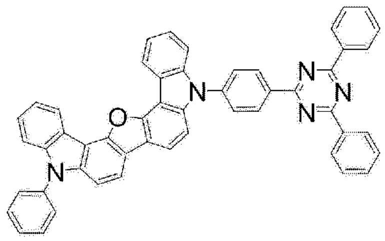

[Formula 76]

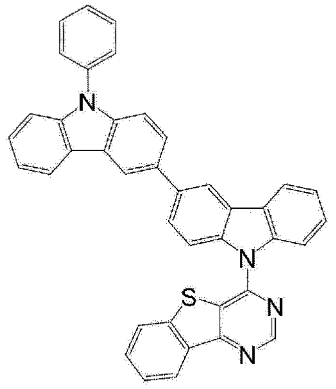

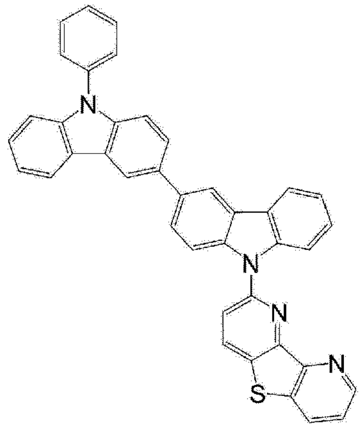

[Formula 77]

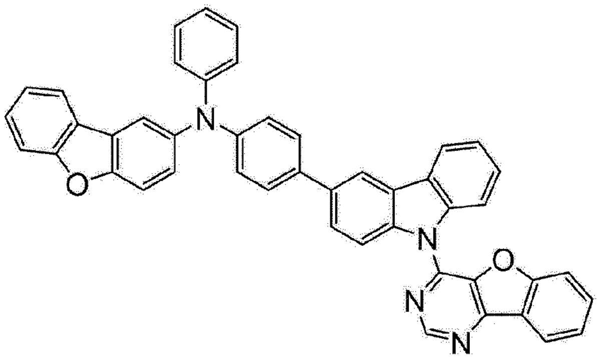

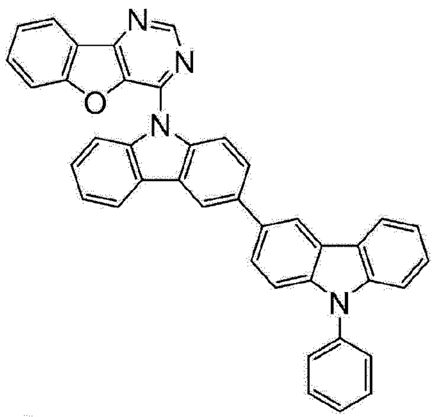

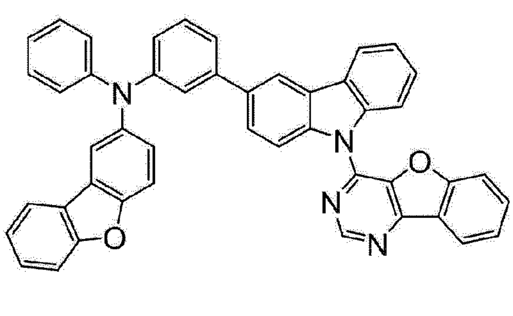

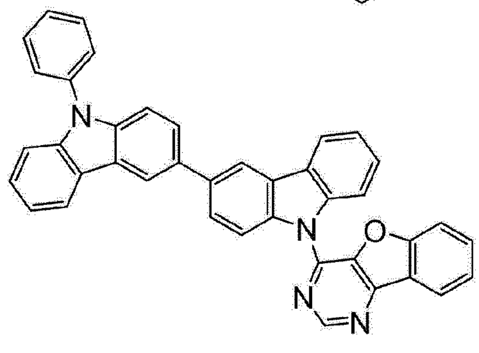

[Formula 78]

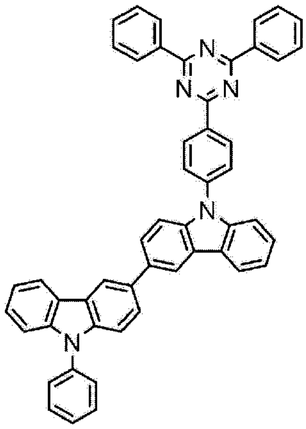 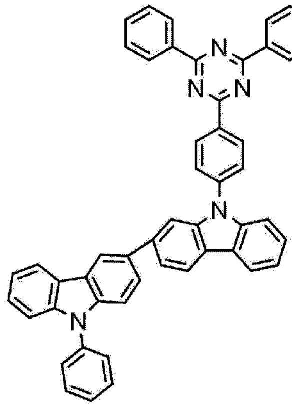 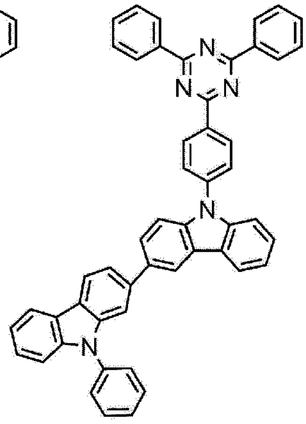 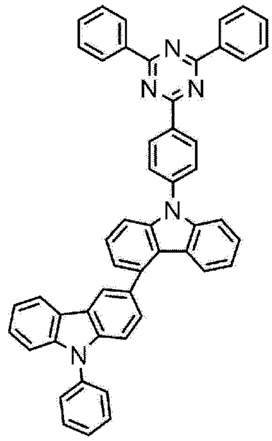

[Formula 79]

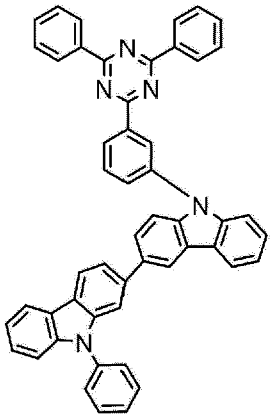 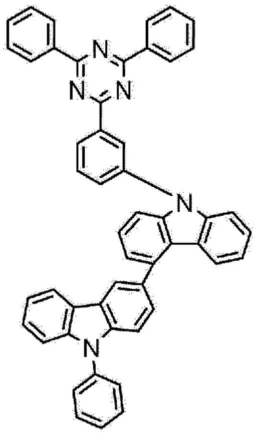 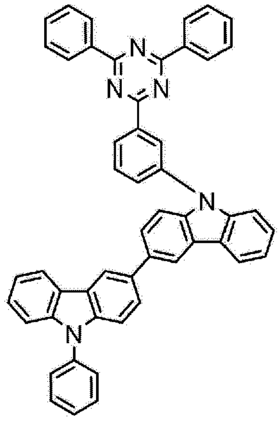 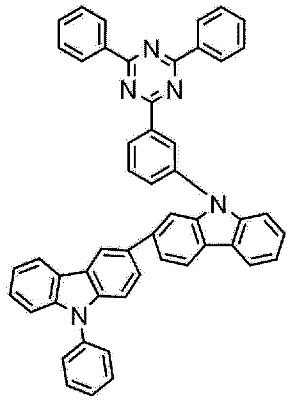

[Formula 80]

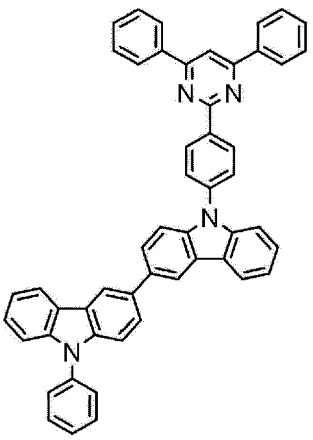 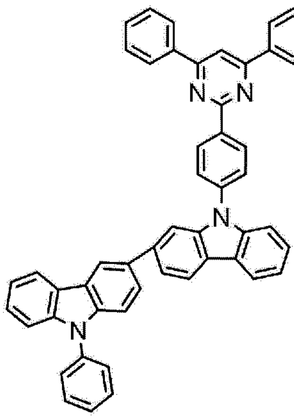 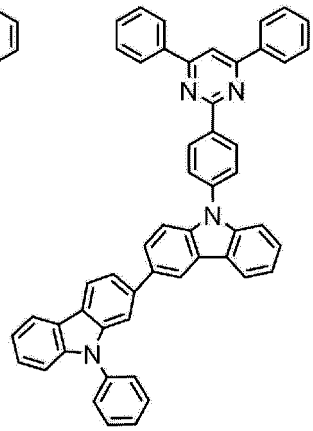 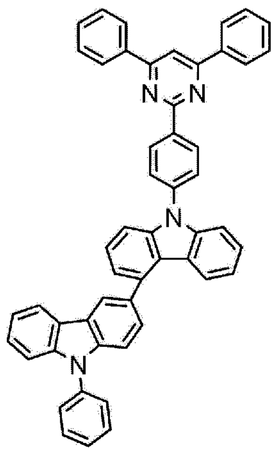

[Formula 81]

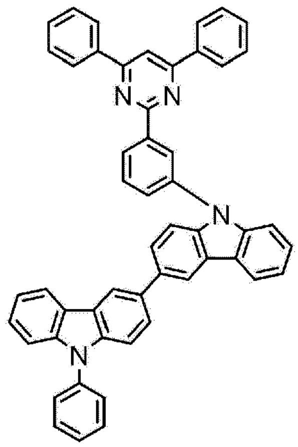

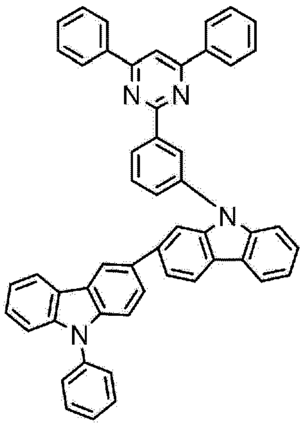

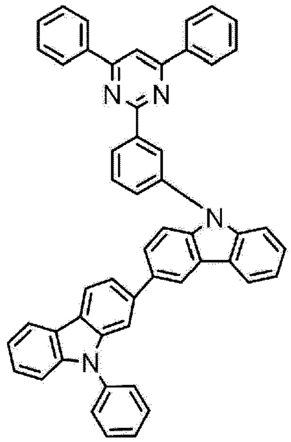

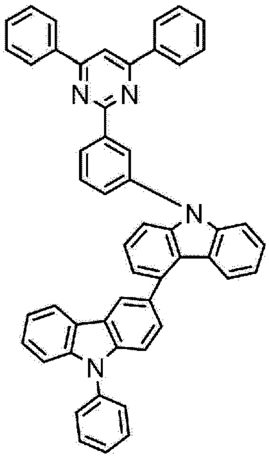

[Formula 82]

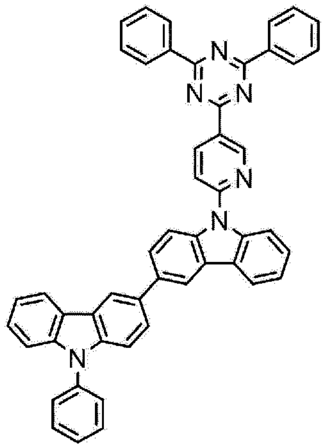

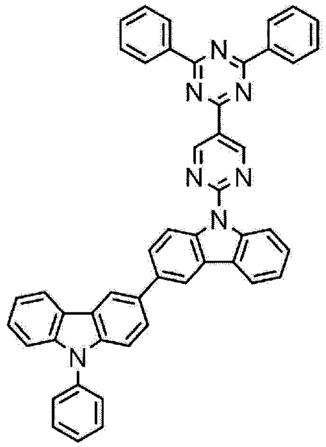

[Formula 83]

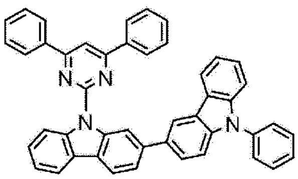

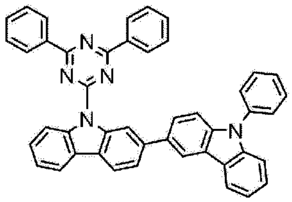

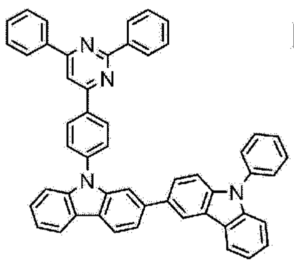

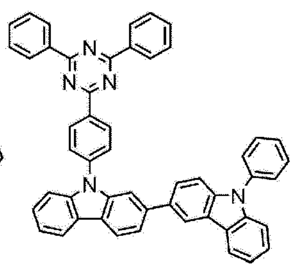

[Formula 84]

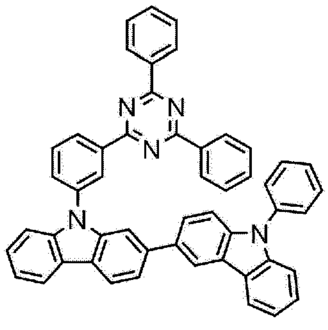
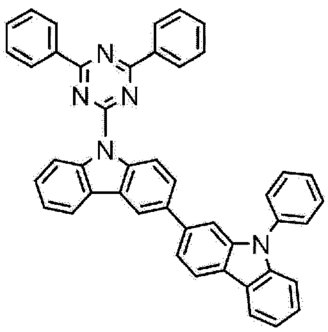

[Formula 85]

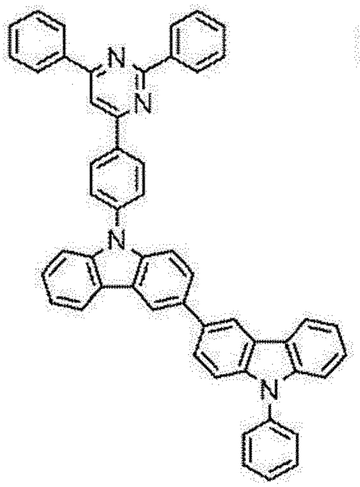
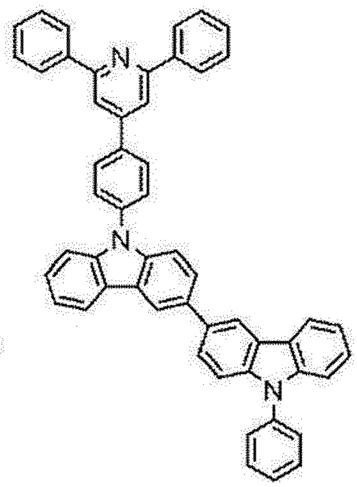
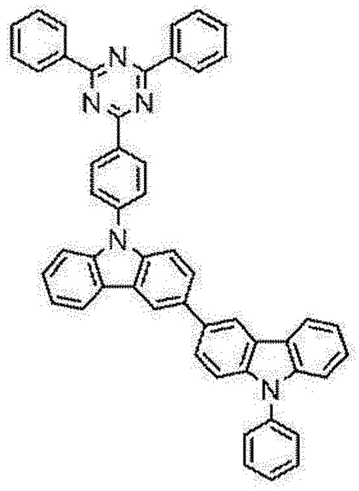

[Formula 86]

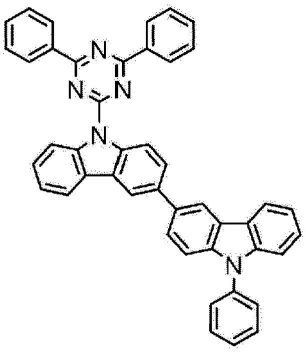
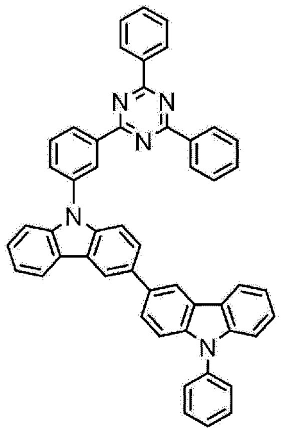
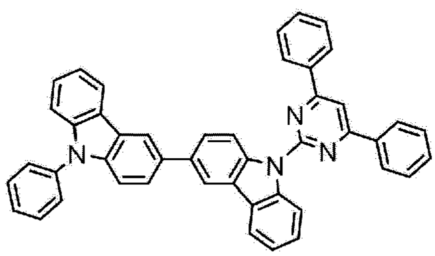

Relationship between First Compound and Second Compound in Emitting Layer

The emitting layer of the first exemplary embodiment includes the first compound and the second compound each as defined in the claims. The first compound is a delayed fluorescent compound. The second compound is a fluorescent compound having the characteristics of the above-described emission peak wavelength, molar absorbance coefficient, and Stokes shift in combination. Use of the second compound in combination with the first compound in the emitting layer allows the second compound to efficiently emit light, resulting in improvement in the luminous efficiency of the organic EL device.

A singlet energy $S_1(M1)$ of the first compound and a singlet energy $S_1(M2)$ of the second compound preferably

satisfy a relationship of a numerical formula (Numerical Formula 1) below.

$$S_1(M1) > S_1(M2) \quad \text{(Numerical Formula 1)}$$

An energy gap $T_{77K}$(M1) at 77 [K] of the first compound is preferably larger than an energy gap $T_{77K}$(M2) at 77 [K] of the second compound. Specifically, the energy gap T77K(M1) and the energy gap T77K(M2) preferably satisfy a relationship represented by a numerical formula below (Numerical Formula 4).

$$T_{77K}(M1) > T_{77K}(M2) \quad \text{(Numerical Formula 4)}$$

When the organic EL device 1 of the exemplary embodiment emits light, it is preferable that the second compound mainly emits light in the emitting layer 5.

Relationship between Triplet Energy and Energy Gap at 77 [K] Description will be made on a relationship between a triplet energy and an energy gap at 77 [K]. In the first exemplary embodiment, the energy gap at 77 [K] is different from a typically defined triplet energy in some aspects.

The triplet energy is measured as follows. Firstly, a target compound for measurement is dissolved in an appropriate solvent to prepare a solution and the solution is encapsulated in a quartz glass tube to prepare a sample. A phosphorescent spectrum (ordinate axis: phosphorescent luminous intensity, abscissa axis: wavelength) of the sample is measured at a low temperature (77K). A tangent is drawn to a rise of the phosphorescent spectrum on the short-wavelength side. The triplet energy is calculated by a predetermined conversion equation based on a wavelength value at an intersection of the tangent and the abscissa axis.

The delayed fluorescent compound usable in the first exemplary embodiment is preferably a compound having a small $\Delta$ST. When $\Delta$ST is small, intersystem crossing and inverse intersystem crossing are likely to occur even at a low temperature (77K), so that the singlet state and the triplet state coexist. As a result, the spectrum to be measured in the same manner as the above includes emission from both the singlet state and the triplet state. Although it is difficult to distinguish the emission from the singlet state from the emission from the triplet state, the value of the triplet energy is basically considered dominant.

Accordingly, in the first exemplary embodiment, the triplet energy is measured by the same method as a typical triplet energy T, but a value measured in the following manner is referred to as an energy gap $T_{77K}$ in order to differentiate the measured energy from the typical triplet energy in a strict meaning. The measurement target compound is dissolved in EPA (diethyl ether: isopentane : ethanol = 5 : 5 : 2 (by volume ratio)) such that a concentration of the compound becomes 10 $\mu$mol/L. The obtained solution is put into a quartz cell to provide a measurement sample. A phosphorescent spectrum (ordinate axis: phosphorescent luminous intensity, abscissa axis: wavelength) of the sample is measured at a low temperature (77K). A tangent is drawn to a rise of the phosphorescent spectrum on the short-wavelength side. An energy amount is calculated as an energy gap $T_{77K}$ at 77[K] by the following conversion equation based on a wavelength value $\lambda_{edge}$ [nm] at an intersection of the tangent and the abscissa axis.

$$\text{Conversion Equation (F1): } T_{77K}\ [\text{eV}] = 1239.85/\lambda_{edge}$$

The tangent to the rise of the phosphorescence spectrum on the short-wavelength side is drawn as follows. While moving on a curve of the phosphorescence spectrum from the short-wavelength side to the maximum spectral value closest to the short-wavelength side among the maximum spectral values, a tangent is checked at each point on the curve toward the long-wavelength of the phosphorescence spectrum. An inclination of the tangent is increased as the curve rises (i.e., a value of the ordinate axis is increased). A tangent drawn at a point of the maximum inclination (i.e., a tangent at an inflection point) is defined as the tangent to the rise of the phosphorescence spectrum on the short-wavelength side.

The maximum with peak intensity being 15% or less of the maximum peak intensity of the spectrum is not included in the above-mentioned maximum closest to the short-wavelength side of the spectrum. The tangent drawn at a point of the maximum spectral value being closest to the short-wavelength side and having the maximum inclination is defined as a tangent to the rise of the phosphorescence spectrum on the short-wavelength side.

For phosphorescence measurement, a spectrophotofluorometer body F-4500 (manufactured by Hitachi High-Technologies Corporation) is usable. The measurement instrument is not limited to this arrangement. A combination of a cooling unit, a low temperature container, an excitation light source and a light-receiving unit may be used for measurement.

Singlet Energy $S_1$

A method of measuring a singlet energy $S_1$ using a solution (hereinafter, occasionally referred to as a solution method) is exemplified by a method below.

10 μmol/L of a toluene solution of the measurement target compound is prepared and put in a quartz cell to prepare a sample. An absorption spectrum (ordinate axis: luminous intensity, abscissa axis: wavelength) of the sample is measured at a normal temperature (300K).A tangent is drawn to the fall of the absorption spectrum on the long-wavelength side, and a wavelength value λedge [nm] at an intersection of the tangent and the abscissa axis is substituted in the following conversion equation (F2) to calculate a singlet energy.

Conversion Equation (F2): $S_1$ [eV] = 1239.85/λedge

An absorption spectrum measurement device is not limited to but exemplified by a spectrophotometer (device name: U3310 manufactured by Hitachi, Ltd.).

The tangent to the fall of the absorption spectrum on the long-wavelength side is drawn as follows. While moving on a curve of the absorption spectrum from the maximum spectral value closest to the long-wavelength side in a long-wavelength direction, a tangent at each point on the curve is checked. An inclination of the tangent is decreased and increased in a repeated manner as the curve falls (i.e., a value of the ordinate axis is decreased).A tangent drawn at a point of the minimum inclination closest to the long-wavelength side (except when absorbance is 0.1 or less) is defined as the tangent to the fall of the absorption spectrum on the long-wavelength side.

The maximum absorbance of 0.2 or less is not included in the above-mentioned maximum absorbance on the long-wavelength side.

Content Ratio of Compounds in Emitting Layer

A content ratio between the first compound and the second compound in the emitting layer 5 is preferably in an exemplary range below.

The content ratio of the first compound is preferably in a range from 90 mass% to 99.9 mass%, more preferably in a range from 95 mass% to 99.9 mass%, particularly preferably in a range from 99 mass% to 99.9 mass%.

The content ratio of the second compound is preferably in a range from 0.01 mass% to 10 mass%, more preferably in a range from 0.01 mass% to 5 mass%, further preferably in a range from 0.01 mass% to 1 mass%.

It should be noted that the emitting layer 5 of the exemplary embodiment may further contain another material in addition to the first and second compounds.

Film Thickness of Emitting Layer

A film thickness of the emitting layer 5 is preferably in a range from 5 nm to 50 nm, more preferably in a range from 7 nm to 50 nm, and further preferably in a range from 10 nm to 50 nm. At 5 nm or more of the film thickness, the emitting layer 5 is easily formable and chromaticity of the emitting layer 5 is easily adjustable. When the film thickness of the emitting layer 5 is 50 nm or less, an increase in the drive voltage is suppressible.

TADF Mechanism

Fig. 4 exemplarily shows a relationship in energy levels between the first compound and the second compound in the emitting layer. In Fig. 4, S0 represents a ground state. S1(M1) represents the lowest singlet state of the first compound. T1(M1) represents the lowest triplet state of the first compound. S1(M2) represents the lowest singlet state of the second compound. T1(M2) represents the lowest triplet state of the second compound.

A dashed arrow directed from S1(M1) to S1(M2) in Fig. 4 represents Forster energy transfer from the lowest singlet state of the first compound to the second compound.

As shown in Fig. 4, when a compound having a small ΔST(M1) is used as the first compound, inverse intersystem crossing from the lowest triplet state T1(M1) to the lowest singlet state S1(M1) can be caused by a heat energy. Accordingly, Forster energy transfer from the lowest singlet state S1(M1) of the first compound to the second compound is caused to generate the lowest singlet state S1(M2).As a result, fluorescence from the lowest singlet state S1(M2) of the second compound is observable. It is considered that the internal quantum efficiency can be theoretically raised up to 100% also by using delayed fluorescence by the TADF mechanism.

Substrate

A substrate 2 is used as a support for the organic EL device 1. For instance, glass, quartz, plastics and the like are usable for the substrate 2. A flexible substrate is also usable. The flexible substrate is a bendable substrate, which

is exemplified by a plastic substrate. A material for the plastic substrate include polycarbonate, polyarylate, polyethersulfone, polypropylene, polyester, polyvinyl fluoride, polyvinyl chloride, polyimide, and polyethylene naphthalate. Moreover, an inorganic vapor deposition film is also usable.

Anode

Metal, alloy, an electrically conductive compound, a mixture thereof and the like, which have a large work function (specifically, of 4.0 eV or more) is preferably usable as the anode 3 formed on the substrate 2. Specific examples of the material for the anode include indium tin oxide (ITO), indium tin oxide containing silicon or silicon oxide, indium zinc oxide, tungsten oxide, indium oxide containing zinc oxide and graphene. In addition, gold (Au), platinum (Pt), nickel (Ni), tungsten (W), chrome (Cr), molybdenum (Mo), iron (Fe), cobalt (Co), copper (Cu), palladium (Pd), titanium (Ti), or nitrides of these metal materials (e.g., titanium nitride) are usable.

The above materials are typically deposited as a film by sputtering. For instance, indium zinc oxide can be deposited as a film by sputtering using a target that is obtained by adding zinc oxide in a range from 1 mass% to 10 mass% to indium oxide. Moreover, for instance, indium oxide containing tungsten oxide and zinc oxide can be deposited as a film by sputtering using a target that is obtained by adding tungsten oxide in a range from 0.5 mass% to 5 mass% and zinc oxide in a range from 0.1 mass% to 1 mass% to indium oxide. In addition, vapor deposition, coating, ink jet printing, spin coating and the like may be used for forming a film.

Among the organic layers formed on the anode 3, the hole injecting layer 6 formed in contact with the anode 3 is formed using a composite material that facilitates injection of holes irrespective of the work function of the anode 3. Accordingly, a material usable as an electrode material (e.g., metal, alloy, an electrically conductive compound, a mixture thereof, and elements belonging to Groups 1 and 2 of the periodic table of the elements) is usable as the material for the anode 3. The elements belonging to Groups 1 and 2 of the periodic table of the elements, a rare earth metal and alloy thereof, which are materials having a small work function, are also usable as the material for the anode 3. The elements belonging to Group 1 of the periodic table of the elements are alkali metal. The elements belonging to Group 2 of the periodic table of the elements are alkaline earth metal. Examples of alkali metal are lithium (Li) and cesium (Cs).Examples of alkaline earth metal are magnesium (Mg), calcium (Ca), and strontium (Sr).Examples of the rare earth metal are europium (Eu) and ytterbium (Yb).Examples of the alloys including these metals are MgAg and AILi.

When the anode 3 is formed of the alkali metal, alkaline earth metal and alloy thereof, vapor deposition and sputtering are usable. Further, when the anode is formed of silver paste and the like, coating, ink jet printing and the like are usable.

Hole Injecting Layer

A hole injecting layer 6 is a layer containing a highly hole-injectable substance. Examples of the highly hole-injectable substance include molybdenum oxide, titanium oxide, vanadium oxide, rhenium oxide, ruthenium oxide, chromium oxide, zirconium oxide, hafnium oxide, tantalum oxide, silver oxide, tungsten oxide, and manganese oxide.

In addition, the examples of the highly hole-injectable substance further include: an aromatic amine compound, which is a low-molecule compound, such that 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), 4,4'-bis[N-(4-diphenylaminophenyl)-N-phenylamino]biphenyl(abbreviation: DPAB), 4,4'-bis(N-{4-[N'-(3-methylphenyl)-N'-phenylamino]phenyl}-N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylamino]-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCN1); and dipyrazino[2,3-f:20,30-h]quinoxaline-2,3,6,7,10,11-hexacarbonitrile (HAT-CN).

Moreover, a high-molecule compound is also usable as the highly hole-injectable substance. Examples of the high-molecule compound are an oligomer, dendrimer and polymer. Specific examples of the high-molecule compound include poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N'-phenylamino}phenyl)methacrylamido] (abbreviation: PTPDMA), and poly[N,N'-bis(4-butylphenyl)-N,N'-bis(phenyl)benzidine] (abbreviation: Poly-TPD). Furthermore, the examples of the high-molecule compound include a high-molecule compound added with an acid such as poly(3,4-ethylene dioxythiophene)/poly(styrene sulfonic acid) (PEDOT/PSS), and polyaniline/poly(styrene sulfonic acid) (PAni/PSS).

Hole Transporting Layer

A hole transporting layer 7 is a layer containing a highly hole-transportable substance. An aromatic amine compound, carbazole derivative, anthracene derivative and the like are usable for the hole transporting layer 7. Specific

examples of a material for the hole transporting layer include 4,4'-bis[N-(1-naphthyl)-N-phenylamino]biphenyl (abbreviation: NPB), N,N'-bis(3-methylphenyl)-N,N'-diphenyl-[1,1'-biphenyl]-4,4'-diamine (abbreviation: TPD), 4-phenyl-4'-(9-phenylfluorene-9-yl)triphenylamine (abbreviation: BAFLP), 4,4'-bis[N-(9,9-dimethylfluorene-2-yl)-N-phenylamino]biphenyl (abbreviation: DFLDPBi), 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), and 4,4'-bis[N-(spiro-9,9'-bifluorene-2-yl)-N-phenylamino]biphenyl (abbreviation: BSPB).The above-described substances mostly have a hole mobility of $10^{-6}cm^2/(V{\cdot}S)$ or more.

A carbazole derivative (e.g., CBP, 9-[4-(N-carbazolyl)]phenyl-10-phenylanthracene (CzPA), and 9-phenyl-3-[4-(10-phenyl-9-anthryl)phenyl]-9H-carbazole (PCzPA)) and an anthracene derivative (e.g., t-BuDNA, DNA, and DPAnth) are usable for the hole transporting layer 7. A high polymer compound such as poly(N-vinylcarbazole) (abbreviation: PVK) and poly(4-vinyltriphenylamine) (abbreviation: PVTPA) is also usable.

However, any substance having a hole transporting performance higher than an electron transporting performance is usable in addition to the above substances. A highly hole-transportable substance may be provided in the form of a single layer or a laminated layer of two or more layers of the above substance.

When the hole transporting layer includes two or more layers, one of the layers with a larger energy gap is preferably provided closer to the emitting layer 5.

Electron Transporting Layer

The electron transporting layer 8 is a layer containing a highly electron-transportable substance. As the electron transporting layer 8, (1) a metal complex such as an aluminum complex, beryllium complex and zinc complex, (2) heteroaromatic compound such as an imidazole derivative, benzimidazole derivative, azine derivative, carbazole derivative, and phenanthroline derivative, and (3) a high-molecule compound are usable. Specifically, as a low-molecule organic compound, a metal complex such as Alq, tris(4-methyl-8-quinolinato)aluminum (abbreviation: Almq3), bis(10-hydroxybenzo[h]quinolinato)beryllium (abbreviation: BeBq2), BAlq, Znq, ZnPBO and ZnBTZ are usable. In addition to the metal complex, a heteroaromatic compound such as 2-(4-biphenyl)-5-(4-tert-butylphenyl)-1,3,4-oxadiazole (abbreviation: PBD), 1,3-bis[5-(ptert-butylphenyl)-1,3,4-oxadiazole-2-yl]benzene (abbreviation: OXD-7), 3-(4-tert-butylphenyl)-4-phenyl-5-(4-biphenyl)-1,2,4-triazole (abbreviation: TAZ), 3-(4-tert-butylphenyl)-4-(4-ethylphenyl)-5-(4-biphenyl)-1,2,4-triazole (abbreviation: p-EtTAZ), bathophenanthroline (abbreviation: BPhen), bathocuproine (abbreviation: BCP), and 4,4'-bis(5-methylbenzoxazole-2-yl)stilbene (abbreviation: BzOs) are usable. In the exemplary embodiment, a benzimidazole compound is suitably usable. The above-described substances mostly have an electron mobility of $10^{-6}$ $cm^2/(V{\cdot}s)$ or more. However, any substance having an electron transporting performance higher than a hole transporting performance may be used for the electron transporting layer 8 in addition to the above substances. The electron transporting layer 8 may be provided in the form of a single layer or a laminated layer of two or more layers of the above substance(s).

Moreover, a high-molecule compound is also usable for the electron transporting layer 8. For instance, poly[(9,9-dihexylfluorene-2,7-diyl)-co-(pyridine-3,5-diyl)] (abbreviation: PF-Py), poly[(9,9-dioctylfluorene-2,7-diyl)-co-(2,2'-bipyridine-6,6'-diyl)](abbreviation: PF-BPy) and the like are usable.

Electron Injecting Layer

The electron injecting layer 9 is a layer containing a highly electron-injectable substance. Examples of a material for the electron injecting layer 9 include an alkali metal, alkaline earth metal and a compound thereof, examples of which include lithium (Li), cesium (Cs), calcium (Ca), lithium fluoride (LiF), cesium fluoride (CsF), calcium fluoride (CaF2), and lithium oxide (LiOx). In addition, a substance obtained by containing an alkali metal, alkaline earth metal or a compound thereof in the electron transportable substance, specifically, for instance, a substance obtained by containing magnesium (Mg) in Alq may be used. With this substance, electrons can be more efficiently injected from the cathode 4.

Alternatively, a composite material provided by mixing an organic compound with an electron donor may be used for the electron injecting layer 9. The composite material exhibits excellent electron injecting performance and electron transporting performance since the electron donor generates electron in the organic compound. In this arrangement, the organic compound is preferably a material exhibiting an excellent transforming performance of the generated electrons. Specifically, for instance, the above-described substance for the electron transporting layer 8 (e.g., the metal complex and heteroaromatic compound) is usable. The electron donor may be any substance exhibiting an electron donating performance to the organic compound. Specifically, an alkali metal, alkaline earth metal and a rare earth metal are preferable, examples of which include lithium, cesium, magnesium, calcium, erbium and ytterbium. Moreover, an alkali metal oxide or alkaline earth metal oxide is preferably used as the electron donor, examples of which include lithium oxide, calcium oxide, and barium oxide. Further, Lewis base such as magnesium oxide is also usable. Furthermore, tetrathiafulvalene (abbreviation: TTF) is also usable.

Cathode

Metal, alloy, an electrically conductive compound, a mixture thereof and the like, which have a small work function, specifically, of 3.8 eV or less, is preferably usable as a material for the cathode 4. Specific examples of the material for the cathode are the elements belonging to Groups 1 and 2 of the periodic table of the elements, a rare earth metal and alloys thereof. The elements belonging to Group 1 of the periodic table of the elements are alkali metal. The elements belonging to Group 2 of the periodic table of the elements are alkaline earth metal. Examples of alkali metal are lithium (Li) and cesium (Cs).Examples of alkaline earth metal are magnesium (Mg), calcium (Ca), and strontium (Sr).Examples of the rare earth metal are europium (Eu) and ytterbium (Yb).Examples of the alloys including these metals are MgAg and AlLi.

When the cathode 4 is formed of the alkali metal, alkaline earth metal and alloy thereof, vapor deposition and sputtering are usable. Further, when the anode is formed of silver paste and the like, coating, ink jet printing and the like are usable.

By providing the electron injecting layer 9, various conductive materials such as Al, Ag, ITO, graphene and indium tin oxide containing silicon or silicon oxide are usable for forming the cathode 4 irrespective of the magnitude of the work function. The conductive materials can be deposited as a film by sputtering, ink jet printing, spin coating and the like.

Layer Formation Method(s)

There is no restriction except for the above particular description for a method for forming each layer of the organic EL device 1 in the exemplary embodiment. Known methods such as dry film-forming and wet film-forming are applicable. Examples of the dry film-forming include vacuum deposition, sputtering, plasma and ion plating. Examples of the wet film-forming include spin coating, dipping, flow coating and ink-jet.

Film Thickness

There is no restriction except for the above particular description for a film thickness of each of the organic layers of the organic EL device 1 in the exemplary embodiment. The thickness is generally preferably in a range from several nanometers to 1 $\mu$m in order to cause less defects (e.g., a pin hole) and prevent deterioration in the efficiency caused by requiring high voltage to be applied.

Herein, the number of carbon atoms forming a ring (also referred to as ring carbon atoms) means the number of carbon atoms included in atoms forming the ring itself of a compound in which the atoms are bonded to form the ring (e.g., a monocyclic compound, a fused ring compound, a cross-linked compound, a carbocyclic compound, and a heterocyclic compound).When the ring is substituted by a substituent, carbon atom(s) included in the substituent is not counted as the ring carbon atoms. The same applies to the "ring carbon atoms" described below, unless particularly noted. For instance, a benzene ring has 6 ring carbon atoms, a naphthalene ring has 10 ring carbon atoms, a pyridinyl group has 5 ring carbon atoms, and a furanyl group has 4 ring carbon atoms. When a benzene ring or a naphthalene ring is substituted, for instance, by an alkyl group, the carbon atoms of the alkyl group are not counted as the ring carbon atoms. For instance, when a fluorene ring (inclusive of a spirofluorene ring) is bonded as a substituent to a fluorene ring, the carbon atoms of the fluorene ring as a substituent are not counted as the ring carbon atoms.

Herein, the number of atoms forming a ring (also referred to as ring atoms) means the number of atoms forming the ring itself of a compound in which the atoms are bonded to form the ring (e.g., a monocyclic compound, a fused ring compound, a cross-linked compound, a carbocyclic compound, and a heterocyclic compound).Atom(s) not forming the ring and atom(s) included in the substituent substituting the ring are not counted as the ring atoms. The same applies to the "ring atoms" described below, unless particularly noted. For instance, a pyridine ring has 6 ring atoms, a quinazoline ring has 10 ring atoms, and a furan ring has 5 ring atoms. Hydrogen atoms respectively bonded to carbon atoms of the pyridine ring or the quinazoline ring and atoms forming a substituent are not counted as the ring atoms. For instance, when a fluorene ring (inclusive of a spirofluorene ring) is bonded as a substituent to a fluorene ring, the atoms of the fluorene ring as a substituent are not counted as the ring atoms.

Next, each of substituents described in the above formulae will be described.

Herein, examples of an aryl group (occasionally referred to as an aromatic hydrocarbon group) having 6 to 30 ring carbon atoms include a phenyl group, biphenyl group, terphenyl group, naphthyl group, anthryl group, phenanthryl group, fluorenyl group, pyrenyl group, chrysenyl group, fluoranthenyl group, benz[a]anthryl group, benzo[c]phenanthryl group, triphenylenyl group, benzo[k]fluoranthenyl group, benzo[g]chrysenyl group, benzo[b]triphenylenyl group, picenyl group, and perylenyl group.

Herein, the aryl group preferably has 6 to 20 ring carbon atoms, more preferably 6 to 14 ring carbon atoms, further preferably 6 to 12 ring carbon atoms. Among the aryl group, a phenyl group, biphenyl group, naphthyl group,

phenanthryl group, terphenyl group and fluorenyl group are further more preferable. A carbon atom at a position 9 of each of 1-fluorenyl group, 2-fluorenyl group, 3-fluorenyl group and 4-fluorenyl group is preferably substituted by a substituted or unsubstituted alkyl group having 1 to 30 carbon atoms or a substituted or unsubstituted aryl group having 6 to 18 ring carbon atoms described herein below.

Herein, the heteroaryl group (occasionally referred to as heterocyclic group, heteroaromatic ring group or aromatic heterocyclic group) having 5 to 30 ring atoms preferably contains at least one hetero atom selected from the group consisting of nitrogen, sulfur, oxygen, silicon, selenium atom and germanium atom, and more preferably contains at least one hetero atom selected from the group consisting of nitrogen, sulfur and oxygen.

Herein, examples of the heterocyclic group having 5 to 30 ring atoms include a pyridyl group, pyrimidinyl group, pyrazinyl group, pyridazynyl group, triazinyl group, quinolyl group, isoquinolinyl group, naphthyridinyl group, phthalazinyl group, quinoxalinyl group, quinazolinyl group, phenanthridinyl group, acridinyl group, phenanthrolinyl group, pyrrolyl group, imidazolyl group, pyrazolyl group, triazolyl group, tetrazolyl group, indolyl group, benzimidazolyl group, indazolyl group, imidazopyridinyl group, benzotriazolyl group, carbazolyl group, furyl group, thienyl group, oxazolyl group, thiazolyl group, isoxazolyl group, isothiazolyl group, oxadiazolyl group, thiadiazolyl group, benzofuranyl group, benzothienyl group, benzoxazolyl group, benzothiazolyl group, benzisoxazolyl group, benzisothiazolyl group, benzoxadiazolyl group, benzothiadiazolyl group, dibenzofuranyl group, dibenzothienyl group, piperidinyl group, pyrrolidinyl group, piperazinyl group, morpholyl group, phenazinyl group, phenothiazinyl group, and phenoxazinyl group.

Herein, the heterocyclic group preferably has 5 to 20 ring atoms, more preferably 5 to 14 ring atoms. Among the above heterocyclic group, a 1-dibenzofuranyl group, 2-dibenzofuranyl group, 3-dibenzofuranyl group, 4-dibenzofuranyl group, 1-dibenzothienyl group, 2-dibenzothienyl group, 3-dibenzothienyl group, 4-dibenzothienyl group, 1-carbazolyl group, 2-carbazolyl group, 3-carbazolyl group, 4-carbazolyl group, and 9-carbazolyl group are further preferable. A nitrogen atom at a position 9 of each of 1-carbazolyl group, 2-carbazolyl group, 3-carbazolyl group and 4-carbazolyl group is preferably substituted by a substituted or unsubstituted aryl group having 6 to 30 ring carbon atoms or a substituted or unsubstituted heterocyclic group having 5 to 30 ring atoms described herein.

Herein, the heterocyclic group may be a group derived from any one of partial structures represented by formulae (XY-1) to (XY-18).

[Formula 87]

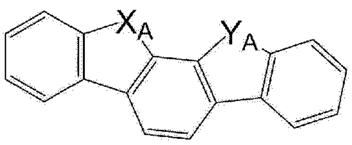

(XY-1)

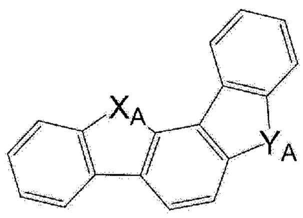

(XY-2)

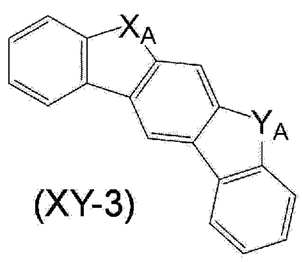

(XY-3)

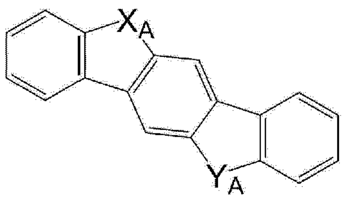

(XY-4)

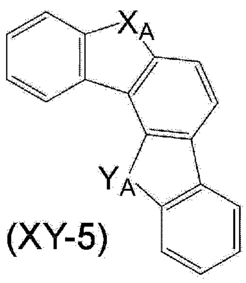

(XY-5)

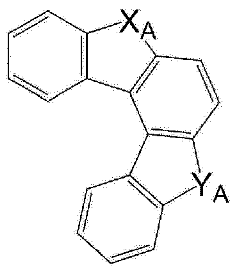

(XY-6)

[Formula 88]

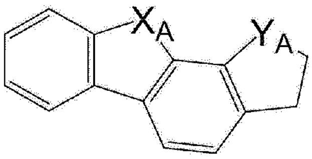

(XY-7)

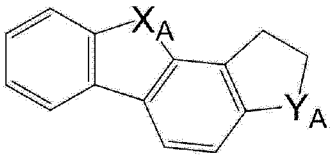

(XY-8)

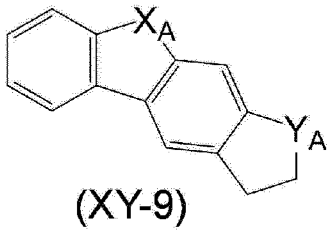

(XY-9)

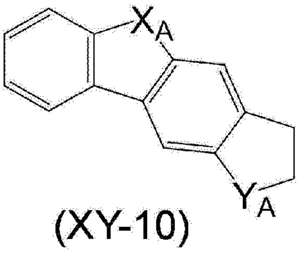

(XY-10)

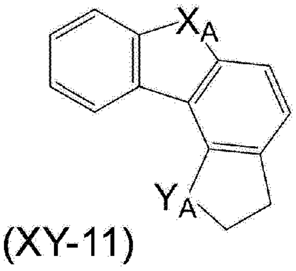

(XY-11)

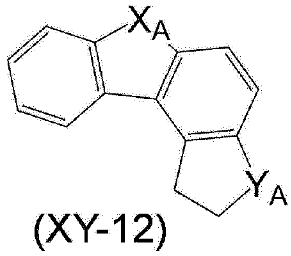

(XY-12)

[Formula 89]

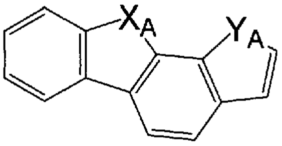

(XY-13)

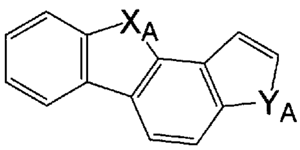

(XY-14)

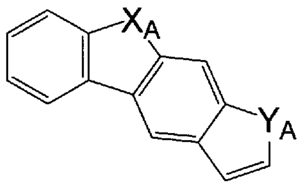

(XY-15)

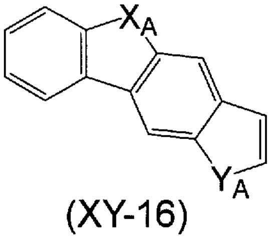

(XY-16)

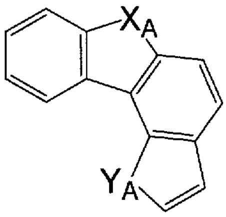

(XY-17)

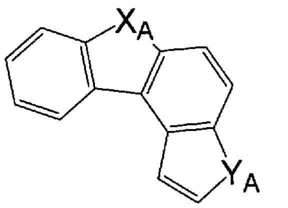

(XY-18)

In the formulae (XY-1) to (XY-18), $X_A$ and $Y_A$ are each independently a hetero atom, and are preferably an oxygen atom, sulfur atom, selenium atom, silicon atom or germanium atom. The partial structures represented by the formulae (XY-1) to (XY-18) may each have a bond(s) in any position to become a heterocyclic group, in which the heterocyclic group may be substituted.

Herein, examples of the substituted or unsubstituted carbazolyl group may include a group in which a carbazole ring is further fused with a ring(s) as shown in the following formulae. Such a group also may be substituted. Bonding positions of the group are alterable as desired.

[Formula 90]

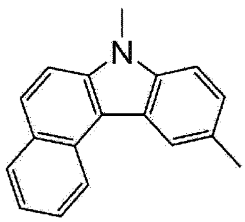 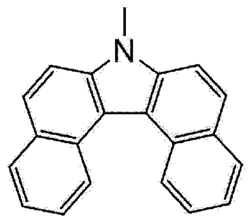 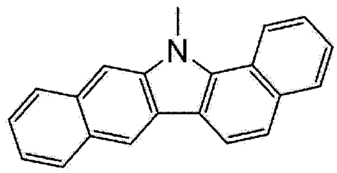 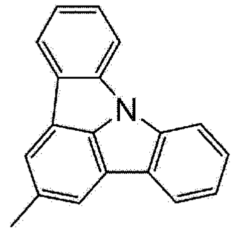

The alkyl group having 1 to 30 carbon atoms herein may be linear, branched or cyclic. Alternatively, the alkyl group having 1 to 30 carbon atoms herein may be an alkyl halide group.

Examples of the linear or branched alkyl group include: a methyl group, ethyl group, n-propyl group, isopropyl group, n-butyl group, s-butyl group, isobutyl group, t-butyl group, n-pentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, neopentyl group, amyl group, isoamyl group, 1-methylpentyl group, 2-methylpentyl group, 1-pentylhexyl group, 1-butylpentyl group, 1-heptyloctyl group, and 3-methylpentyl group.

Herein, the linear or branched alkyl group preferably has 1 to 10 carbon atoms, more preferably 1 to 6 carbon atoms. Among the linear or branched alkyl group, a methyl group, ethyl group, propyl group, isopropyl group, n-butyl group, s-butyl group, isobutyl group, t-butyl group, n-pentyl group, n-hexyl group, amyl group, isoamyl group and neopentyl group are further preferable.

Examples of the cyclic alkyl group herein include a cycloalkyl group having 3 to 30 ring carbon atoms.

Herein, examples of the cycloalkyl group having 3 to 30 ring carbon atoms include a cyclopropyl group, cyclobutyl group, cyclopentyl group, cyclohexyl group, 4-metylcyclohexyl group, adamantyl group and norbornyl group. The cycloalkyl group preferably has 3 to 10 ring carbon atoms, more preferably 5 to 8 ring carbon atoms. Among the cycloalkyl group, a cyclopentyl group and a cyclohexyl group are further preferable.

Herein, examples of the alkyl halide group provided by substituting an alkyl group with a halogen atom include an alkyl halide group provided by substituting the above alkyl group having 1 to 30 carbon atoms with one or more halogen atoms, preferably a fluorine atom(s).

Herein, specific examples of the alkyl halide group having 1 to 30 carbon atoms include a fluoromethyl group, difluoromethyl group, trifluoromethyl group, fluoroethyl group, trifluoromethylmethyl group, trifluoroethyl group and pentafluoroethyl group.

Herein, examples of the substituted silyl group include an alkylsilyl group having 3 to 30 carbon atoms and an arylsilyl group having 6 to 30 ring carbon atoms.

Herein, examples of the alkylsilyl group having 3 to 30 carbon atoms include a trialkylsilyl group having the above examples of the alkyl group having 1 to 30 carbon atoms. Specific examples of the alkylsilyl group include a trimethylsilyl group, triethylsilyl group, tri-n-butylsilyl group, tri-n-octylsilyl group, triisobutylsilyl group, dimethylethylsilyl group, dimethylisopropylsilyl group, dimethyl-n-propylsilyl group, dimethyl-n-butylsilyl group, dimethyl-t-butylsilyl group, diethylisopropylsilyl group, vinyl dimethylsilyl group, propyldimethylsilyl group, and triisopropylsilyl group. Three alkyl groups in the trialkylsilyl group may be mutually the same or different.

Herein, examples of the arylsilyl group having 6 to 30 ring carbon atoms include a dialkylarylsilyl group, alkyldiarylsilyl group and triarylsilyl group.

The dialkylarylsilyl group is exemplified by a dialkylarylsilyl group including two of the alkyl group listed as the examples of the alkyl group having 1 to 30 carbon atoms and one of the aryl group listed as the examples of the aryl group having 6 to 30 ring carbon atoms. The dialkylarylsilyl group preferably has 8 to 30 carbon atoms.

The alkyldiarylsilyl group is exemplified by an alkyldiarylsilyl group including one of the alkyl group listed as the examples of the alkyl group having 1 to 30 carbon atoms and two of the aryl group listed as the examples of the aryl group having 6 to 30 ring carbon atoms. The alkyldiarylsilyl group preferably has 13 to 30 carbon atoms.

The triarylsilyl group is exemplified by a triarylsilyl group including three of the aryl group listed as the examples of the aryl group having 6 to 30 ring carbon atoms. The triarylsilyl group preferably has 18 to 30 carbon atoms.

Herein, an aryl group included in an aralkyl group (occasionally referred to as an arylalkyl group) is an aromatic hydrocarbon group or a heterocyclic group.

Herein, the aralkyl group having 5 to 30 carbon atoms is preferably an aralkyl group having 6 to 30 ring carbon atoms and is represented by $-Z_3-Z_4$.$Z_3$ is exemplified by an alkylene group corresponding to the above alkyl group having

1 to 30 carbon atoms. $Z_4$ is exemplified by the above aryl group having 6 to 30 ring carbon atoms. The aralkyl group is preferably an aralkyl group having 7 to 30 carbon atoms, in which an aryl moiety has 6 to 30 carbon atoms, preferably 6 to 20 carbon atoms, more preferably 6 to 12 carbon atoms and an alkyl moiety has 1 to 30 carbon atoms, preferably 1 to 20 carbon atoms, more preferably 1 to 10 carbon atoms, further preferably 1 to 6 carbon atoms. Examples of the aralkyl group include a benzyl group, 2-phenylpropane-2-yl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, and 2-β-naphthylisopropyl group.

Herein, a substituted phosphoryl group is represented by a formula (P) below.

[Formula 91]

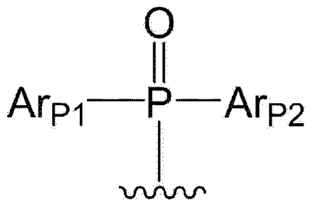

(P)

$Ar_{P1}$ and $Ar_{P2}$ of the formula (P) are each independently a substituent and are preferably any substituent selected from the group consisting of an alkyl group having 1 to 30 carbon atoms and an aryl group having 6 to 30 ring carbon atoms, more preferably any substituent selected from the group consisting of an alkyl group having 1 to 10 carbon atoms and an aryl group having 6 to 20 ring carbon atoms, further preferably any substituent selected from the group consisting of an alkyl group having 1 to 6 carbon atoms and an aryl group having 6 to 14 ring carbon atoms.

Herein, the alkoxy group having 1 to 30 carbon atoms is represented by $-OZ_1$.$Z_1$ is exemplified by the above alkyl group having 1 to 30 carbon atoms. Examples of the alkoxy group include a methoxy group, ethoxy group, propoxy group, butoxy group, pentyloxy group and hexyloxy group. The alkoxy group preferably has 1 to 20 carbon atoms.

A halogenated alkoxy group provided by substituting an alkoxy group with a halogen atom is exemplified by one provided by substituting an alkoxy group having 1 to 30 carbon atoms with one or more fluorine atoms.

Herein, an aryl group included in an arylalkoxy group (occasionally referred to as an aryloxy group) also include a heteroaryl group.

Herein, the arylalkoxy group having 5 to 30 carbon atoms is represented by $-OZ_2$.$Z_2$ is exemplified by the above aryl group having 6 to 30 ring carbon atoms. The arylalkoxy group preferably has 6 to 20 ring carbon atoms. The arylalkoxy group is exemplified by a phenoxy group.

Herein, a substituted amino group is represented by -NHRv or $-N(Rv)_2$. Examples of Rv include the above alkyl group having 1 to 30 carbon atoms and the above aryl group having 6 to 30 ring carbon atoms.

Herein, an alkenyl group having 2 to 30 carbon atoms is linear or branched. Examples of the alkenyl group having 2 to 30 carbon atoms include a vinyl group, propenyl group, butenyl group, oleyl group, eicosapentaenyl group, docosahexaenyl group, styryl group, 2,2-diphenylvinyl group, 1,2,2-triphenylvinyl group, and 2-phenyl-2-propenyl group.

Herein, examples of a cycloalkenyl group having 3 to 30 carbon atoms include a cyclopentadienyl group, cyclopentenyl group, cyclohexenyl group and cyclohexadienyl group.

Herein, an alkynyl group having 2 to 30 carbon atoms is linear or branched. Examples of the alkynyl group having 2 to 30 carbon atom include ethynyl, propynyl, and 2-phenylethynyl.

Herein, examples of a cycloalkynyl group having 3 to 30 carbon atoms include a cyclopentynyl group and cyclohexynyl group.

Herein, examples of a substituted sulfanyl group include a methyl sulfanyl group, phenyl sulfanyl group, diphenyl sulfanyl group, naphthyl sulfanyl group, and triphenyl sulfanyl group.

Herein, examples of a substituted sulfinyl group include a methyl sulfinyl group, phenyl sulfinyl group, diphenyl sulfinyl group, naphthyl sulfinyl group, and triphenyl sulfinyl group.

Herein, examples of a substituted sulfonyl group include a methyl sulfonyl group, phenyl sulfonyl group, diphenyl sulfonyl group, naphthyl sulfonyl group, and triphenyl sulfonyl group.

Herein, a substituted phosphanyl group is exemplified by a phenyl phosphanyl group.

Herein, examples of a substituted carbonyl group include a methyl carbonyl group, phenyl carbonyl group, diphenyl carbonyl group, naphthyl carbonyl group, and triphenyl carbonyl group.

Herein, an alkoxycarbonyl group having 2 to 30 carbon atoms is represented by -COOY'.Y' is exemplified by the above-described alkyl group.

Herein, a substituted carboxy group is exemplified by a benzoyloxy group.

Herein, an alkylthio group having 1 to 30 carbon atoms and an arylthio group having 6 to 30 ring carbon atoms are represented by -SRv. Examples of Rv include the above alkyl group having 1 to 30 carbon atoms and the above aryl group having 6 to 30 ring carbon atoms.The alkythio group preferably has 1 to 20 carbon atoms, and the arylthio group has 6 to 20 ring carbon atoms.

Examples of the halogen atom herein include a fluorine atom, chlorine atom, bromine atom and iodine atom, among which a fluorine atom is preferable.

Herein, "carbon atoms forming a ring (ring carbon atoms)" mean carbon atoms forming a saturated ring, unsaturated ring, or aromatic ring. "Atoms forming a ring (ring atoms)" mean carbon atoms and hetero atoms forming a hetero ring including a saturated ring, unsaturated ring, or aromatic ring.

Herein, a hydrogen atom includes isotope having different numbers of neutrons, specifically, protium, deuterium and tritium.

Herein, a substituent when referring to the "substituted or unsubstituted" is at least one group selected from the group consisting of an aryl group having 6 to 30 ring carbon atoms, heteroaryl group having 5 to 30 ring atoms, alkyl group (linear or branched alkyl group) having 1 to 30 carbon atoms, cycloalkyl group having 3 to 30 ring carbon atoms, alkyl halide group having 1 to 30 carbon atoms, alkylsilyl group having 3 to 30 carbon atoms, arylsilyl group having 6 to 30 ring carbon atoms, alkoxy group having 1 to 30 carbon atoms, aryloxy group having 5 to 30 carbon atoms, substituted amino group, alkylthio group having 1 to 30 carbon atoms, arylthio group having 6 to 30 ring carbon atoms, aralkyl group having 5 to 30 carbon atoms, alkenyl group having 2 to 30 carbon atoms, alkynyl group having 2 to 30 carbon atoms, halogen atom, cyano group, hydroxyl group, nitro group, and carboxy group.

Herein, the substituent when referring to the "substituted or unsubstituted" is preferably at least one group selected from the group consisting of an aryl group having 6 to 30 ring carbon atoms, heteroaryl group having 5 to 30 ring atoms, alkyl group (linear or branched alkyl group) having 1 to 30 carbon atoms, cycloalkyl group having 3 to 30 ring carbon atoms, alkyl halide group having 1 to 30 carbon atoms, halogen atom, alkylsilyl group having 3 to 30 carbon atoms, arylsilyl group having 6 to 30 ring carbon atoms, and cyano group, more preferably the specific examples of the substituent that are rendered preferable in the description of each of the substituents.

Herein, the substituent when referring to the "substituted or unsubstituted" may be further substituted by at least one group selected from the group consisting of an aryl group having 6 to 30 ring carbon atoms, heteroaryl group having 5 to 30 ring atoms, alkyl group (linear or branched alkyl group) having 1 to 30 carbon atoms, cycloalkyl group having 3 to 30 carbon atoms, alkyl halide group having 1 to 30 carbon atoms, alkylsilyl group having 3 to 30 carbon atoms, arylsilyl group having 6 to 30 ring carbon atoms, alkoxy group having 1 to 30 carbon atoms, aryloxy group having 5 to 30 carbon atoms, substituted amino group, alkylthio group having 1 to 30 carbon atoms, arylthio group having 6 to 30 ring carbon atoms, aralkyl group having 5 to 30 carbon atoms, alkenyl group having 2 to 30 carbon atoms, alkynyl group having 2 to 30 carbon atoms, halogen atom, cyano group, hydroxyl group, nitro group, and carboxy group. In addition, plural ones of these substituents may be mutually bonded to form a ring.

Herein, a substituent further substituting the substituent when referring to the "substituted or unsubstituted" is preferably at least one group selected from the group consisting of an aryl group having 6 to 30 ring carbon atoms, heteroaryl group having 5 to 30 ring atoms, alkyl group (linear or branched alkyl group) having 1 to 30 carbon atoms, halogen atom, and cyano group, more preferably the specific examples of the substituent that are rendered preferable in the description of each of the substituents.

"Unsubstituted" in "substituted or unsubstituted" means that a group is not substituted by the above-described substituents but bonded with a hydrogen atom.

Herein, "XX to YY carbon atoms" in the description of "substituted or unsubstituted ZZ group having XX to YY carbon atoms" represent carbon atoms of an unsubstituted ZZ group and do not include carbon atoms of a substituent(s) of a substituted ZZ group.

Herein, "XX to YY atoms" in the description of "substituted or unsubstituted ZZ group having XX to YY atoms" represent atoms of an unsubstituted ZZ group and does not include atoms of a substituent(s) of a substituted ZZ group.

The same description as the above applies to "substituted or unsubstituted" as for a compound or a partial structure thereof described herein.

Herein, when substituents are mutually bonded to form a ring, the ring in structure is a saturated ring, unsaturated ring, aromatic hydrocarbon ring, or a heterocyclic ring.

Herein, examples of the aromatic hydrocarbon group and the heterocyclic group in the linking group include divalent or higher-valent groups obtained by eliminating at least one hydrogen atom from the above monovalent groups.

The organic EL device of the exemplary embodiment emits light in a blue wavelength region with a high efficiency.

## Electronic Device

The organic EL device 1 according to the exemplary embodiment of the invention is usable in an electronic device such as a display unit and a light-emitting unit. Examples of the display unit include display components such as

an organic EL panel module, TV, mobile phone, tablet, and personal computer. Examples of the light-emitting unit include an illuminator and a vehicle light.

Second Exemplary Embodiment

An arrangement of an organic EL device according to a second exemplary embodiment will be described below. In the description of the second exemplary embodiment, the same components as those in the first exemplary embodiment are denoted by the same reference signs and names to simplify or omit an explanation of the components. In the second exemplary embodiment, the same materials and compounds as described in the first exemplary embodiment are usable for a material and a compound which are not particularly described.
The organic EL device according to the second exemplary embodiment is different from the organic EL device according to the first exemplary embodiment in that the emitting layer further includes a third compound. Other components are the same as those in the first exemplary embodiment.

Third Compound

A singlet energy $S_1(M3)$ of the third compound and the singlet energy $S_1(M1)$ of the first compound preferably satisfy a relationship of a numerical formula (Numerical Formula 2) below.

$$S_1(M3) > S_1(M1) \quad \text{(Numerical Formula 2)}$$

The third compound may be a compound exhibiting delayed fluorescence or a compound exhibiting no delayed fluorescence.
The third compound is also preferably a host material (occasionally referred to as a matrix material). When the first compound and the third compound are the host materials, one of the compounds may be referred to as a first host material and the other of the compounds may be referred to as a second host material.
Although the third compound is not particularly limited, the third compound is preferably a compound other than an amine compound. For instance, a carbazole derivative, dibenzofuran derivative and dibenzothiophene derivative are usable as the third compound. However, the third compound is not limited thereto.
The third compound also preferably has at least one of a partial structure represented by a formula (31) below or a partial structure represented by a formula (32) below in one molecule.

[Formula 92]

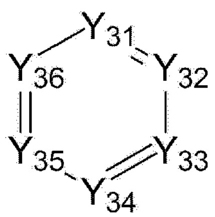

(31)

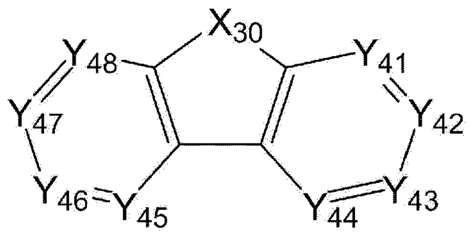

(32)

In the formula (31): $Y_{31}$ to $Y_{36}$ each independently represent a nitrogen atom or a carbon atom bonded to another atom in the molecule of the third compound.
However, at least one of $Y_{31}$ to $Y_{36}$ is a carbon atom bonded to another atom in the molecule of the third compound.
In the formula (32), $Y_{41}$ to $Y_{48}$ each independently represent a nitrogen atom or a carbon atom bonded to another atom in the molecule of the third compound.
However, at least one of $Y_{41}$ to $Y_{48}$ is a carbon atom bonded to another atom in the molecule of the third compound.
$X_{30}$ represents a nitrogen atom, an oxygen atom or a sulfur atom.
In the formula (32), it is also preferable that at least two of $Y_{41}$ to $Y_{48}$ are carbon atoms bonded to another atom in the molecule of the third compound and a cyclic structure is formed including the carbon atoms.
For instance, the partial structure represented by the formula (32) is preferably a partial structure selected from

the group consisting of partial structures represented by formulae (321), (322), (323), (324), (325) and (326) below.

[Formula 93]

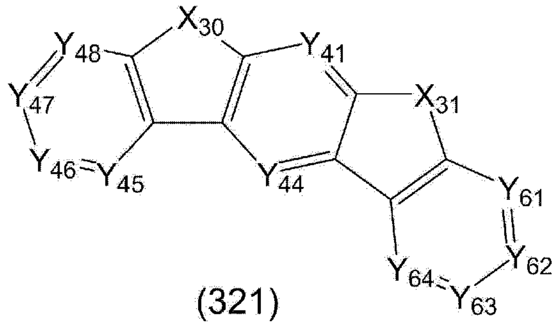

(321)

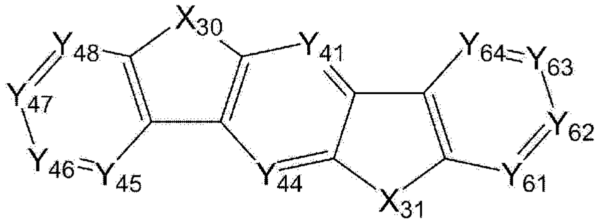

(322)

[Formula 94]

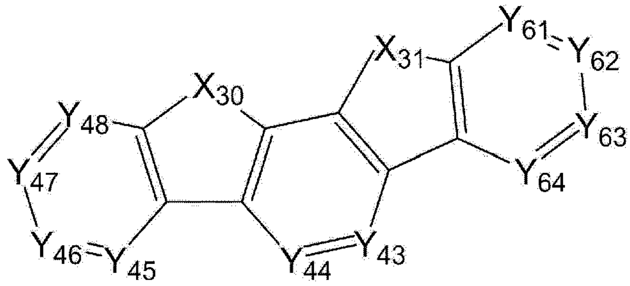

(323)

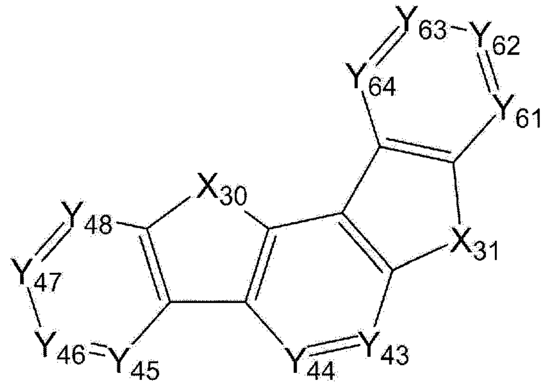

(324)

[Formula 95]

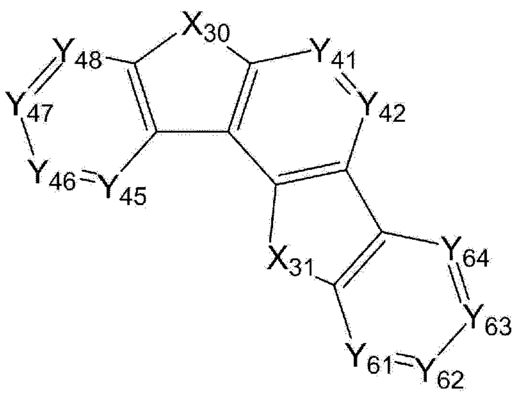

(325)

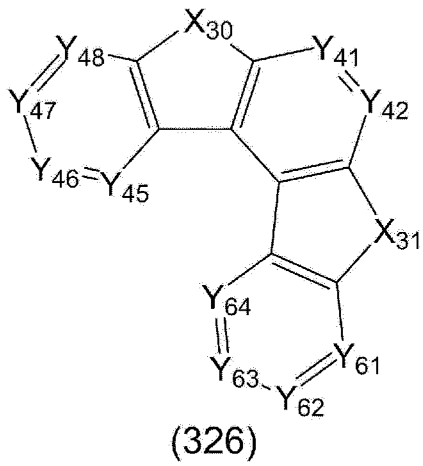

(326)

In the above formulae (321) to (326), $X_{30}$ each independently represents a nitrogen atom, an oxygen atom or a sulfur atom.

Y41 to Y48 each independently represent a nitrogen atom or a carbon atom bonded to another atom in the molecule of the third compound.

$X_{31}$ each independently represents a nitrogen atom, an oxygen atom, a sulfur atom or a carbon atom.

$Y_{61}$ to $Y_{64}$ each independently represent a nitrogen atom or a carbon atom bonded to another atom in the molecule of the third compound.

In the exemplary embodiment, the third compound preferably includes the partial structure represented by the formula (323) among the formulae (321) to (326).

The partial structure represented by the formula (31) is preferably in the form of at least one group selected from the group consisting of groups represented by formulae (33) and (34) below and contained in the third compound.

The third compound preferably includes at least one of the partial structure represented by the formula (33) and the partial structure represented by the formula (34).Since bonding positions are situated in meta positions as shown in the partial structures represented by the formulae (33) and (34), the energy gap $T_{77K}$(M3) at 77 [K] of the third compound can be kept high.

[Formula 96]

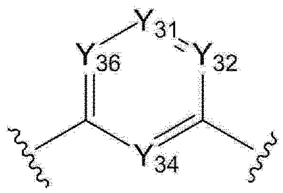

(33)

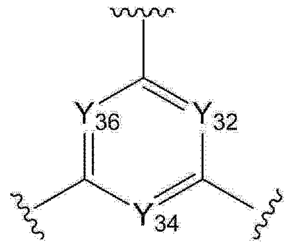

(34)

In the above formulae (33) and (34), $Y_{31}$, $Y_{32}$, $Y_{34}$ and $Y_{36}$ each independently represent a nitrogen atom or $CR_{31}$. $R_{31}$ is a hydrogen atom or a substituent. $R_{31}$ serving as the substituent is each independently selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 carbon atoms, a substituted or unsubstituted aralkyl group having 7 to 30 carbon atoms, a substituted or unsubstituted silyl group, a substituted germanium group, a substituted phosphine oxide group, a halogen atom, a cyano group, a nitro group, and a substituted or unsubstituted carboxy group.

The substituted or unsubstituted aryl group having 6 to 30 ring carbon atoms in $R_{31}$ is preferably a non-fused ring.

Wavy lines in the formulae (33) and (34) each show a bonding position with another atom or another structure in the molecule of the third compound.

In the formula (33), $Y_{31}$, $Y_{32}$, $Y_{34}$ and $Y_{36}$ are preferably each independently $CR_{31}$. A plurality of $R_{31}$ may be the same or different.

In the formula (34), $Y_{32}$, $Y_{34}$ and $Y_{36}$ are preferably each independently $CR_{31}$. A plurality of $R_{31}$ may be the same or different.

The substituted germanium group is preferably represented by - $Ge(R_{301})_3$.$R_{301}$ are each independently a substituent. The substituent $R_{301}$ is preferably a substituted or unsubstituted alkyl group having 1 to 30 carbon atoms or a substituted or unsubstituted aryl group having 6 to 30 ring carbon atoms. A plurality of $R_{301}$ are mutually the same or different.

The partial structure represented by the formula (32) is preferably in the form of at least one group selected from the group consisting of groups represented by formulae (35) to (39) and (30a) below and contained in the third compound.

[Formula 97]

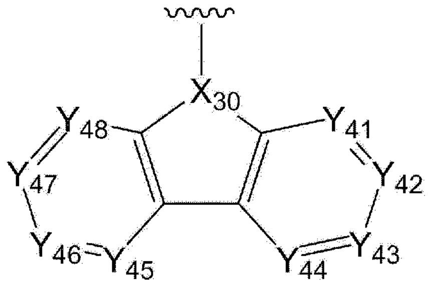

(35)

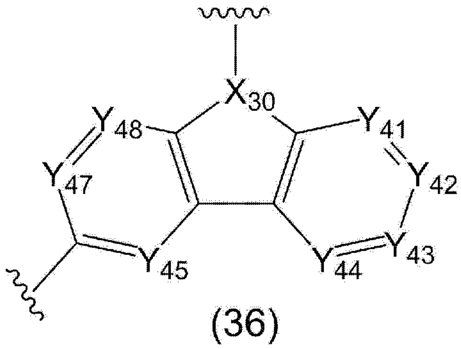

(36)

[Formula 98]

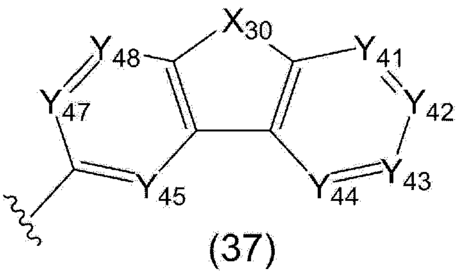

(37)

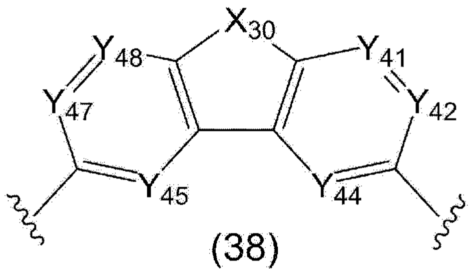

(38)

[Formula 99]

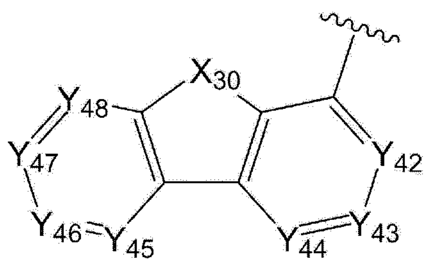

(39)

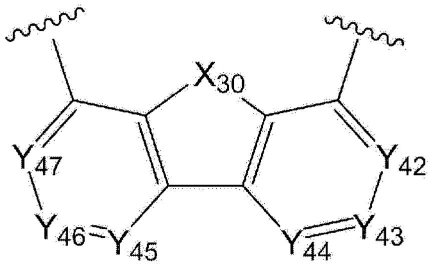

(30a)

In the formulae (35) to (39) and (30a), $Y_{41}$ to $Y_{48}$ each independently represent a nitrogen atom or $CR_{32}$.

$R_{32}$ is each independently a hydrogen atom or a substituent.

$R_{32}$ serving as the substituent is selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 carbon atoms, a substituted or unsubstituted aralkyl group having 7 to 30 carbon atoms, a substituted or unsubstituted silyl group, a substituted germanium group, a substituted phosphine oxide group, a halogen atom, a cyano group, a nitro group, and a substituted or unsubstituted carboxy group.

A plurality of $R_{32}$ are mutually the same or different.

$X_{30}$ in the formulae (35) and (36) represents a nitrogen atom.

In the formulae (37) to (39) and (30a), $X_{30}$ represents $NR_{33}$, an oxygen atom or a sulfur atom.

$R_{33}$ is selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 carbon atoms, a substituted or unsubstituted aralkyl group having 7 to 30 carbon atoms, a substituted or unsubstituted silyl group, a substituted germanium group, a substituted phosphine oxide group, a fluorine atom, a cyano group, a nitro group, and a substituted or unsubstituted carboxy group.

A plurality of $R_{33}$ are mutually the same or different.

The substituted or unsubstituted aryl group having 6 to 30 ring carbon atoms in $R_{33}$ is preferably a non-fused ring.

Wavy lines in the formulae (35) to (39) and (30a) each show a bonding position with another atom or another structure in the molecule of the third compound.

In the formula (35), $Y_{41}$ to $Y_{48}$ are preferably each independently $CR_{32}$. In the formulae (36) and (37), $Y_{41}$ to $Y_{45}$, $Y_{47}$ and $Y_{48}$ are preferably each independently $CR_{32}$. In the formula (38), $Y_{41}$, $Y_{42}$, $Y_{44}$, $Y_{45}$, $Y_{47}$ and $Y_{48}$ are preferably each independently $CR_{32}$. In the formula (39), $Y_{42}$ to $Y_{48}$ are preferably each independently $CR_{32}$. In the formula (30a), $Y_{42}$ to $Y_{47}$ are preferably each independently $CR_{32}$. A plurality of $R_{32}$ may be the same or different.

In the third compound, $X_{30}$ is preferably an oxygen atom or a sulfur atom, more preferably an oxygen atom.

In the third compound, $R_{31}$ and $R_{32}$ each independently represent a hydrogen atom or a substituent. $R_{31}$ and $R_{32}$ serving as the substituent are preferably each independently a group selected from the group consisting of a fluorine atom, a cyano group, a substituted or unsubstituted alkyl group having 1 to 30 carbon atoms, a substituted or unsubstituted aryl group having 6 to 30 ring carbon atoms, and a substituted or unsubstituted heteroaryl group having 5 to 30 ring atoms. $R_{31}$ and $R_{32}$ are more preferably a hydrogen atom, a cyano group, a substituted or unsubstituted aryl group having 6 to 30 ring carbon atoms, or a substituted or unsubstituted heteroaryl group having 5 to 30 ring atoms. When $R_{31}$ and $R_{32}$ serving as the substituent are each a substituted or unsubstituted aryl group having 6 to 30 ring carbon atoms, the aryl group is preferably a non-fused ring.

The third compound is also preferably an aromatic hydrocarbon compound or an aromatic heterocyclic compound. It is also preferable that the third compound has no fused aromatic hydrocarbon ring in a molecule.

Method of Preparing Third Compound

The third compound may be prepared by a method described in International Publication Nos. WO2012/153780, WO2013/038650 and the like. Moreover, the third compound can be exemplarily prepared with use of a known alternative reaction and a starting material depending on a target object.

Examples of the substituent in the third compound are shown below, but the invention is not limited to the examples.

Specific examples of the aryl group (occasionally referred to as an aromatic hydrocarbon group) include a phenyl group, tolyl group, xylyl group, naphthyl group, phenanthryl group, pyrenyl group, chrysenyl group, benzo[c]phenanthryl group, benzo[g]chrysenyl group, benzoanthryl group, triphenylenyl group, fluorenyl group, 9,9-dimethylfluorenyl group, benzofluorenyl group, dibenzofluorenyl group, biphenyl group, terphenyl group, quarterphenyl group and fluoranthenyl group, among which a phenyl group, biphenyl group, terphenyl group, quarterphenyl group, naphthyl group, triphenylenyl group and fluorenyl group may be preferable.

Specific examples of the substituted aryl group include a tolyl group, xylyl group and 9,9-dimethylfluorenyl group.

As is understood from the specific examples, the aryl group includes both fused aryl group and non-fused aryl group.

Preferable examples of the aryl group include a phenyl group, biphenyl group, terphenyl group, quarterphenyl group, naphthyl group, triphenylenyl group and fluorenyl group.

Specific examples of the heteroaryl group (occasionally referred to as a heterocyclic group, heteroaromatic ring group or aromatic heterocyclic group) include a pyrrolyl group, pyrazolyl group, pyrazinyl group, pyrimidinyl group, pyridazynyl group, pyridyl group, triazinyl group, indolyl group, isoindolyl group, imidazolyl group, benzimidazolyl group, indazolyl group, imidazo[1,2-a]pyridinyl group, furyl group, benzofuranyl group, isobenzofuranyl group, dibenzofuranyl group, azadibenzofuranyl group, thienyl group, benzothienyl group, dibenzothienyl group, azadibenzothienyl group, quinolyl group, isoquinolyl group, quinoxalinyl group, quinazolinyl group, naphthyridinyl group, carbazolyl group, azacarbazolyl group, phenanthridinyl group, acridinyl group, phenanthrolinyl group, phenazinyl group, phenothiazinyl group, phenoxazinyl group, oxazolyl group, oxadiazolyl group, furazanyl group, benzoxazolyl group, thienyl group, thiazolyl group, thiadiazolyl group, benzothiazolyl group, triazolyl group and tetrazolyl group, among which a dibenzofuranyl group, dibenzothienyl group, carbazolyl group, pyridyl group, pyrimidinyl group, triazinyl group, azadibenzofuranyl group and azadibenzothienyl group are preferable.

Preferable examples of the heteroaryl group include a dibenzofuranyl group, dibenzothienyl group, carbazolyl group, pyridyl group, pyrimidinyl group, triazinyl group, azadibenzofuranyl group or azadibenzothienyl group. Further preferable examples of the heteroaryl group include a dibenzofuranyl group, dibenzothienyl group, azadibenzofuranyl group and azadibenzothienyl group.

In the third compound, the substituted silyl group is also preferably selected from the group consisting of a substituted or unsubstituted trialkylsilyl group, a substituted or unsubstituted arylalkylsilyl group, or a substituted or unsubstituted triarylsilyl group.

Specific examples of the substituted or unsubstituted trialkylsilyl group include a trimethylsilyl group and a triethylsilyl group.

Specific examples of the substituted or unsubstituted arylalkylsilyl group include a diphenylmethylsilyl group, ditolylmethylsilyl group, and phenyldimethylsilyl group.

Specific examples of the substituted or unsubstituted triaryllsilyl group include a triphenylsilyl group and a tritolylsilyl group.

In the third compound, the substituted phosphine oxide group is also preferably a substituted or unsubstituted diarylphosphine oxide group.

Specific examples of the substituted or unsubstituted diarylphosphine oxide group include a diphenylphosphine oxide group and ditolylphosphine oxide group.

Relationship between First Compound, Second Compound and Third Compound in Emitting Layer

The first compound, the second compound, and the third compound in the emitting layer preferably satisfy the relationships represented by the numerical formulae (Numerical Formulae 1 and 2). Specifically, the first compound, the second compound, and the third compound in the emitting layer preferably satisfy a relationship represented by a numerical formula below (Numerical Formula 3).

$$S_1(M3) > S_1(M1) > S_1(M2) \quad \text{(Numerical Formula 3)}$$

The energy gap $T_{77K}(M3)$ at 77 [K] of the third compound is preferably larger than the energy gap $T_{77K}(M1)$ at 77 [K] of the first compound. Specifically, the energy gap $T_{77K}(M3)$ at 77 [K] of the third compound and the energy gap $T_{77K}(M1)$ at 77 [K] of the first compound preferably satisfy a relationship represented by a numerical formula below (Numerical Formula 5).

$$T_{77K}(M3) > T_{77K}(M1) \quad \text{(Numerical Formula 5)}$$

The first compound, the second compound, and the third compound in the emitting layer preferably satisfy the relationships represented by the numerical formulae (Numerical Formulae 4 and 5). Specifically, the first compound, the second compound, and the third compound in the emitting layer preferably satisfy a relationship represented by a numerical formula below (Numerical Formula 6).

$$T_{77K}(M3) > T_{77K}(M1) > T_{77K}(M2) \quad \text{(Numerical Formula 6)}$$

When the organic EL device in the exemplary embodiment emits light, it is preferable that the second compound mainly emits light in the emitting layer.

Content Ratio of Compounds in Emitting Layer

A content ratio between the first compound, the second compound and the third compound in the emitting layer is preferably in an exemplary range below.

The content ratio of the first compound is preferably in a range from 10 mass% to 80 mass%, more preferably in a range from 10 mass% to 60 mass%, particularly preferably in a range from 20 mass% to 60 mass%.

The content ratio of the second compound is preferably in a range from 0.01 mass% to 10 mass%, more preferably in a range from 0.01 mass% to 5 mass%, further preferably in a range from 0.01 mass% to 1 mass%.

A content ratio of the third compound is preferably in a range from 10 mass% to 80 mass%.

An upper limit of the total of the respective content ratios of the first to third compounds in the emitting layer is 100 mass%. It should be noted that the emitting layer of the exemplary embodiment may further contain another material in addition to the first to third compounds.

Fig. 5 exemplarily shows a relationship in energy levels between the first compound, the second compound and the third compound in the emitting layer. In Fig. 5, S0 represents a ground state. $S_1(M1)$ represents the lowest singlet state of the first compound. T1(M1) represents the lowest triplet state of the first compound. S1(M2) represents the lowest singlet state of the second compound. T1(M2) represents the lowest triplet state of the second compound. S1(M3) represents the lowest singlet state of the third compound. T1(M3) represents the lowest triplet state of the third compound. A dashed arrow directed from S1(M1) to S1(M2) in Fig. 5 represents Forster energy transfer from the lowest singlet state of the first compound to the lowest singlet state of the second compound.

As shown in Fig. 5, when a compound having a small $\Delta ST(M1)$ is used as the first compound, inverse intersystem

crossing from the lowest triplet state T1(M1) to the lowest singlet state $S_1$(M1) can be caused by a heat energy. Accordingly, Forster energy transfer from the lowest singlet state S1(M1) of the first compound to the second compound is caused to generate the lowest singlet state S1(M2). As a result, fluorescence from the lowest singlet state S1(M2) of the second compound is observable. It is considered that the internal quantum efficiency can be theoretically raised up to 100% also by using delayed fluorescence by the TADF mechanism.

The organic EL device of the second exemplary embodiment emits light in a blue wavelength region with a high efficiency.

The emitting layer of the organic EL device according to the second exemplary embodiment includes the delayed fluorescent first compound, the fluorescent second compound, and the third compound having a larger singlet energy than the first compound, whereby the luminous efficiency of the organic EL device is improved. It is deduced that the luminous efficiency is improved because a carrier balance in the emitting layer is improved by containing the third compound.

The organic EL device according to the second exemplary embodiment is usable in an electronic device such as a display unit and a light-emitting unit in the same manner as the organic EL device according to the first exemplary embodiment.

Modification of Embodiments

It should be noted that the invention is not limited to the above exemplary embodiments but may include any modification and improvement as long as such modification and improvement are compatible with the invention.

The emitting layer is not limited to a single layer. In some embodiments, the emitting layer is provided in a form of a laminate of a plurality of emitting layers. When the organic EL device has a plurality of emitting layers, it is only required that at least one of the emitting layers satisfies the conditions described in the above exemplary embodiments. In some embodiments, for instance, the rest of the emitting layers is a fluorescent emitting layer, or a phosphorescent emitting layer with use of emission caused by electron transfer from the triplet state directly to the ground state.

When the organic EL device includes the plurality of emitting layers, in some embodiments, for instance, the plurality of emitting layers are adjacent to each other, or provide a so-called tandem-type organic EL device in which a plurality of emitting units are layered through an intermediate layer.

In some embodiments, for instance, a blocking layer is provided in contact with at least one of an anode-side and a cathode-side of the emitting layer. The blocking layer is preferably provided in contact with the emitting layer to block holes, electrons, excitons or combinations thereof.

For instance, when the blocking layer is provided in contact with the cathode-side of the emitting layer, the blocking layer permits transport of electrons, but prevents holes from reaching a layer provided near the cathode (e.g., the electron transporting layer) beyond the blocking layer. When the organic EL device includes the electron transporting layer, the blocking layer is preferably interposed between the emitting layer and the electron transporting layer.

When the blocking layer is provided in contact with the emitting layer near the anode, the blocking layer permits transport of holes, but prevents electrons from reaching a layer provided near the anode (e.g., the hole transporting layer) beyond the blocking layer. When the organic EL device includes the hole transporting layer, the blocking layer is preferably interposed between the emitting layer and the hole transporting layer.

Further, in some embodiments, the blocking layer is provided in contact with the emitting layer to prevent an excitation energy from leaking from the emitting layer into a layer in the vicinity thereof. Excitons generated in the emitting layer are prevented from moving into a layer provided near the electrode (e.g., the electron transporting layer and the hole transporting layer) beyond the blocking layer.

The emitting layer and the blocking layer are preferably bonded to each other.

Specific structure and shape of the components for implementing the invention are alterable in any manner as long as an object of the invention is achievable.

Examples

Examples of the invention will be described below. However, the invention is not limited to Examples.

Compounds

Compounds used for preparing the organic EL device will be shown below.

[Formula 100]

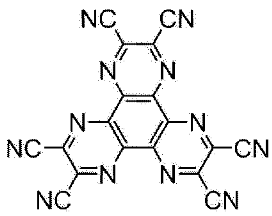

HI

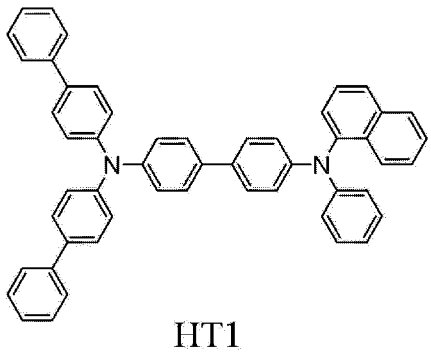

HT1

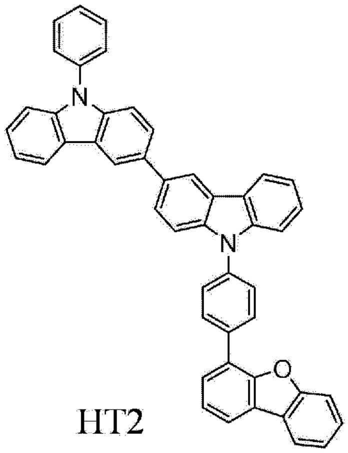

HT2

[Formula 101]

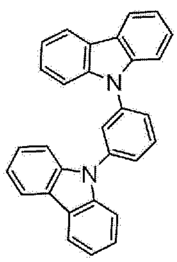

mCP

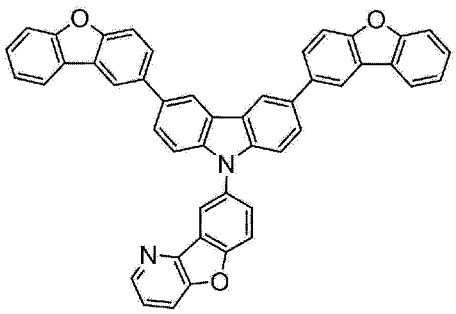

ET-1

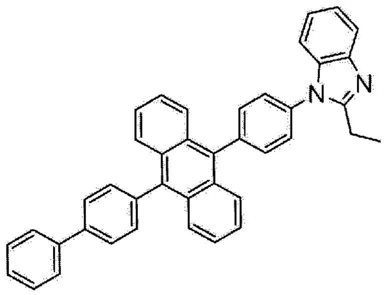

ET-2

[Formula 102]

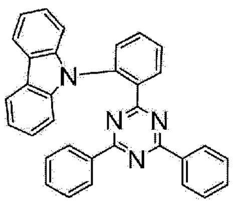

TADF-1

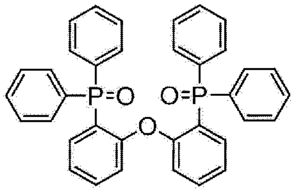

DPEPO

[Formula 103]

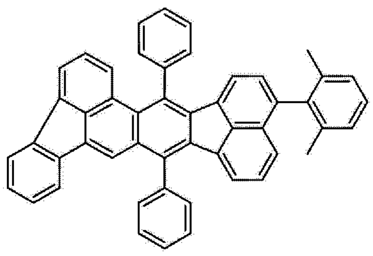

BD-1a

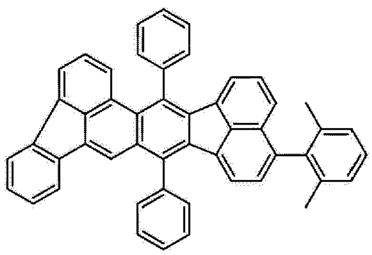

BD-1b

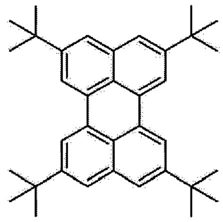

TBPe

[Formula 104]

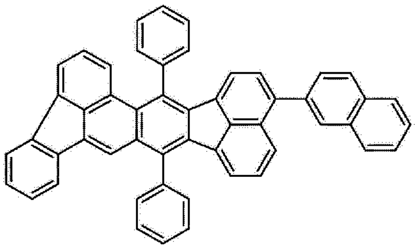

BD-2a

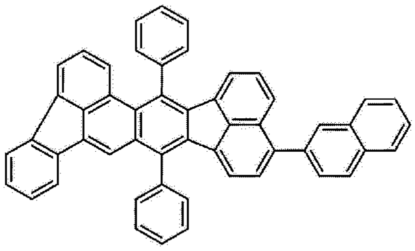

BD-2b

[Formula 105]

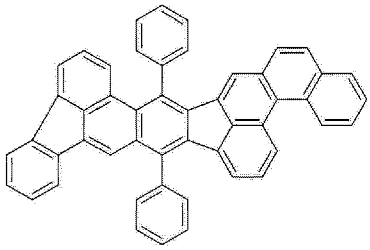

BD-3a

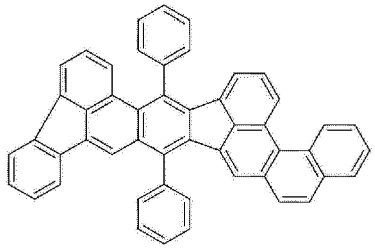

BD-3b

(Reference compounds):

[Formula 106]

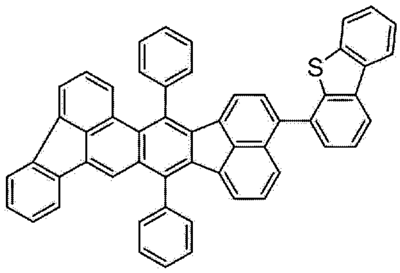

BD-4a

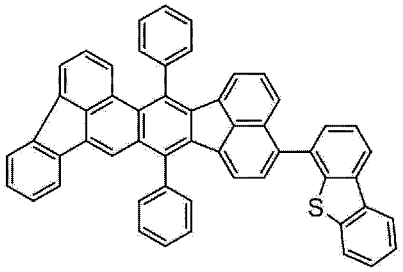

BD-4b

[Formula 107]

(Reference compounds):

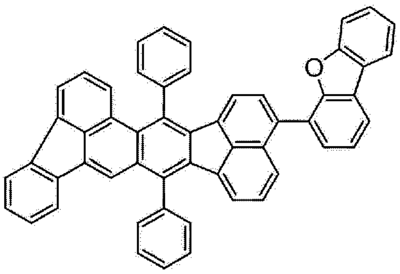

BD-5a

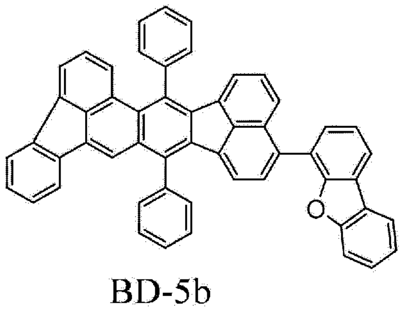

BD-5b

[Formula 108]

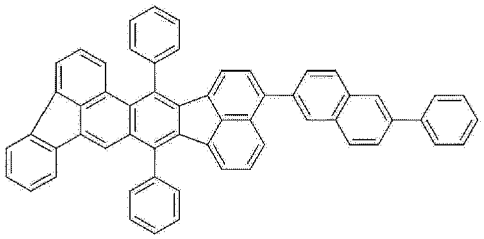

BD-6a

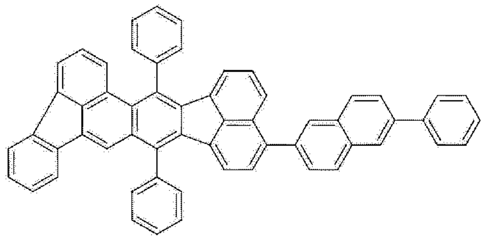

BD-6b

[Formula 109]

(Reference compounds):

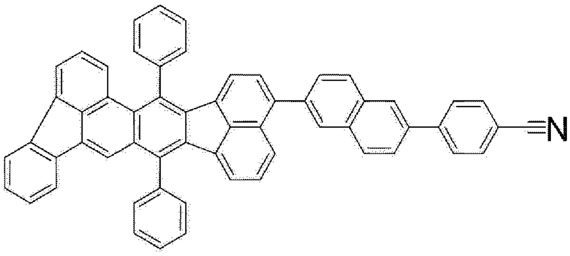

BD-7a

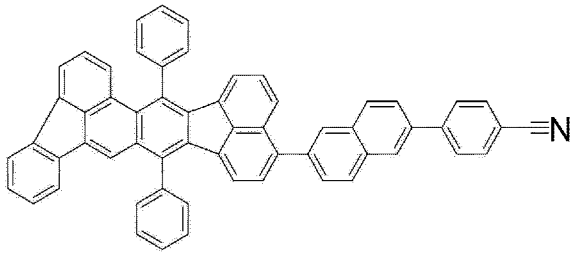

BD-7b

[Formula 110]

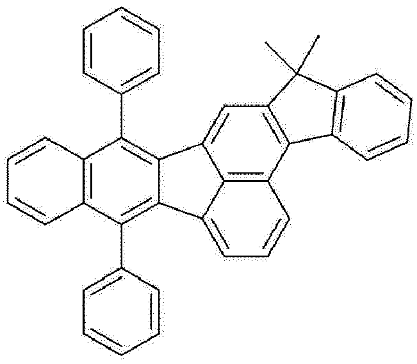

BD-8a

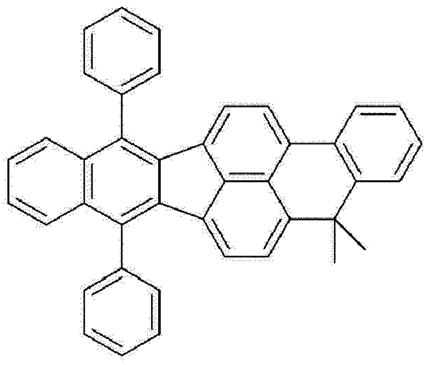

BD-8b

[Formula 111]

(Reference compound):

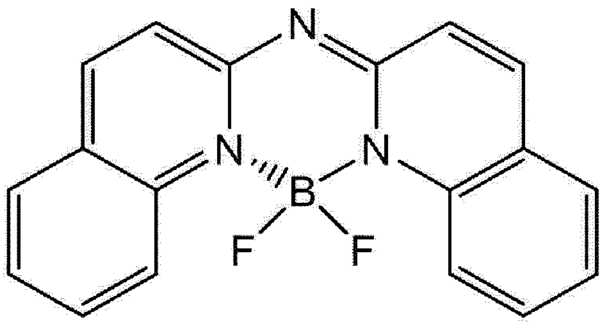

BD-9

[Formula 112]

(Reference compounds):

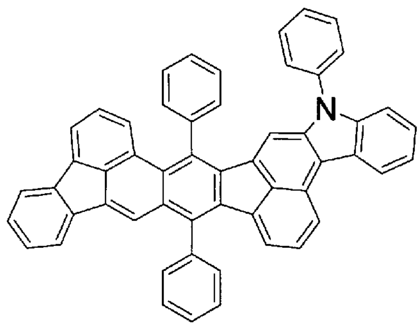

BD-10a

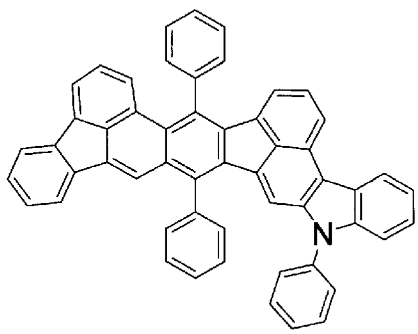

BD-10b

[Formula 113]

(Reference compounds):

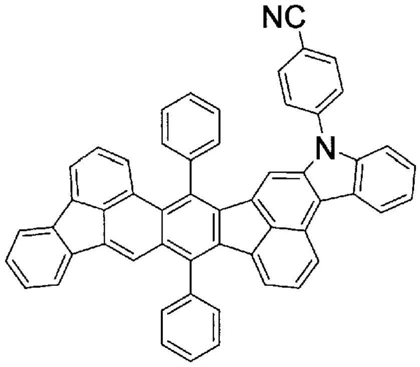

BD-11a

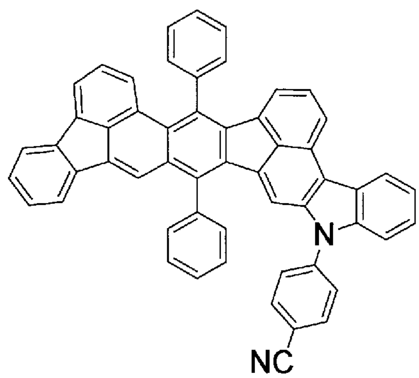

BD-11b

[Formula 114]

(Reference compounds):

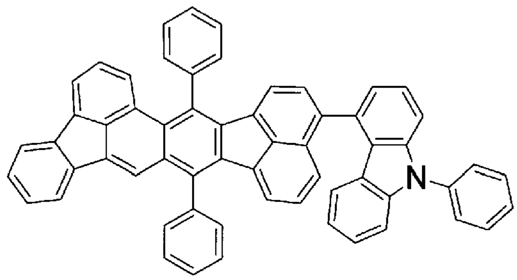

BD-12a

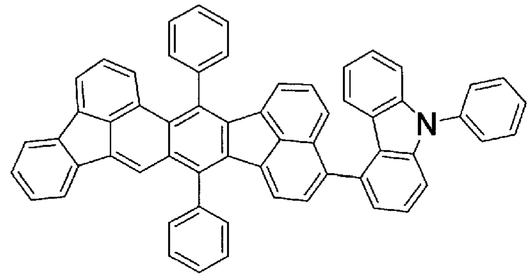

BD-12b

[Formula 115]

(Reference compounds):

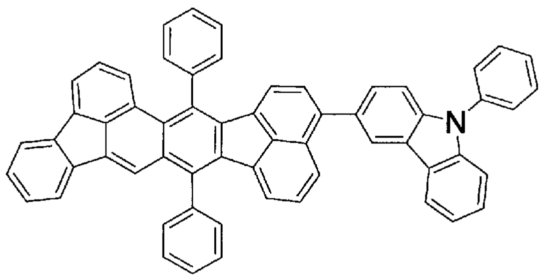

BD-13a

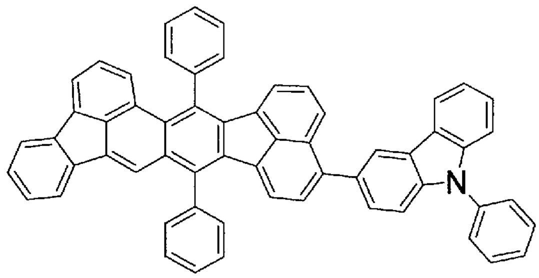

BD-13b

[Formula 116]

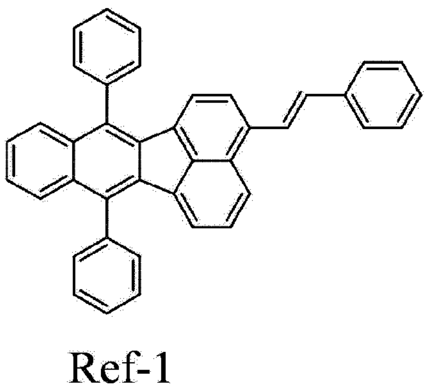

Ref-1

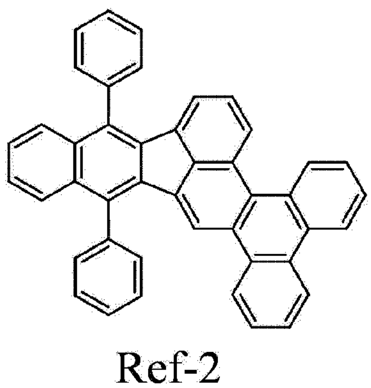

Ref-2

[Formula 117]

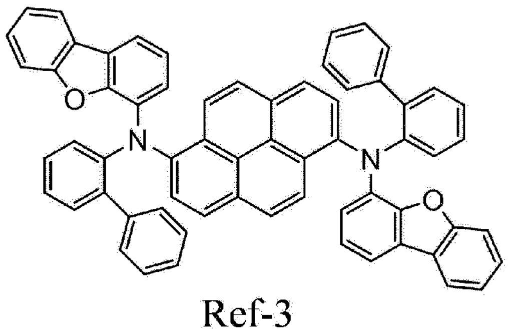

Ref-3

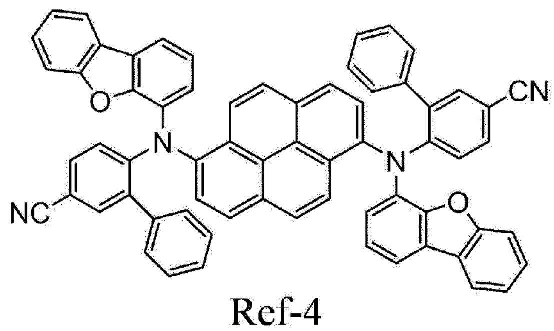

Ref-4

Synthesis of Compounds
Synthesis Example 1: Synthesis of Compound TADF-1
Synthesis of Intermediate A

[Formula 118]

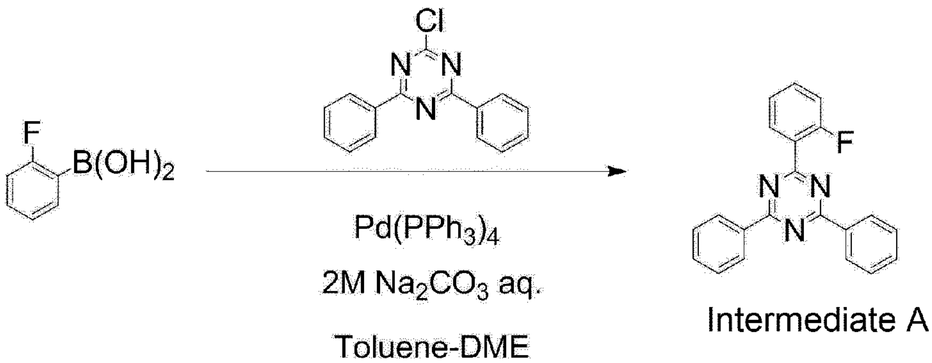


To a three-necked flask, 7.0 g (50 mmol) of 2-fluorophenylboronic acid, 13.4 g (50 mmol) of 2-chloro-4,6-diphenyltriazine, 62.5 mL of 2M sodium carbonate aqueous solution, 100 mL of 1,2-dimethoxyethane (DME), and 100 mL of toluene were added. Subsequently, 1.73 (1.5 mmol) of tetrakis(triphenylphosphine)palladium was further added to the three-necked flask and the obtained mixture was heated to reflux with stirring under argon atmosphere for eight hours. After the mixture was heated to reflux with stirring, an organic layer was separated from the mixture. The separated organic layer was condensed under reduced pressure. The obtained residue was purified by silica-gel column chromatography. A toluene solvent was used as an eluent. The solid obtained after the residue was purified was suspended and washed in methanol to obtain an intermediate A in a form of a white solid. A yield was 11.6 g and a yield rate was 71%.

(2) Synthesis of Compound TADF-1

[Formula 119]

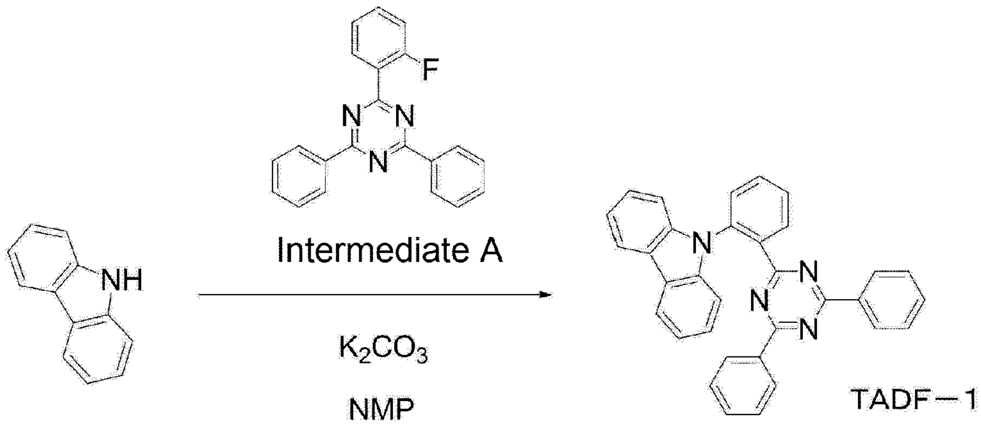

TADF−1

To a three-necked flask, 7.3 g (43.6 mmol) of carbazole, 8.0 g (24.4 mmol) of intermediate A, 7.4 g (53.5 mmol) of potassium carbonate, and 50 mL of N-methyl-2-pyrrolidone (NMP) were added and heated at 150 degree C with stirring under argon atmosphere for 20 hours. After heated with stirring, the reaction solution was poured into 200 mL of water and filtrated to obtain a deposited solid. Subsequently, the solid was repeatedly suspended and washed in ethanol to obtain a target (a compound TADF-1) in a form of a white solid. A yield was 6.3 g and a yield rate was 54%. As a result of FD-MS (Field Desorption Mass Spectrometry) analysis, m/e=474 was relative to a molecular weight of 474.

Synthesis Example 2: Synthesis of Compound BD-1 and Compound BD-2

A compound BD-1 was synthesized according to a method described in JP2010-270103A. As a result, the compound BD-1 was a mixture containing the compound BD-1a and the compound BD-1b.
The compound BD-2 was also synthesized according to the method described in JP2010-270103A. As a result, the compound BD-2 was a mixture containing the compound BD-2a and the compound BD-2b.

Synthesis Example 3: Synthesis of Compound BD-3

Synthesis of Intermediate (Int-1)

[Formula 120]

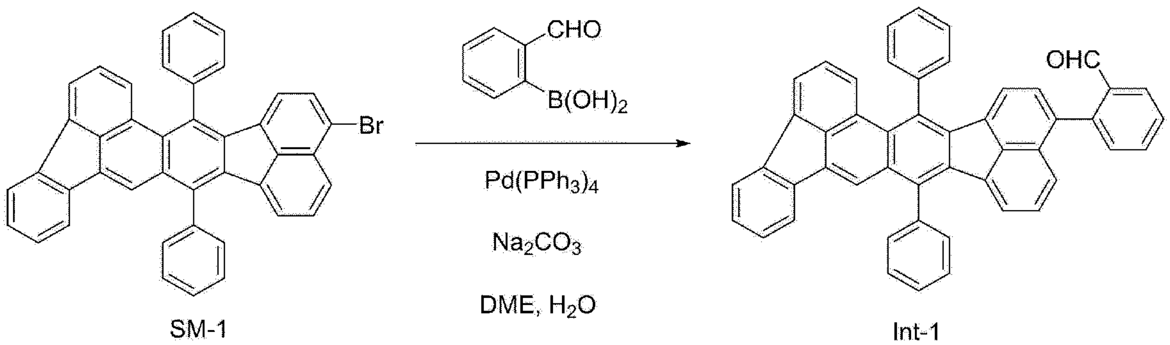

SM-1
Int-1

To a three-necked flask, 2.0 g (3.29 mmol) of a starting material (SM-1: isomer mixture), 0.59 g (3.95 mmol) of 2-formylphenylboronic acid, 0.87 g (8.23 mmol) of sodium carbonate, 10 mL of 1,2-dimethoxyethane (DME), and 10 mL of water were added, to which 0.19 g (0.17 mmol) tetrakis(triphenylphosphine)palladium was further added. The obtained mixture was heated to reflux with stirring under argon atmosphere for eight hours. After the mixture was heated to reflux with stirring, an organic layer was separated from the mixture. The separated organic layer was condensed under reduced pressure. The obtained residue was purified by silica-gel column chromatography. A mixture solvent of dichloromethane and n-hexane was used as an eluent. The solid obtained after the residue was purified was suspended

and washed in methanol to obtain an intermediate (Int-1).A yield was 2.1 g and a yield rate was 100%.

(2) Synthesis of Intermediate (Int-2)

[Formula 121]

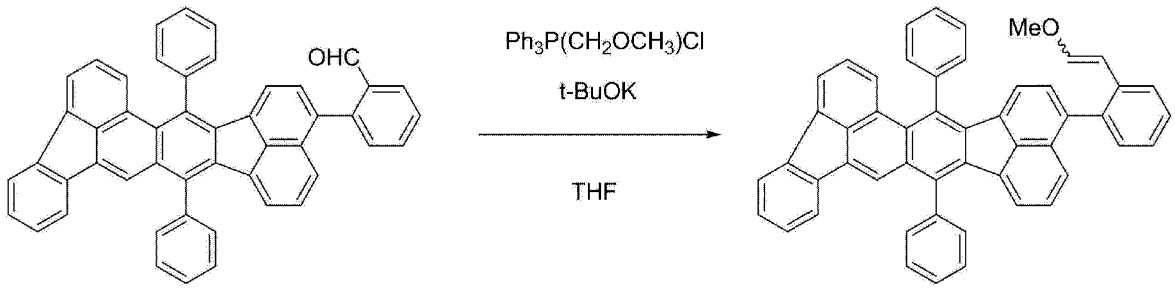


Int-1 Int-2

To a three-necked flask, 1.7 g (4.98 mmol) of (methoxymethyl)triphenylphosphonium chloride and 3.5 mL of tetrahydrofuran (THF) were added, to which 0.61 g (5.48 mmol) of potassium t-butoxide was further added, thereby preparing a red ylide solution. A THF solution (5mL) of 2.1 g (3.29 mmol) of the intermediate (Int-1) was further added to the ylide solution and stirred under argon atmosphere for two hours. Water was added to the reaction solution to stop the reaction, and subsequently, an organic layer was extracted with dichloromethane. The extracted organic layer was condensed under reduced pressure. The obtained residue was purified by silica-gel column chromatography. A mixture solvent of dichloromethane and n-hexane was used as an eluent. The solid obtained after the residue was purified was suspended and washed in methanol to obtain an intermediate (Int-2).A yield was 1.8 g and a yield rate was 82%.

(3) Synthesis of Compound BD-3

[Formula 122]

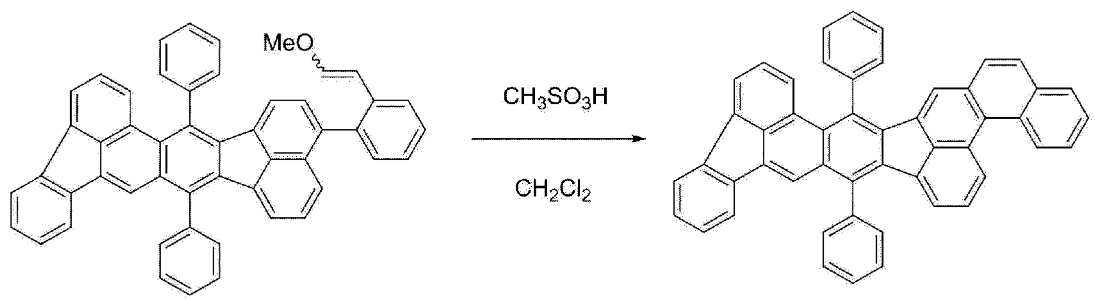


Int-2 BD-3

To a three-necked flask, 1.8 g (2.72 mmol) of intermediate (Int-2) and 25 mL of dichloromethane were added, to which 0.26 g (2.72 mmol) of methanesulfonic acid was dropped and stirred for 0.5 hours. A sodium hydrogen carbonate solution was added to the reaction solution to stop the reaction, and subsequently, an organic layer was extracted with dichloromethane. The extracted organic layer was condensed under reduced pressure. The obtained residue was purified by silica-gel column chromatography. A mixture solvent of dichloromethane and n-hexane was used as an eluent. The solid obtained after the residue was purified was suspended and washed in methanol to obtain a target (compound BD-3) in a form of a yellow solid. A yield was 0.84 g and a yield rate was 49%. As a result of FD-MS (Field Desorption Mass Spectrometry) analysis, m/e=628 was relative to a molecular weight of 628. Derived from the starting material (SM-1) that was an isomer mixture (a mixture containing SM-1a and SM-1b), the compound BD-3 was a mixture containing BD-3a and BD-3b. It should be noted that only a structure of SM-1a was exemplarily shown as "SM-1" and only a structure

of BD-3a was exemplarily shown as "BD-3" in a reaction formula for Synthesis Example 3. Only intermediates obtained from SM-1a were exemplarily shown as the intermediate Int-1 and the intermediate Int-2.

[Formula 123]

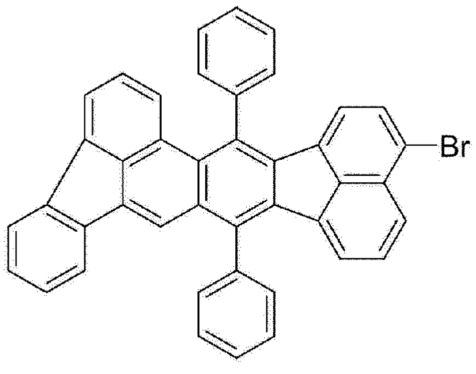

SM-1a

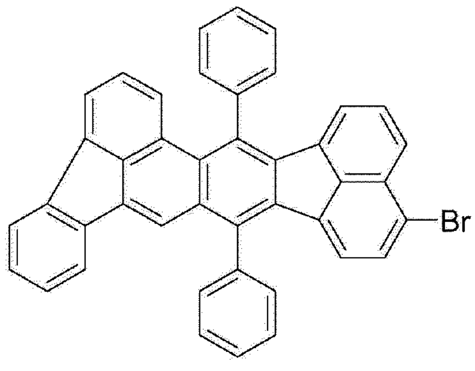

SM-1b

Synthesis Example 4: Synthesis of Compound BD-4

[Formula 124]

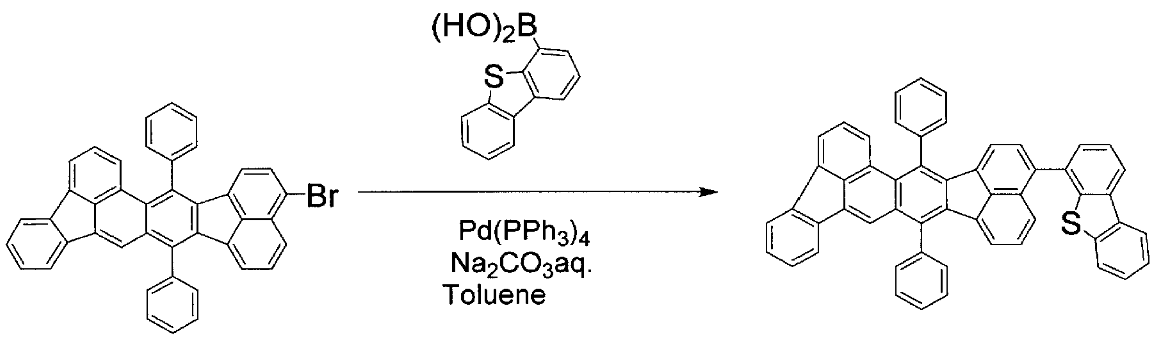


SM-1

BD-4

To a three-necked flask, 1.0 g (1.65 mmol) of a starting material (SM-1: isomer mixture), 0.45 g (1.98 mmol) of dibenzothiophene-4-boronic acid, 2.5 mL of 2M sodium carbonate aqueous solution, 20 mL of toluene were added, to which 0.08 g (0.07 mmol) of tetrakis(triphenylphosphine)palladium was further added, and heated with stirring at 90 degrees C under nitrogen atmosphere for seven hours. After heated with stirring, the reaction solution was returned to the room temperature and filtrated to obtain a deposited solid. The obtained solid was dissolved in toluene, to which silica gel was further added and stirred. Subsequently, the reaction solution was filtrated and condensed to obtain a compound BD-4.A yield was 0.62 g and a yield rate was 52%.

A result of FD-MS analysis shows m/e=710 relative to a molecular weight of 710. Derived from the starting material (SM-1) that was an isomer mixture (a mixture containing SM-1a and SM-1b), the compound BD-4 was a mixture containing BD-4a and BD-4b. It should be noted that only a structure of SM-1a was exemplarily shown as "SM-1" and only a structure of BD-4a was exemplarily shown as "BD-4" in a reaction formula for Synthesis Example 4.

Synthesis Example 5: Synthesis of Compound BD-5

[Formula 125]

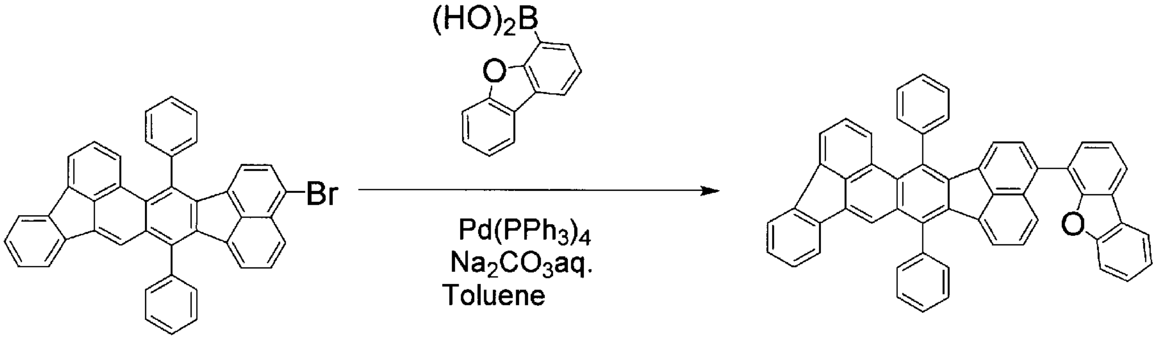


SM-1 BD-5

To a three-necked flask, 2.0 g (3.29 mmol) of a starting material (SM-1: isomer mixture), 0.84 g (3.95 mmol) of dibenzofuran-4-boronic acid, 5 mL of 2M sodium carbonate aqueous solution, 20 mL of toluene were added, to which 0.16 g (0.14 mmol) of tetrakis(triphenylphosphine)palladium was further added, and heated with stirring at 90 degrees C under nitrogen atmosphere for seven hours. After heated with stirring, the reaction solution was returned to the room temperature and filtrated to obtain a deposited solid. The obtained solid was dissolved in toluene, to which silica gel was further added and stirred. Subsequently, the reaction solution was filtrated and condensed to obtain a compound BD-5. A yield was 2.08 g and a yield rate was 91%.

A result of FD-MS analysis shows m/e=694 relative to a molecular weight of 694. Derived from the starting material (SM-1) that was an isomer mixture (a mixture containing SM-1a and SM-1b), the compound BD-5 was a mixture containing BD-5a and BD-5b. It should be noted that only a structure of SM-1a was exemplarily shown as "SM-1" and only a structure of BD-5a was exemplarily shown as "BD-5" in a reaction formula for Synthesis Example 5.

Synthesis Example 6: Synthesis of Compound BD-6

[Formula 126]

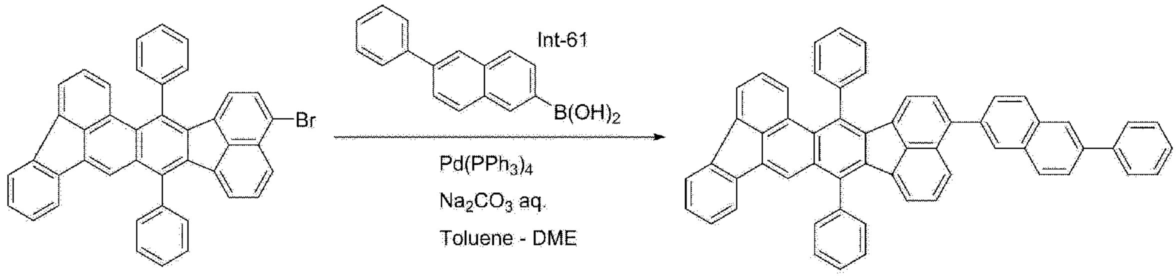


SM-1 BD-6

To a three-necked flask, 2.0 g (3.29 mmol) of SM-1 (isomer mixture), 1.31 g (5.28 mmol) of boronic acid (Int-61), 3.3 mL of 2M sodium carbonate aqueous solution, 22 mL of 1,2-dimethoxyethane (DME), and 11 mL of toluene were added, to which 0.08 g (0.07 mmol) tetrakis(triphenylphosphine)palladium was further added. The obtained mixture was heated to reflux with stirring under nitrogen atmosphere for 24 hours. After heated to reflux with stirring, the reaction solution was cooled to the room temperature and filtrated to obtain a deposited solid. The obtained solid was suspended and washed in a mixture solvent of toluene and methanol and subsequently suspended and washed in ethyl acetate to obtain a target (compound BD-6).A yield was 0.77 g and a yield rate was 32%. A result of FD-MS analysis shows m/e=730 relative to a molecular weight of 730. Derived from the starting material (SM-1) that was an isomer mixture (a mixture containing SM-1a and SM-1b), the compound BD-6 was a mixture containing BD-6a and BD-6b. It should be noted that only a structure of SM-1a was exemplarily shown as "SM-1" and only a structure of BD-6a was exemplarily shown as "BD-6" in a reaction formula for Synthesis Example 6.

Synthesis Example 7: Synthesis of Compound BD-7

[Formula 127]

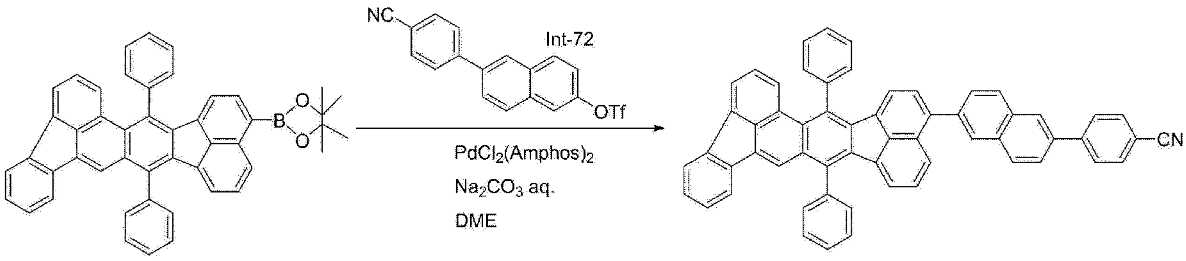


SM-72 BD-7

Int-72 was synthesized by replacing boronic acid in the method of Synthesis Example 10 of International Publication No. 2011/086935 with p-cyanophenylboronic acid.

To a three-necked flask, 0.66 g (1.00 mmol) of SM-72 (isomer mixture), 0.38 g (1.00 mmol) of Int-72, 1 mL of 2M sodium carbonate aqueous solution, and 10 mL of 1,2-dimethoxyethane (DME) were added, to which 28 mg (0.04 mmol) of $PdCl_2$ $(Amphos)_2$ was further added. The obtained mixture was heated to reflux with stirring under nitrogen atmosphere for 24 hours. After heated to reflux with stirring, the reaction solution was cooled to the room temperature and filtrated to obtain a deposited solid. The obtained solid was purified by silica-gel column chromatography (eluent: toluene). After purified, the obtained solid was suspended and washed in methanol to obtain a target (compound BD-7).A yield was 0.61 g and a yield rate was 80%. A result of FD-MS analysis shows m/e=755 relative to a molecular weight of 755. Derived from the starting material (SM-72) that was an isomer mixture (a mixture containing SM-72a and SM-72b), the compound BD-7 was a mixture containing BD-7a and BD-7b. It should be noted that only a structure of SM-72a was exemplarily shown as "SM-72" and only a structure of BD-7a was exemplarily shown as "BD-7" in a reaction formula for Synthesis Example 7. "-OTf" is an abbreviation of a trifluoromethane sulfonyloxy group (trifluoromethane sulfonate). "Amphos" is an abbreviation of [4-(N,N-dimethylamino)phenyl]di-tert-butylphosphine.

[Formula 128]

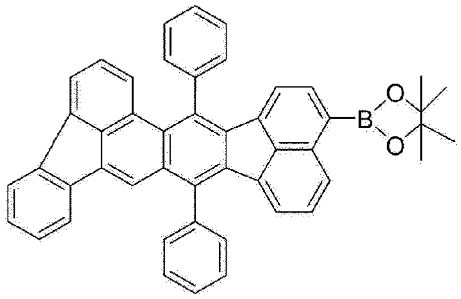

SM-72a

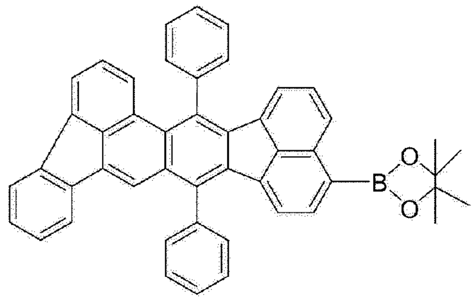

SM-72b

Synthesis Example 8: Synthesis of Compound BD-8

A compound BD-8 that was synthesized in the following manner in Synthesis Example 8 was a mixture containing a compound BD-8a and a compound BD-8b.

(1) Synthesis of Intermediate (Int-81)

[Formula 129]

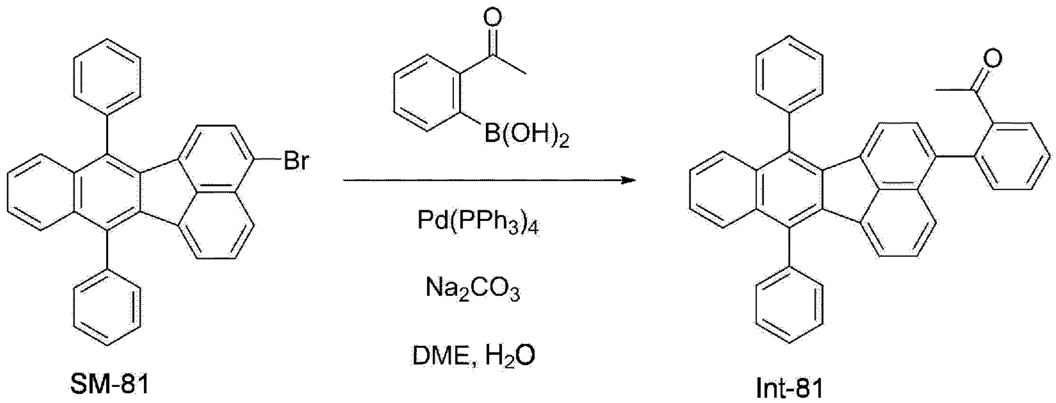


SM-81 Int-81

Under argon atmosphere, to a three-necked flask, 2.0 g (4.46 mmol) of a starting material (SM-81), 1.07 g (5.35 mmol) of 2-acetylphenylboronic acid, 1.18 g (11.2 mmol) of sodium carbonate, 10 mL of 1,2-dimethoxyethane (DME), and 10 mL of water were added, to which 0.10 g (0.089 mmol) of tetrakis(triphenylphosphine)palladium was further added. The obtained mixture was heated to reflux with stirring for 6.5 hours. After the mixture was heated to reflux with stirring, an organic layer was separated from the mixture. The separated organic layer was condensed under reduced pressure. The obtained residue was purified by silica-gel column chromatography. A mixture solvent of ethyl acetate and n-hexane was used as an eluent. The solid obtained after the residue was purified was suspended and washed in methanol to obtain an intermediate (Int-81).A yield was 2.1 g and a yield rate was 90%.

(2) Synthesis of Intermediate (Int-82)

[Formula 130]

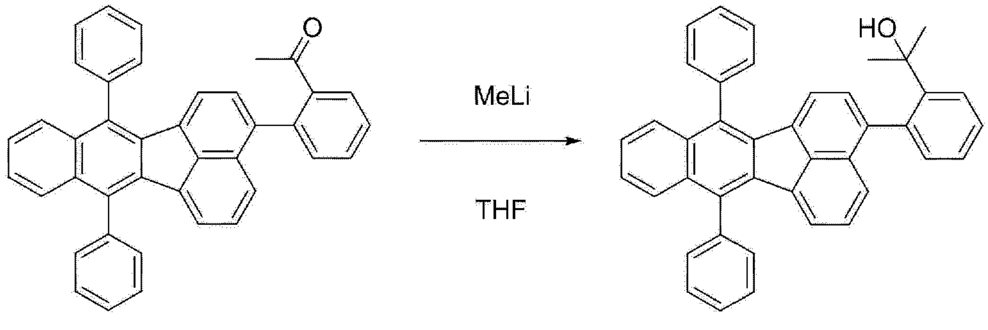


Int-81 Int-82

Under argon atmosphere, 2.1 g (4.02 mmol) of intermediate (Int-81) and 60 mL of tetrahydrofuran (THF) were added to a three-necked flask. Subsequently, the solution in the three-necked flask was cooled to zero degree C, to which a THF solution (4.82 ml) of 1M methyl lithium (MeLi) was added. The obtained solution was stirred for one hour. After the stirring, water was added to the reaction solution to stop the reaction, and subsequently, an organic layer was extracted with dichloromethane. The extracted organic layer was condensed under reduced pressure. The obtained residue was purified by silica-gel column chromatography. A mixture solvent of dichloromethane and n-hexane was used as an eluent. The solid obtained after the residue was purified was suspended and washed in methanol to obtain an intermediate (Int-82).A yield was 1.5 g and a yield rate was 69%.

(3) Synthesis of Compounds BD-8a and BD-8b

[Formula 131]

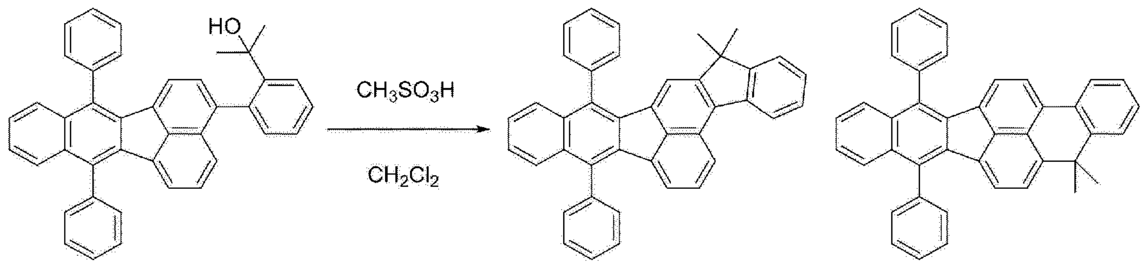


Int-82 BD-8a BD-8b

To a three-necked flask, 1.5 g (2.78 mmol) of intermediate (Int-82) and 300 mL of dichloromethane were added, to which 0.27 g (2.78 mmol) of methanesulfonic acid was dropped and stirred for one hour. After the stirring, a sodium hydrogen carbonate solution was added to the reaction solution to stop the reaction, and subsequently, an organic layer was extracted with dichloromethane. The extracted organic layer was condensed under reduced pressure. The obtained residue was purified by silica-gel column chromatography. A mixture solvent of dichloromethane and n-hexane was used as an eluent. The solid obtained after the residue was purified was suspended and washed in methanol to obtain a target (a mixture of compounds BD-8a and BD-8b) in a form of a yellow solid. A yield was 1.05 g and a yield rate was 72%. A result of FD-MS analysis shows m/e=520 relative to a molecular weight of 520. Since a small amount of the compound BD-8a was isolated from the mixture containing the compound BD-8a and the compound BD-8b, an emission spectrum of a toluene solution of the mixture was compared with an emission spectrum of a toluene solution of the isolated compound BD-8a. As a result, it was found that both the spectra were the same in shape.

Synthesis Example 9: Synthesis of Compound BD-10

Synthesis of Intermediate 8A

[Formula 132]

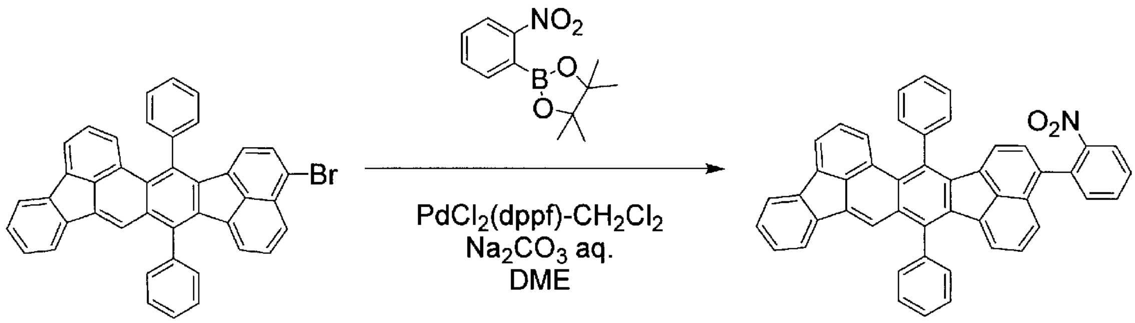


SM-1 Intermediate 8A

To a three-necked flask, under argon atmosphere, 3.0 g (4.94 mmol) of a starting material (SM-1: isomer mixture), 2.5 g (9.88 mmol) of 2-nitrophenylboronic acid pinacol ester, and 30 mL of 1,2-dimethoxyethane (DME) were added, to which 0.16 g (0.2 mmol) of $PdCl_2(dppf)/CH_2Cl_2$ was added and then 7.4 mL of 2M sodium carbonate aqueous solution was added. The obtained mixture solution was heated with stirring at 80 degrees C for six hours. After heated with stirring, the reaction solution was returned to the room temperature and filtrated to obtain a deposited solid. The obtained solid was dissolved in toluene, to which silica gel was further added and stirred. Subsequently, the reaction solution was filtrated and condensed to obtain an intermediate 8A. A yield was 2.74 g and a yield rate was 85%.

(1-2) Synthesis of Intermediate 8B

[Formula 133]

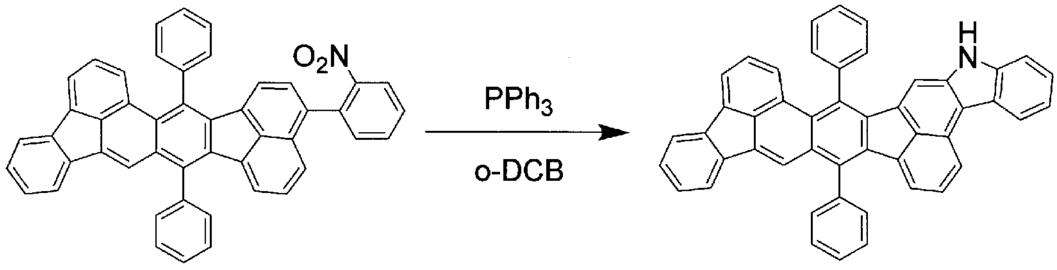


Intermediate 8A

Intermediate 8B

To a three-necked flask, 2.1 g (3.23 mmol) of the intermediate 8A, 2.12 g (8.08 mmol) of triphenylphosphine ($PPh_3$) and 20mL of orthodichlorobenzene (o-DCB) were added and heated with stirring at 140 degrees C under argon atmosphere for 24 hours. During the reaction, 2.11 g of triphenylphosphine was additionally put into the flask. After the solvent was distilled away from the reaction solution under reduced pressure, the obtained residue was purified by silica-gel column chromatography (a mobile phase that is hexane : toluene = 2 : 1 (a ratio by volume)), so that an intermediate 8B was obtained. A yield was 1.59 g and a yield rate was 75%.

(3) Synthesis of Compound BD-10

[Formula 134]

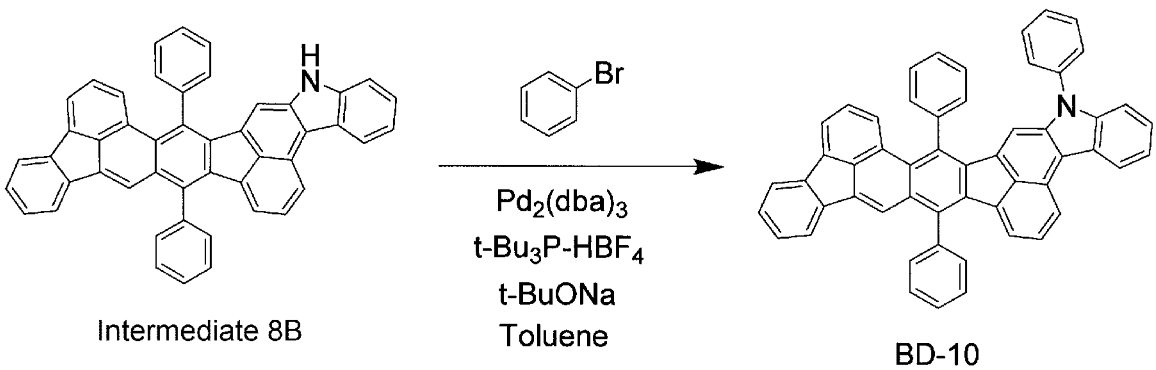


Intermediate 8B

BD-10

To a three-necked flask, under argon atmosphere, 1.57 g (2.54 mmol) of the intermediate 8B, 0.6 g (3.81 mmol) of bromobenzene, 47 mg (0.051 mmol) of $Pd_2(dba)_3$, 0.13 g (0.20 mmol) of t-$Bu_3P$-$HBF_4$, and 0.34 g (3.56 mmol) of t-BuONa were added, to which 20 mL of toluene was further added. The obtained mixture solution was heated with stirring at 105 degrees C for seven hours. After heated with stirring, the reaction solution was returned to the room temperature and filtrated to obtain a deposited solid. The obtained solid was dissolved in chlorobenzene, to which silica gel was further added and stirred. Subsequently, the reaction solution was filtrated and condensed to obtain a residue. The obtained residue was purified by silica-gel column chromatography (mobile phase that is hexane : toluene = 2 : 1 (ratio by volume). The purified residue was suspended and washed in ethyl acetate to obtain a compound BD-10. A yield was 0.78 g and a yield rate was 44%. A result of FD-MS analysis shows m/e=693 relative to a molecular weight of 693. Derived from the starting material (SM-1) that was an isomer mixture (a mixture containing SM-1a and SM-1b), the compound BD-10 was a mixture containing BD-10a and BD-10b. It should be noted that only a structure of SM-1a was exemplarily shown as "SM-1", only a structure of BD-10a was exemplarily shown as "BD-10", and only intermediates obtained from SM-1a were exemplarily shown as the intermediate 8A and the intermediate 8B in a reaction formula for Synthesis Example 9.

Synthesis Example 10: Synthesis of Compound BD-11

[Formula 135]

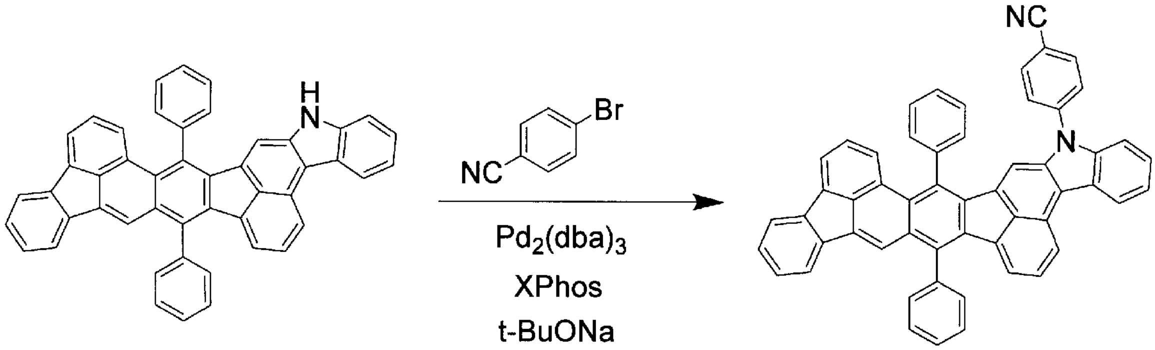


Intermediate 8B

BD-11

To a three-necked flask, under argon atmosphere, 2.33 g (3.77 mmol) of the intermediate 8B, 1.37 g (7.53 mmol) of 4-bromobenzonitrile, 0.14 g (0.15 mmol) of $Pd_2(dba)_3$, 0.29 g (0.61 mmol) of XPhos, and 0.51 g (5.27 mmol) of t-BuONa were added, to which 20 mL of toluene was further added. The obtained mixture solution was heated with stirring at 100 degrees C for 15 hours. After heated with stirring, the reaction solution was returned to the room temperature and filtrated to obtain a deposited solid. The obtained solid was dissolved in chlorobenzene, to which silica gel was further added and stirred. Subsequently, the reaction solution was filtrated and condensed to obtain a solid. The obtained solid was suspended and washed in methanol to obtain a compound BD-11. A yield was 2.19 g and a yield rate was 81%. A result of FD-MS analysis shows m/e=718 relative to a molecular weight of 718. Derived from the starting material (SM-1) that was an isomer mixture (a mixture containing SM-1a and SM-1b), the compound BD-11 was a mixture containing BD-11 a and BD-11 b. It should be noted that only a structure of BD-11 a was exemplarily shown as "BD-11" and only a structure of an intermediate obtained from SM-1a was exemplarily shown as the "intermediate 8B" in a reaction formula for Synthesis Example 10.

Synthesis Example 11: Synthesis of Compound BD-12

[Formula 136]

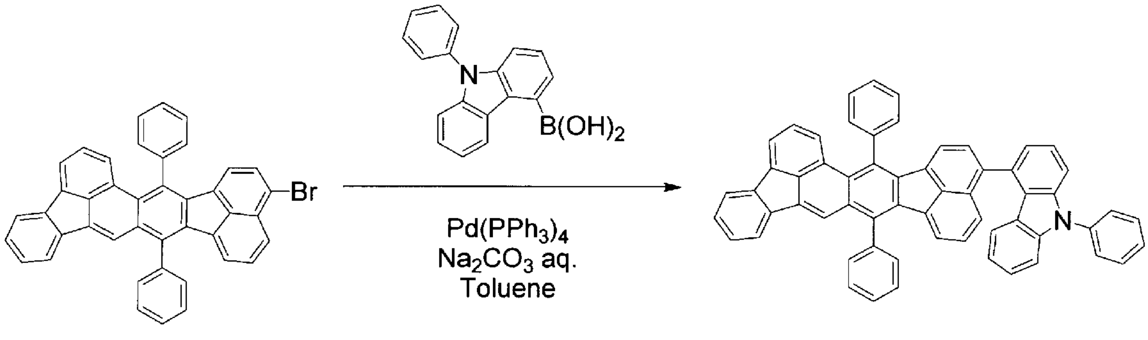


SM-1

BD-12

To a three-necked flask, 1.0 g (1.65 mmol) of a starting material (SM-1: isomer mixture), 0.57 g (1.98 mmol) of 9-phenylcarbazol-4-boronic acid, 2.5 mL of 2M sodium carbonate aqueous solution, 10 mL of toluene were added, to which 76 mg (0.066 mmol) of tetrakis(triphenylphosphine)palladium was further added, and heated with stirring at 90

degrees C under argon atmosphere for seven hours. After heated with stirring, the reaction solution was returned to the room temperature and filtrated to obtain a deposited solid. The obtained solid was dissolved in toluene, to which silica gel was further added and stirred. Subsequently, the reaction solution was filtrated and condensed to obtain a compound BD-12. A yield was 0.65 g and a yield rate was 51%. A result of FD-MS analysis shows m/e=769 relative to a molecular weight of 769. Derived from the starting material (SM-1) that was an isomer mixture (a mixture containing SM-1a and SM-1b, the compound BD-12 was a mixture containing BD-12a and BD-12b. It should be noted that only a structure of SM-1a was exemplarily shown as "SM-1" and only a structure of BD-12a was exemplarily shown as "BD-12" in a reaction formula for Synthesis Example 11.

Synthesis Example 12: Synthesis of Compound BD-13

[Formula 137]

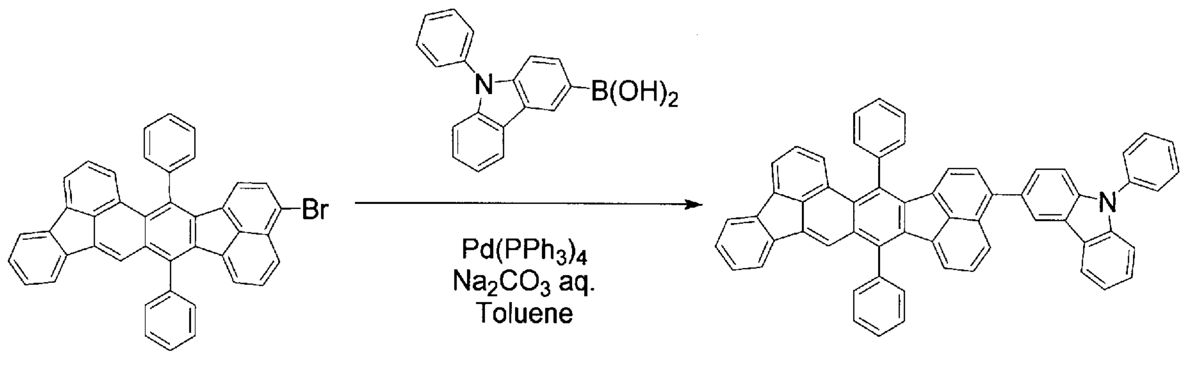


SM-1 BD-13

To a three-necked flask, 2.0 g (3.29 mmol) of a starting material (SM-1: isomer mixture), 1.42 g (4.94 mmol) of 9-phenylcarbazol-3-boronic acid, 5 mL of 2M sodium carbonate aqueous solution, 20 mL of toluene were added, to which 0.15 g (0.13 mmol) of tetrakis(triphenylphosphine)palladium was further added, and heated with stirring at 90 degrees C under argon atmosphere for four hours. After heated with stirring, the reaction solution was returned to the room temperature and filtrated to obtain a deposited solid. The obtained solid was dissolved in toluene, to which silica gel was further added and stirred. Subsequently, the reaction solution was filtrated and condensed. The obtained solid was washed in ethanol to obtain a compound BD-13. A yield was 0.85 g and a yield rate was 33%. A result of FD-MS analysis shows m/e=769 relative to a molecular weight of 769. Derived from the starting material (SM-1) that was an isomer mixture (a mixture containing SM-1a and SM-1b), the compound BD-13 was a mixture containing BD-13a and BD-13b. It should be noted that only a structure of SM-1a was exemplarily shown as "SM-1" and only a structure of BD-13a was exemplarily shown as "BD-13" in a reaction formula for Synthesis Example 12.

Evaluation of Compounds

A method of measuring properties of the compounds is shown below.

Delayed Fluorescence

Occurrence of delayed fluorescence was determined by measuring transient photoluminescence (PL) using a device shown in Fig. 2. A sample was prepared by co-depositing the compound TADF-1 and the compound TH-2 on a quartz substrate at a ratio of the compound TADF-1 of 12 mass% to form a 100-nm-thick thin film. Emission from the compound TADF-1 include: Prompt emission observed immediately when the excited state is achieved by exciting the compound TADF-1 with a pulse beam (i.e., a beam emitted from a pulse laser) having an absorbable wavelength; and Delayed emission observed not immediately when but after the excited state is achieved. Delayed fluorescence emission in the exemplary embodiment means that the amount of Delay emission is 5% or more relative to the amount of Prompt emission. Specifically, when the amount of Prompt emission is denoted by $X_P$ and the amount of Delayed emission is denoted by $X_D$, the delayed fluorescence means that a value of $X_D/X_P$ is 0.05 or more.

It was found that the amount of Delayed emission of the compound TADF-1 was 5% or more relative to the amount of Prompt emission. Specifically, it was found that the value of $X_D/X_P$ in the compound TADF-1 was 0.05 or more.

The amount of Prompt emission and the amount of Delayed emission can be obtained in the same method as a method described in "Nature 492, 234-238, 2012." A device used for calculating the amounts of Prompt Emission and Delayed Emission is not limited to the device of Fig. 2 and a device described in the above document.

Singlet Energy $S_1$

The compound TADF-1, compound BD-1, compound BD-2, compound BD-3, compound BD-4, compound BD-5, compound BD-6, compound BD-7, compound BD-8, compound BD-9, compound BD-10, compound BD-11, compound BD-12, and compound BD-13 were measured in terms of a singlet energy $S_1$ according to the above-described solution method.

The measurement results are shown in Table 2.

Table 2

| Measurement Target | Singlet Energy [eV] |
|---|---|
| TADF-1 | 2.90 |
| BD-1 | 2.77 |
| BD-2 | 2.71 |
| BD-3 | 2.75 |
| BD-4 | 2.73 |
| BD-5 | 2.73 |
| BD-6 | 2.68 |
| BD-7 | 2.68 |
| BD-8 | 2.75 |
| BD-9 | 2.81 |
| BD-10 | 2.64 |
| BD-11 | 2.64 |
| BD-12 | 2.73 |
| BD-13 | 2.68 |

A singlet energy of a compound DPEPO is 4.0 eV as described in a document (APPLIED PHYSICS LETTERS 101, 093306 (2012)).

Emission Peak Wavelength of Compounds

Emission Spectrum

An emission spectrum of the second compound was measured by the following method.

5 μmol/L of a toluene solution of a target compound for the measurement was prepared and put in a quartz cell to prepare a sample. An emission spectrum (ordinate axis: luminous intensity, abscissa axis: wavelength) of the sample was measured at a normal temperature (300K).In Examples, the emission spectrum was measured using a spectrophotometer manufactured by Hitachi, Ltd. (device name: F-7000). It should be noted that the emission spectrum measuring device is not limited to the above device.

In the emission spectrum, a peak wavelength of the emission spectrum at the maximum luminous intensity is defined as an emission peak wavelength.

An emission peak wavelength of the compound BD-1 was 445 nm.

An emission peak wavelength of the compound BD-2 was 455 nm.

An emission peak wavelength of the compound BD-3 was 448 nm.

An emission peak wavelength of the compound BD-4 was 452 nm.

An emission peak wavelength of the compound BD-5 was 452 nm.
An emission peak wavelength of the compound BD-6 was 462 nm.
An emission peak wavelength of the compound BD-7 was 459 nm.
An emission peak wavelength of the compound BD-8 was 449 nm.
An emission peak wavelength of the compound BD-9 was 438 nm.
An emission peak wavelength of the compound BD-10 was 462 nm.
An emission peak wavelength of the compound BD-11 was 460 nm.
An emission peak wavelength of the compound BD-12 was 454 nm.
An emission peak wavelength of the compound BD-13 was 463 nm.

Molar Absorbance Coefficient ε

A molar absorbance coefficient ε was calculated by dividing an absorption intensity of the closest absorption peak to the long-wavelength side of the absorption spectrum by a solution concentration. A unit of the molar absorbance coefficient ε was defined as L/(mol·cm). The closest absorption peak to the long-wavelength side means an absorption peak closest to the long-wavelength side among absorption peaks appearing in a range from 350 nm 500 nm and each having an absorption intensity of at least one-tenth of the maximum absorption peak.

Absorption Spectrum

An absorption spectrum of the second compound was measured by the following method.
20 μmol/L of a toluene solution of the target compound for the measurement was prepared and put in a quartz cell to prepare a sample. An absorption spectrum (ordinate axis: absorption intensity, abscissa axis: wavelength) of the sample was measured at a normal temperature (300K). In Examples, the absorption spectrum was measured using a spectrophotometer manufactured by Hitachi, Ltd. (device name: U3310). It should be noted that the absorption spectrum measuring device is not limited to the above device.

Stokes Shift ss

Stokes shift (ss) was calculated by subtracting an absorption peak wavelength, where a molar absorbance coefficient of an absorption spectrum was obtained, from an emission peak wavelength of an emission spectrum. A unit of the Stokes shift (ss) was defined as nm.
Table 3 shows the molar absorbance coefficient, emission peak wavelength, absorption peak wavelength, and Stokes shift of each of the compounds.

Table 3

| Compound | Molar absorbance coefficient [ L/(mol ▪ cm) ] | Emission peak wavelength [nm] | Absorption peak wavelength [nm] | Stokes shift [nm] |
|---|---|---|---|---|
| BD-1 | 48100 | 445 | 438 | 7 |
| BD-2 | 47000 | 455 | 443 | 12 |
| BD-3 | 69400 | 448 | 442 | 6 |
| BD-4 | 43200 | 452 | 441 | 11 |
| BD-5 | 45100 | 452 | 441 | 11 |
| BD-6 | 47600 | 462 | 446 | 16 |
| BD-7 | 51300 | 459 | 445 | 14 |
| BD-8 | 30400 | 449 | 438 | 11 |
| BD-9 | 118000 | 438 | 434 | 4 |
| BD-10 | 53500 | 462 | 454 | 8 |
| BD-11 | 59600 | 460 | 454 | 6 |
| BD-12 | 41800 | 454 | 440 | 14 |

(continued)

| Compound | Molar absorbance coefficient [ L/(mol ▪ cm) ] | Emission peak wavelength [nm] | Absorption peak wavelength [nm] | Stokes shift [nm] |
|---|---|---|---|---|
| BD-13 | 43800 | 463 | 446 | 17 |
| TBPe | 28100 | 458 | 439 | 19 |
| Ref-1 | 26300 | 459 | 442 | 17 |
| Ref-2 | 28600 | 440 | 428 | 12 |
| Ref-3 | 40500 | 452 | 429 | 23 |
| Ref-4 | 41100 | 449 | 424 | 25 |

Preparation of Organic EL device

Organic EL devices were prepared in the following manner and evaluated.

Example 1

A glass substrate (size: 25 mm × 75 mm × 1.1 mm thick, manufactured by Geomatec Co., Ltd.) having an ITO transparent electrode (anode) was ultrasonic-cleaned in isopropyl alcohol for five minutes. After the ultrasonic-cleaning, the substrate was UV/ozone-cleaned for 30 minutes. A film of ITO was 130 nm thick.

The cleaned glass substrate was mounted on a substrate holder of a vacuum evaporation apparatus. Initially, a compound HI was deposited on a surface of the glass substrate where the transparent electrode line was provided in a manner to cover the transparent electrode, thereby forming a 5-nm-thick hole injecting layer.

Next, a compound HT1 was deposited on the hole injecting layer to form a 80-nm-thick first hole transporting layer.

Next, a compound HT-2 was deposited on the first hole transporting layer to form a 10-nm-thick second hole transporting layer.

Next, a compound mCP was deposited on the second hole transporting layer to form a 5-nm-thick electron blocking layer.

Next, the compound TADF-1 (the first compound), the compound BD-1 (the second compound) and the compound DPEPO (the third compound) were codeposited on the electron blocking layer to form a 25-nm-thick emitting layer. A concentration of the compound BD-1 was defined as 1 mass%, a concentration of the compound TADF-1 was defined as 24 mass%, and a concentration of the compound DPEPO was defined as 75 mass% in the emitting layer.

Next, a compound ET-1 was deposited on the emitting layer to form a 5-nm-thick hole blocking layer.

Next, a compound ET-2 was deposited on the hole blocking layer to form a 20-nm-thick electron transporting layer.

Lithium fluoride (LiF) was then deposited on the electron transporting layer to form a 1-nm-thick electron injecting electrode (cathode).

A metal aluminum (Al) was then deposited on the electron injecting electrode to form an 80-nm-thick metal Al cathode.

A device arrangement of the organic EL device in Example 1 is schematically shown as follows.

ITO(130)/HI(5)/HT1(80)/HT2(10)/mCP(5)/DPEPO:TADF-1:BD-1(25, 24%, 1%)/ET-1(5)/ET-2(20)/LiF(1)/Al(80)

Numerals in parentheses represent a film thickness (unit: nm).The numerals in the form of percentage in parentheses, sequentially from the left, indicate the respective ratios of the first and second compounds in the emitting layer. A unit of the ratios is mass%,

Example 2

An organic EL device of Example 2 was prepared in the same manner as the organic EL device of Example 1 except that a compound BD-2 was used in place of the compound BD-1 in the emitting layer of Example 1.

A device arrangement of the organic EL device in Example 2 is schematically shown as follows.

ITO(130)/HI(5)/HT1(80)/HT2(10)/mCP(5)/DPEPO:TADF-1:BD-2(25, 24%, 1%)/ET-1(5)/ET-2(20)/LiF(1)/Al(80)

Example 3

An organic EL device of Example 3 was prepared in the same manner as the organic EL device of Example 1 except that a compound BD-3 was used in place of the compound BD-1 in the emitting layer of Example 1.
A device arrangement of the organic EL device in Example 3 is schematically shown as follows.
ITO(13O)/HI(5)/HT1(80)/HT2(10)/mCP(5)/DPEPO:TADF-1:BD-3(25, 24%, 1%)/ET-1(5)/ET-2(20)/LiF(1)/Al(80)

Example 4 (Reference Example not covered by the claims)

An organic EL device of Example 4 was prepared in the same manner as the organic EL device of Example 1 except that a compound BD-4 was used in place of the compound BD-1 in the emitting layer of Example 1.
A device arrangement of the organic EL device in Example 4 is schematically shown as follows.
ITO(130)/HI(5)/HT1(80)/HT2(10)/mCP(5)/DPEPO:TADF-1:BD-4(25, 24%, 1%)/ET-1(5)/ET-2(20)/LiF(1)/Al(80)

Example 5 (Reference Example not covered by the claims)

An organic EL device of Example 5 was prepared in the same manner as the organic EL device of Example 1 except that a compound BD-5 was used in place of the compound BD-1 in the emitting layer of Example 1.
A device arrangement of the organic EL device in Example 5 is schematically shown as follows.
ITO(130)/HI(5)/HT1(80)/HT2(10)/mCP(5)/DPEPO:TADF-1:BD-5(25, 24%, 1%)/ET-1(5)/ET-2(20)/LiF(1)/Al(80)

Example 6

An organic EL device of Example 6 was prepared in the same manner as the organic EL device of Example 1 except that a compound BD-6 was used in place of the compound BD-1 in the emitting layer of Example 1.
A device arrangement of the organic EL device in Example 6 is schematically shown as follows.
ITO(130)/HI(5)/HT1(80)/HT2(10)/mCP(5)/DPEPO:TADF-1:BD-6(25, 24%, 1%)/ET-1(5)/ET-2(20)/LiF(1)/Al(80)

Example 7 (Reference Example not covered by the claims)

An organic EL device of Example 7 was prepared in the same manner as the organic EL device of Example 1 except that a compound BD-7 was used in place of the compound BD-1 in the emitting layer of Example 1.
A device arrangement of the organic EL device in Example 7 is schematically shown as follows.
ITO(130)/HI(5)/HT1(80)/HT2(10)/mCP(5)/DPEPO-TADF-1-BD-7(25, 24%, 1%)/ET-1(5)/ET-2(20)/LiF(1)/Al(80)

Example 8

An organic EL device of Example 8 was prepared in the same manner as the organic EL device of Example 1 except that a compound BD-8 was used in place of the compound BD-1 in the emitting layer of Example 1.
A device arrangement of the organic EL device in Example 8 is schematically shown as follows.
ITO(130)/HI(5)/HT1(80)/HT2(10)/mCP(5)/DPEPO:TADF-1:BD-8(25, 24%, 1%)/ET-1(5)/ET-2(20)/LiF(1)/Al(80)

Example 9 (Reference Example not covered by the claims)

An organic EL device of Example 9 was prepared in the same manner as the organic EL device of Example 1 except that a compound BD-9 was used in place of the compound BD-1 in the emitting layer of Example 1.
A device arrangement of the organic EL device in Example 9 is schematically shown as follows.
ITO(130)/HI(5)/HT1(80)/HT2(10)/mCP(5)/DPEPO:TADF-1:BD-9(25, 24%, 1%)/ET-1(5)/ET-2(20)/LiF(1)/Al(80)

Example 10 (Reference Example not covered by the claims)

An organic EL device of Example 10 was prepared in the same manner as the organic EL device of Example 1 except that a compound BD-10 was used in place of the compound BD-1 in the emitting layer of Example 1.
A device arrangement of the organic EL device in Example 10 is schematically shown as follows.
ITO(130)/HI(5)/HT1(80)/HT2(10)/mCP(5)/DPEPO:TADF-1:BD-10(25, 24%, 1%)/ET-1(5)/ET-2(20)/LiF(1)/Al(80)

Example 11 (Reference Example not covered by the claims)

An organic EL device of Example 11 was prepared in the same manner as the organic EL device of Example 1 except that a compound BD-11 was used in place of the compound BD-1 in the emitting layer of Example 1.
A device arrangement of the organic EL device in Example 11 is schematically shown as follows.
ITO(130)/HI(5)/HT1(80)/HT2(10)/mCP(5)/DPEPO:TADF-1:BD-11(25, 24%, 1%)/ET-1(5)/ET-2(20)/LiF(1)/Al(80)

Example 12 (Reference Example not covered by the claims)

An organic EL device of Example 12 was prepared in the same manner as the organic EL device of Example 1 except that a compound BD-12 was used in place of the compound BD-1 in the emitting layer of Example 1.
A device arrangement of the organic EL device in Example 12 is schematically shown as follows.
ITO(130)/HI(5)/HT1(80)/HT2(10)/mCP(5)/DPEPO:TADF-1:BD-12(25, 24%, 1%)/ET-1(5)/ET-2(20)/LiF(1)/Al(80)

Example 13 (Reference Example not covered by the claims)

An organic EL device of Example 13 was prepared in the same manner as the organic EL device of Example 1 except that a compound BD-13 was used in place of the compound BD-1 in the emitting layer of Example 1.
A device arrangement of the organic EL device in Example 13 is schematically shown as follows.
ITO(130)/HI(5)/HT1(80)/HT2(10)/mCP(5)/DPEPO:TADF-1:BD-13(25, 24%, 1%)/ET-1(5)/ET-2(20)/LiF(1)/Al(80)

Comparative 1

An organic EL device of Comparative 1 was prepared in the same manner as the organic EL device of Example 1 except that a compound TBPe was used in place of the compound BD-1 in the emitting layer of Example 1.
A device arrangement of the organic EL device of Comparative 1 is schematically shown as follows.
ITO(130)/HI(5)/HT1(80)/HT2(10)/mCP(5)/DPEPO:TADF-1:TBPe(25, 24%, 1%)/ET-1(5)/ET-2(20)/LiF(1)/Al(80)

Comparative 2

An organic EL device of Comparative 2 was prepared in the same manner as the organic EL device of Example 1 except that a compound Ref-1 was used in place of the compound BD-1 in the emitting layer of Example 1.
A device arrangement of the organic EL device of Comparative 2 is schematically shown as follows.
ITO(130)/HI(5)/HT1(80)/HT2(10)/mCP(5)/DPEPO:TADF-1:Ref-1(25, 24%, 1%)/ET-1(5)/ET-2(20)/LiF(1)/Al(80)

Comparative 3

An organic EL device of Comparative 3 was prepared in the same manner as the organic EL device of Example 1 except that a compound Ref-2 was used in place of the compound BD-1 in the emitting layer of Example 1.
A device arrangement of the organic EL device of Comparative 3 is schematically shown as follows.
ITO(130)/HI(5)/HT1(80)/HT2(10)/mCP(5)/DPEPO:TADF-1:Ref-2(25, 24%, 1%)/ET-1(5)/ET-2(20)/LiF(1)/Al(80)

Comparative 4

An organic EL device of Comparative 4 was prepared in the same manner as the organic EL device of Example 1 except that a compound Ref-3 was used in place of the compound BD-1 in the emitting layer of Example 1.
A device arrangement of the organic EL device of Comparative 4 is schematically shown as follows.
ITO(130)/HI(5)/HT1(80)/HT2(10)/mCP(5)/DPEPO:TADF-1:Ref-3(25, 24%, 1%)/ET-1(5)/ET-2(20)/LiF(1)/Al(80)

Comparative 5

An organic EL device of Comparative 5 was prepared in the same manner as the organic EL device of Example 1 except that a compound Ref-4 was used in place of the compound BD-1 in the emitting layer of Example 1.
A device arrangement of the organic EL device of Comparative 5 is schematically shown as follows.
ITO(130)/HI(5)/HT1(80)/HT2(10)/mCP(5)/DPEPO:TADF-1:Ref-4(25, 24%, 1%)/ET-1(5)/ET-2(20)/LiF(1)/Al(80)

Evaluation of Organic EL Devices

The manufactured organic EL devices were evaluated as follows. The evaluation results are shown in Table 4.

External Quantum Efficiency EQE

Voltage was applied on each of the organic EL devices such that the current density was 0.1 mA/cm$^2$, where spectral-radiance spectra were measured using a spectroradiometer CS-1000 (manufactured by Konica Minolta, Inc.). The external quantum efficiency EQE (unit: %) was calculated based on the obtained spectral-radiance spectra, assuming that the spectra was provided under a Lambertian radiation.

Table 4

| | Second material | | | EQE [% ] |
|---|---|---|---|---|
| | Compound | Molar absorbance coefficient [L/(mol·cm)] | Stokes shift [nm] | |
| Example 1 | BD-1 | 48100 | 7 | 12.3 |
| Example 2 | BD-2 | 47000 | 12 | 13.8 |
| Example 3 | BD-3 | 69400 | 6 | 12.6 |
| Example 4 | BD-4 | 43200 | 11 | 12.5 |
| Example 5 | BD-5 | 45100 | 11 | 13.0 |
| Example 6 | BD-6 | 47600 | 16 | 14.6 |
| Example 7 | BD-7 | 51300 | 14 | 15.4 |
| Example 8 | BD-8 | 30400 | 11 | 10.9 |
| Example 9 | BD-9 | 118000 | 4 | 11.4 |
| Example 10 | BD-10 | 53500 | 8 | 11.6 |
| Example 11 | BD-11 | 59600 | 6 | 11.1 |
| Example 12 | BD-12 | 41800 | 14 | 14.4 |
| Example 13 | BD-13 | 43800 | 17 | 11.0 |
| Com parative 1 | TBPe | 28100 | 19 | 8.7 |
| Comparative 2 | Ref-1 | 26300 | 17 | 8.7 |
| Comparative 3 | Ref-2 | 28600 | 12 | 9.1 |
| Comparative 4 | Ref-3 | 40500 | 23 | 5.6 |
| Comparative 5 | Ref-4 | 41100 | 25 | 5.6 |

(Examples 4, 5, 7, 9, 10, 11, 12, 13 are Reference Examples not covered by the claims).

According to the organic EL devices in Examples 1 to 13 in which the emitting layer contains the delayed fluorescent first compound and the fluorescent second compound having characteristics of a predetermined emission peak wavelength, a predetermined molar absorbance coefficient, and a predetermined Stokes shift in combination, light in a blue wavelength region is emittable at a high efficiency as compared with the organic EL devices in Comparatives 1 to 5.

EXPLANATION OF CODE(S)

1... organic EL device, 3... anode, 4... cathode, 5... emitting layer, 7... hole transporting layer, 8... electron transporting layer.

## Claims

**1.** An organic electroluminescence device comprising:

an anode;
an emitting layer; and
a cathode, wherein
the emitting layer comprises a first compound and a second compound,
the first compound is a delayed fluorescent compound, wherein
the first compound is a compound represented by a formula,

[Formula 26]

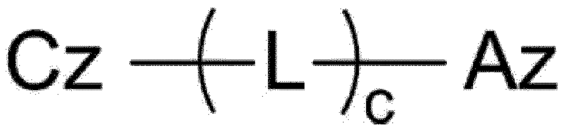

where: Az is a cyclic structure selected from the group consisting of a substituted or unsubstituted pyridine ring, a substituted or unsubstituted pyrimidine ring, a substituted or unsubstituted triazine ring, and a substituted or unsubstituted pyrazine ring;

c is an integer of 0 to 5;
L is a linking group selected from the group consisting of a substituted or unsubstituted aromatic hydrocarbon group having 6 to 30 ring carbon atoms, and a substituted or unsubstituted heterocyclic group having 5 to 30 ring atoms;
when c is 0, Cz is bonded to Az by a single bond;
when c is an integer of 2 to 5, a plurality of L are bonded to each other to form a ring, or are not bonded;
a plurality of L are mutually the same or different; and
Cz is represented by a formula (27),

[Formula 27]

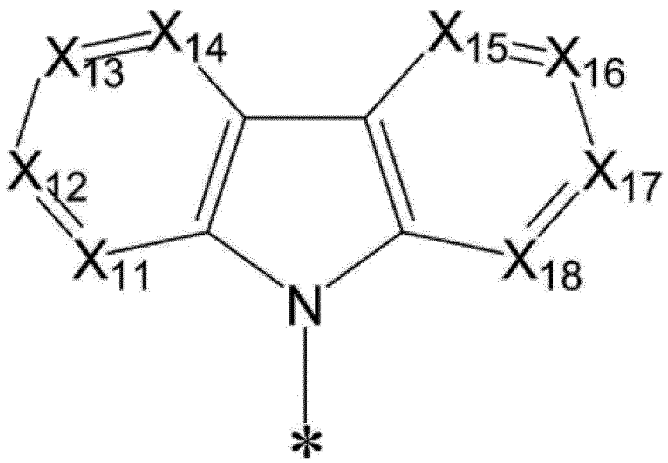

where: $X_{11}$ to $X_{18}$ are each independently a nitrogen atom or C-Rx;

Rx is each independently a hydrogen atom or a substituent;
Rx serving as the substituent is a group selected from the group consisting of a substituted or unsubstituted aryl group having 6 to 30 ring carbon atoms, a substituted or unsubstituted heteroaryl group having 5 to 30 ring atoms, a substituted or unsubstituted alkyl group having 1 to 30 carbon atoms, a substituted or unsubstituted fluoroalkyl group having 1 to 30 carbon atoms, a substituted or unsubstituted cycloalkyl group having 3 to 30 ring carbon atoms, a substituted or unsubstituted aralkyl group having 7 to 30 carbon atoms, a substituted phosphoryl group, a substituted silyl group, a cyano group, a nitro group, and a carboxy group;
a plurality of Rx are mutually the same or different;
when a plurality of ones of $X_{11}$ to $X_{18}$ are C-Rx and Rx are substituents, Rx are not bonded to each other to form a ring; and
* represents a bonding position to a carbon atom in a structure of the linking group represented by L, or a bonding position to a carbon atom in the cyclic structure represented by Az,

the second compound is a fluorescent compound,
the second compound is a hydrocarbon compound consisting of a carbon atom(s) and a hydrogen atom(s) having a mother skeleton including fused rings, the number of the fused rings ranging from 5 to 15, and extending while being bent in one direction, said fused rings comprise a five-membered ring,
an emission peak wavelength of a solution of the second compound in toluene is in a range from 430 nm to 480 nm,
a molar absorbance coefficient of the second compound at an absorption peak closest to a long-wavelength side is in a range from 40000 L/(mol·cm) to 1000000 L/(mol·cm), and
a Stokes shift of the second compound is in a range from 1 nm to 20 nm.

2. The organic electroluminescence device according to claim 1, wherein
the emission peak wavelength of the solution of the second compound in toluene is in a range from 435 nm to 465 nm.

3. The organic electroluminescence device according to claim 1 or 2, wherein
the Stokes shift of the second compound is in a range from 4 nm to 18 nm.

4. The organic electroluminescence device according to any one of claims 1 to 3, wherein
the Stokes shift of the second compound is in a range from 5 nm to 17 nm.

5. The organic electroluminescence device according to any one of claims 1 to 4, wherein
the molar absorbance coefficient of the second compound is in a range from 40000 L/(mol · cm) to 250000 L/(mol · cm).

6. The organic electroluminescence device according to any one of claims 1 to 5, wherein
the second compound is a substituted or unsubstituted aromatic compound.

7. The organic electroluminescence device according to any one of claims 1 to 6, wherein
a singlet energy $S_1(M1)$ of the first compound and a singlet energy $S_1(M2)$ of the second compound satisfy a relationship of Numerical Formula 1,

$$S_1(M1) > S_1(M2) \quad \text{(Numerical Formula 1).}$$

**8.** The organic electroluminescence device according to any one of claims 1 to 7, wherein

the emitting layer further comprises a third compound, and
the singlet energy $S_1(M1)$ of the first compound and a singlet energy $S_1(M3)$ of the third compound satisfy a relationship of Numerical Formula 2,

$$S_1(M3) > S_1(M1) \quad \text{(Numerical Formula 2).}$$

**9.** The organic electroluminescence device according to claim 8, wherein an energy gap $T_{77K}$ at 77 [K] of the third compound is larger than an energy gap $T_{77K}$ at 77 [K] of the second compound.

**10.** The organic electroluminescence device according to claim 8 or 9, wherein an energy gap $T_{77K}$ at 77 [K] of the third compound is larger than an energy gap $T_{77K}$ at 77 [K] of the first compound.

**11.** The organic electroluminescence device according to any one of claims 8 to 10, wherein a content ratio of the first compound in the emitting layer is in a range from 10 mass% to 80 mass%.

**12.** The organic electroluminescence device according to any one of claims 1 to 11, wherein an energy gap $T_{77K}$ at 77 [K] of the first compound is larger than an energy gap $T_{77K}$ at 77 [K] of the second compound.

**13.** The organic electroluminescence device according to any one of claims 1 to 12, further comprising: a hole transporting layer provided between the anode and the emitting layer.

**14.** The organic electroluminescence device according to any one of claims 1 to 13, further comprising: an electron transporting layer provided between the cathode and the emitting layer.

**15.** An electronic device comprising the organic electroluminescence device according to any one of claims 1 to 14.

**Patentansprüche**

**1.** Eine organische Elektrolumineszenzvorrichtung, umfassend:

eine Anode;
eine emittierende Schicht; und
eine Kathode, wobei
die emittierende Schicht eine erste Verbindung und eine zweite Verbindung umfasst,
die erste Verbindung eine verzögert fluoreszierende Verbindung ist, wobei
die erste Verbindung eine Verbindung ist, die durch eine Formel dargestellt wird,

[Formel 26]

$$Cz-(L)_c-Az$$

wobei:

Az eine zyklische Struktur ist, ausgewählt aus der Gruppe bestehend aus einem substituierten oder unsubstituierten Pyridinring, einem substituierten oder unsubstituierten Pyrimidinring, einem substituierten

oder unsubstituierten Triazinring und einem substituierten oder unsubstituierten Pyrazinring;
c eine ganze Zahl von 0 bis 5 ist;
L eine verbindende Gruppe ist, die aus der Gruppe ausgewählt ist, die aus einer substituierten oder unsubstituierten aromatischen Kohlenwasserstoffgruppe mit 6 bis 30 Ringkohlenstoffatomen und einer substituierten oder unsubstituierten heterocyclischen Gruppe mit 5 bis 30 Ringatomen besteht;
wenn c 0 ist, ist Cz an Az durch eine Einfachbindung gebunden;
wenn c eine ganze Zahl von 2 bis 5 ist, sind eine Vielzahl von L aneinandergebunden, um einen Ring zu bilden, oder sind nicht gebunden;
eine Vielzahl von L gleich oder verschieden sind; und
Cz durch eine Formel (27) dargestellt wird,

[Formel 27]

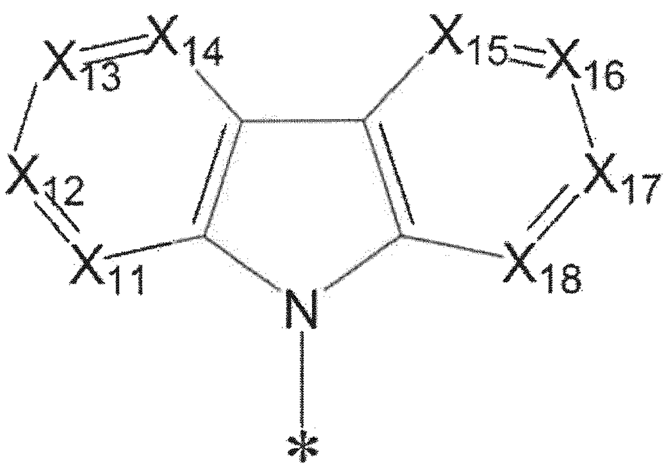

wobei: $X_{11}$ bis $X_{18}$ jeweils unabhängig voneinander ein Stickstoffatom oder C-Rx sind;

Rx ist jeweils unabhängig ein Wasserstoffatom oder ein Substituent;
Rx, das als Substituent dient, eine Gruppe ist, ausgewählt aus der Gruppe, bestehend aus einer substituierten oder unsubstituierten Arylgruppe mit 6 bis 30 Ringkohlenstoffatomen, einer substituierten oder unsubstituierten Heteroarylgruppe mit 5 bis 30 Ringatomen, einer substituierten oder unsubstituierten Alkylgruppe mit 1 bis 30 Kohlenstoffatomen eine substituierte oder unsubstituierte Fluoralkylgruppe mit 1 bis 30 Kohlenstoffatomen, eine substituierte oder unsubstituierte Cycloalkylgruppe mit 3 bis 30 Ringkohlenstoffatomen, eine substituierte oder unsubstituierte Aralkylgruppe mit 7 bis 30 Kohlenstoffatomen, eine substituierte Phosphorylgruppe, eine substituierte Silylgruppe, eine Cyanogruppe, eine Nitrogruppe und eine Carboxygruppe;
eine Vielzahl von Rx gleich oder verschieden sind;
wenn mehrere von $X_{11}$ bis $X_{18}$ C-Rx sind und Rx Substituenten sind, Rx nicht aneinandergebunden sind, um einen Ring zu bilden; und
* eine Bindungsposition zu einem Kohlenstoffatom in einer Struktur der durch L dargestellten verbindenden Gruppe oder eine Bindungsposition zu einem Kohlenstoffatom in der durch Az dargestellten cyclischen Struktur darstellt,
die zweite Verbindung eine fluoreszierende Verbindung ist, die zweite Verbindung eine Kohlenwasserstoffverbindung ist, die aus einem oder mehreren Kohlenstoffatom(en) und einem oder mehreren Wasserstoffatom(en) mit einem Grundgerüst besteht, das kondensierte Ringe enthält, wobei die Anzahl der kondensierten Ringe im Bereich von 5 bis 15 liegt und sich unter Biegung in eine Richtung erstrecken, wobei die genannten kondensierten Ringe einen fünfgliedrigen Ring bilden,
eine Wellenlänge des Emissionspeaks einer Lösung der zweiten Verbindung in Toluol in einem Bereich von 430 nm bis 480 nm liegt,
ein molarer Absorptionskoeffizient der zweiten Verbindung an einem Absorptionspeak, der einer langwelligen Seite am nächsten liegt, im Bereich von 40000 L/(mol·cm) bis 1000000 L/(mol·cm) liegt, und

eine Stokes-Verschiebung der zweiten Verbindung in einem Bereich von 1 nm bis 20 nm liegt.

**2.** Die organische Elektrolumineszenzvorrichtung nach Anspruch 1, wobei die Wellenlänge des Emissionspeaks der Lösung der zweiten Verbindung in Toluol in einem Bereich von 435 nm bis 465 nm liegt.

**3.** Die organische Elektrolumineszenzvorrichtung nach Anspruch 1 oder 2, wobei die Stokes-Verschiebung der zweiten Verbindung in einem Bereich von 4 nm bis 18 nm liegt.

**4.** Die organische Elektrolumineszenzvorrichtung nach einem der Ansprüche 1 bis 3, wobei die Stokes-Verschiebung der zweiten Verbindung in einem Bereich von 5 nm bis 17 nm liegt.

**5.** Die organische Elektrolumineszenzvorrichtung nach einem der Ansprüche 1 bis 4, wobei der molare Absorptionskoeffizient der zweiten Verbindung in einem Bereich von 40000 L/(mol·cm) bis 250000 L/(mol·cm) liegt.

**6.** Die organische Elektrolumineszenzvorrichtung nach einem der Ansprüche 1 bis 5, wobei die zweite Verbindung eine substituierte oder unsubstituierte aromatische Verbindung ist.

**7.** Die organische Elektrolumineszenzvorrichtung nach einem der Ansprüche 1 bis 6, wobei eine Singulett-Energie $S_1(M1)$ der ersten Verbindung und eine Singulett-Energie $S_1(M2)$ der zweiten Verbindung eine Beziehung der numerischen Formel 1 erfüllen,

$$S_1(M1) > S_1(M2) \quad \text{(Numerische Formel 1).}$$

**8.** Die organische Elektrolumineszenzvorrichtung nach einem der Ansprüche 1 bis 7, wobei

die emittierende Schicht ferner eine dritte Verbindung umfasst und die Singulett-Energie $S_1(M1)$ der ersten Verbindung und eine Singulett-Energie $S_1(M3)$ der dritten Verbindung eine Beziehung der numerischen Formel 2 erfüllen,

$$S_1(M3) > S_1(M1) \text{ (Numerische Formel 2).}$$

**9.** Die organische Elektrolumineszenzvorrichtung nach Anspruch 8, wobei eine Energielücke $T_{77K}$ bei 77 [K] der dritten Verbindung größer ist als eine Energielücke $T_{77K}$ bei 77 [K] der zweiten Verbindung.

**10.** Die organische Elektrolumineszenzvorrichtung nach Anspruch 8 oder 9, wobei eine Energielücke $T_{77K}$ bei 77 [K] der dritten Verbindung größer ist als eine Energielücke $T_{77K}$ bei 77 [K] der ersten Verbindung.

**11.** Die organische Elektrolumineszenzvorrichtung nach einem der Ansprüche 8 bis 10, wobei ein Gehaltsverhältnis der ersten Verbindung in der emittierenden Schicht in einem Bereich von 10 Massen-% bis 80 Massen-% liegt.

**12.** Die organische Elektrolumineszenzvorrichtung nach einem der Ansprüche 1 bis 11, wobei eine Energielücke $T_{77K}$ bei 77 [K] der ersten Verbindung größer ist als eine Energielücke $T_{77K}$ bei 77 [K] der zweiten Verbindung.

**13.** Die organische Elektrolumineszenzvorrichtung nach einem der Ansprüche 1 bis 12, ferner umfassend: eine Lochtransportschicht, die zwischen der Anode und der emittierenden Schicht bereitgestellt ist.

**14.** Die organische Elektrolumineszenzvorrichtung nach einem der Ansprüche 1 bis 13, ferner umfassend: eine Elektronentransportschicht, die zwischen der Kathode und der emittierenden Schicht bereitgestellt ist.

**15.** Eine elektronisches Vorrichtung umfassend die organische Elektrolumineszenzvorrichtung nach einem der Ansprüche 1 bis 14.

## Revendications

**1.** Dispositif électroluminescent organique comprenant :

une anode ;
une couche d'émission de lumière ; et
une cathode, dans lequel
la couche d'émission de lumière comprend un premier composé et un deuxième composé, le premier composé est un composé à fluorescence retardée, dans lequel
le premier composé est un composé représenté par une formule,

[formule 26]

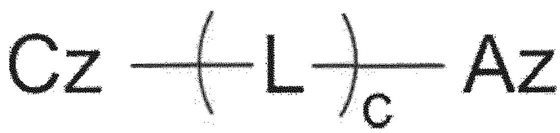

dans laquelle Az est une structure cyclique sélectionnée dans le groupe composé d'un cycle de pyridine substitué ou non substitué, d'un cycle de pyrimidine substitué ou non substitué, d'un cycle triazine substitué ou non substitué et d'un cycle pyrazine substitué ou non substitué ;
c est un nombre entier de 0 à 5 ;
L est un groupe de liaison sélectionné dans le groupe composé d'un groupe hydrocarboné aromatique substitué ou non substitué ayant 6 à 30 atomes de carbone du cycle et d'un groupe hétérocyclique substitué ou non substitué ayant 5 à 30 atomes du cycle ;
si c est 0, Cz est lié à Az par un lien simple ;
si c est un nombre entier de 2 à 5, une pluralité de L sont liés l'une à l'autre pour former un cycle, ou ils ne sont pas liés ;
une puralité de L sont mutuellement identiques ou différents ; et
Cz est représenté par une formule (27),

[formule 27]

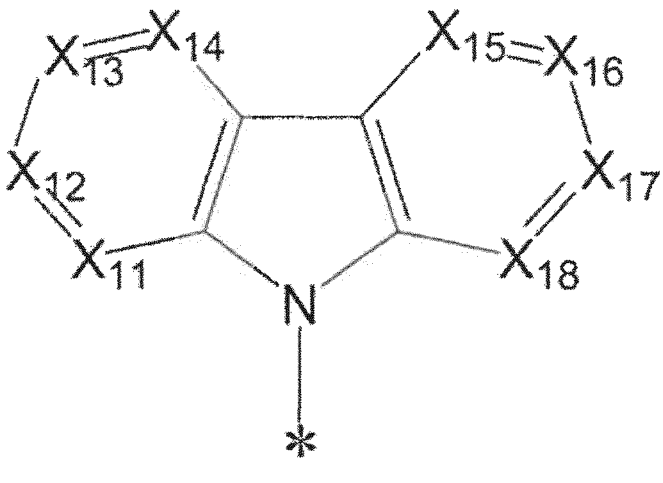

dans laquelle $X_{11}$ à $X_{18}$ sont chacun de manière indépendante un atome d'azote ou C-Rx ;
Rx sont chacun de manière indépendante un atome d'hydrogène ou un substituant ;
Rx servant d'un substituant est un groupe sélectionné dans le groupe composé d'un groupe aryle substitué ou non substitué ayant 6 à 30 atomes de carbone du cycle, d'un groupe hétéroaryle substitué ou non substitué ayant 5 à 30 atomes du cycle, d'un groupe alkyle substitué ou non substitué ayant 1 à 30 atomes de carbone,

d'un groupe fluoroalkyle substitué ou non substitué ayant 1 à 30 atomes de carbone, d'un groupe cycloalkyle substitué ou non substitué ayant 3 à 30 atomes de carbone du cycle, d'un groupe aralkyle substitué ou non substitué ayant 7 à 30 atomes de carbone, d'un groupe phosphoryle substitué,
d'un groupe silyle substitué, d'un groupe cyano, d'un groupe nitro et d'un groupe carboxy ;
une pluralité de Rx sont mutuellement identiques ou différents ;
si une pluralité de quelques-uns de $X_{11}$ à $X_{18}$ sont C-Rx et Rx sont des substituants , les Rx ne sont pas reliés l'un à l'autre pour former un cycle ; et
* représente une position de lien à un atome de carbone dans une structure du groupe de liaison représenté par L, ou une position de lien à un atome de carbone dans la structure cyclique représentée par Az,
le deuxième composé est un composé fluorescent,
le deuxième composé est un composé hydrocarboné, qui consiste en un/des atome(s) de carbone et en un/des atome(s) d'hydrogène, et qui comprend un squelette de mère comprenant des cycles fusionnés, le nombre des cycles fusionnés étant compris entre 5 et 15, et en se prolongeant tout en étant pliés dans une direction, lesdits cycles fusionnés comprennent un cycle à cinq éléments,
une longueur d'onde de pointe émise d'une solution du deuxième composé dans le toluène est comprise dans une gamme entre 430 nm et 480 nm,
un coefficient d'absorption molaire du deuxième composé à un sommet d'absorption le plus proche d'un côté de grande longueur d'onde est dans une gamme comprise entre 40000 L/(mol • cm) et 1000000 L/(mol • cm), et
un déplacement Stokes du deuxième composé est dans une gamme comprise entre 1 nm et 20 nm.

**2.** Dispositif électroluminescent organique selon la revendication 1, dans lequel la longueur d'onde de pointe émise par la solution du deuxième composé dans le toluène est comprise dans une gamme entre 435 nm et 465 nm.

**3.** Dispositif électroluminescent organique selon la revendication 1 ou la revendication 2, dans lequel le déplacement Stokes du deuxième composé est dans une gamme comprise entre 4 nm et 18 nm.

**4.** Dispositif électroluminescent organique selon l'une quelconque des revendications 1 à 3, dans lequel
le déplacement Stokes du deuxième composé est dans une gamme comprise entre 5 nm et 17 nm.

**5.** Dispositif électroluminescent organique selon l'une quelconque des revendications 1 à 4, dans lequel
le coefficient d'absorption molaire du deuxième composé est dans la gamme comprise entre 40000 L/(mol • cm) et 250000 L/(mol • cm).

**6.** Dispositif électroluminescent organique selon l'une quelconque des revendications 1 à 5, dans lequel
le deuxième composé est un composé aromatique substitué ou non substitué.

**7.** Dispositif électroluminescent organique selon l'une quelconque des revendications 1 à 6, dans lequel
une énergie singlet $S_1$(M1) du premier composé et une énergie singlet $S_1$(M2) du deuxième composé satisfont à une relation de la formule numérique 1,

$$S_1(M1) > S_1(M2) \quad \text{(Formule numérique 1).}$$

**8.** Dispositif électroluminescent organique selon l'une quelconque des revendications 1 à 7, dans lequel

la couche d'émission comprend en outre un troisième composé, et
l'énergie singlet $S_1$(M1) du premier composé et une énergie singlet $S_1$(M3) du troisième composé satisfont à une relation de la formule numérique 2,

$$S_1(M3) > S_1(M1) \quad \text{(Formule numérique 2).}$$

**9.** Dispositif électroluminescent organique selon la revendication 8, dans lequel une lacune énergétique $T_{77K}$ à 77 [K] du troisième composé est plus grande qu'une lacune énergétique $T_{77K}$ à 77 [K] du deuxième composé.

**10.** Dispositif électroluminescent organique selon la revendication 8 ou la revendication 9, dans lequel
une lacune énergétique $T_{77K}$ à 77 [K] du troisième composé est plus grande qu'une lacune énergétique $T_{77K}$ à 77 [K] du premier composé.

**11.** Dispositif électroluminescent organique selon l'une quelconque des revendications 8 à 10, dans lequel le taux de teneur en le premier composé dans la couche d'émission est dans une gamme comprise entre 10 % en masse et 80 % en masse.

**12.** Dispositif électroluminescent organique selon l'une quelconque des revendications 1 à 11, dans lequel une lacune énergétique $T_{77K}$ à 77 [K] du premier composé est plus grande qu'une lacune énergétique $T_{77K}$ à 77 [K] du deuxième composé.

**13.** Dispositif électroluminescent organique selon l'une quelconque des revendications 1 à 12, comprenant en outre une couche de transport de lacunes disposée entre l'anode et la couche d'émission.

**14.** Dispositif électroluminescent organique selon l'une quelconque des revendications 1 à 13, comprenant en outre une couche de transport d'électrons disposée entre la cathode et la couche d'émission.

**15.** Dispositif électronique comprenant le dispositif électroluminescent organique selon l'une quelconque des revendications 1 à 14.

FIG.1

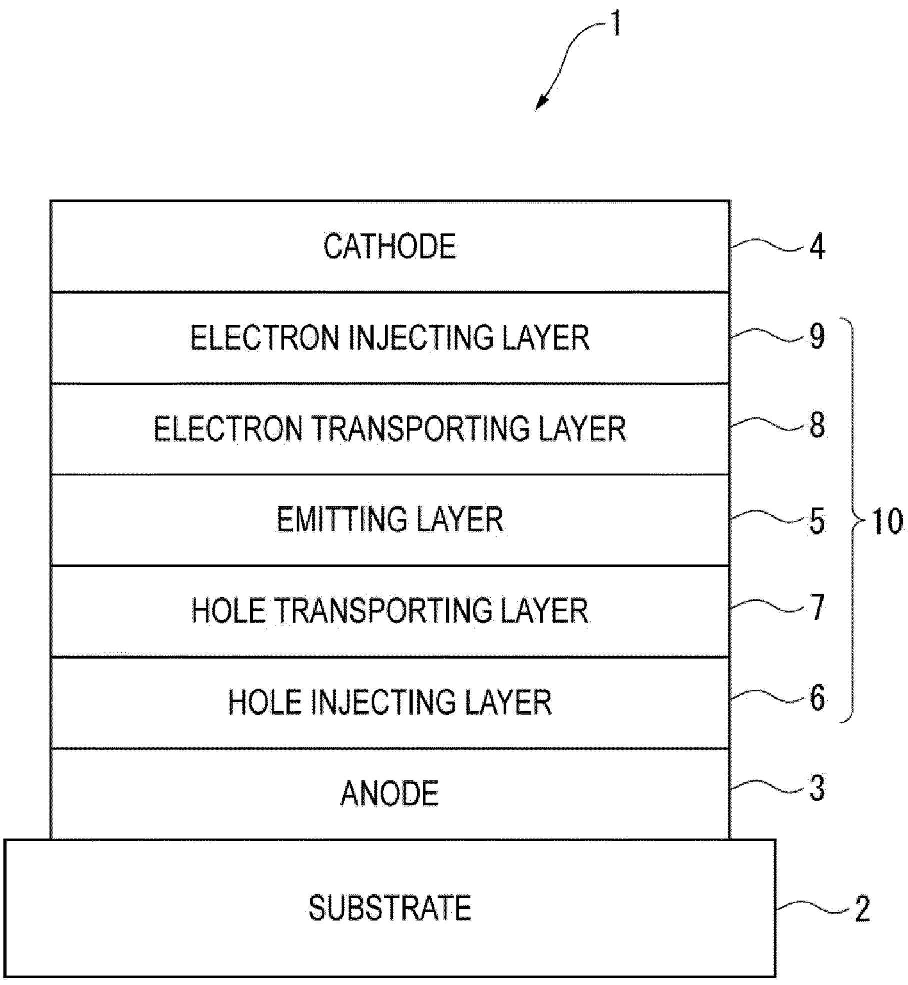
1
CATHODE
4
ELECTRON INJECTING LAYER
9
ELECTRON TRANSPORTING LAYER
8
EMITTING LAYER
5
10
HOLE TRANSPORTING LAYER
7
HOLE INJECTING LAYER
6
ANODE
3
SUBSTRATE
2

FIG.2

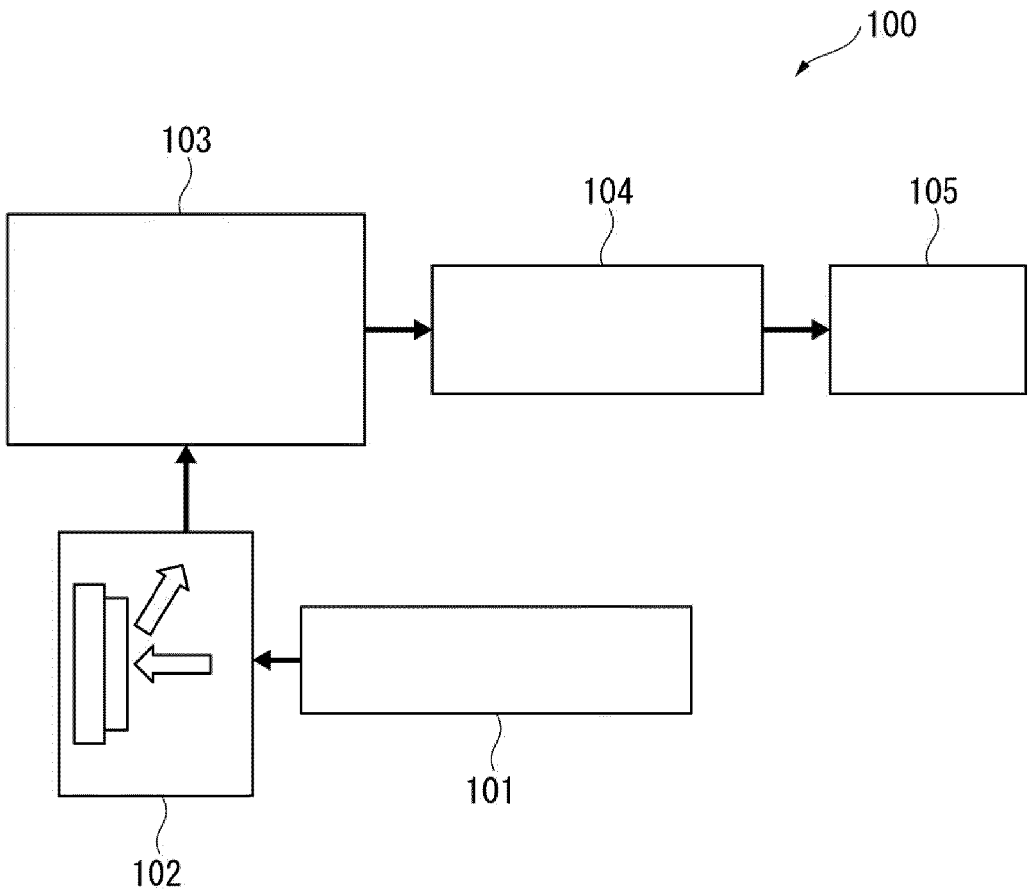

FIG. 3

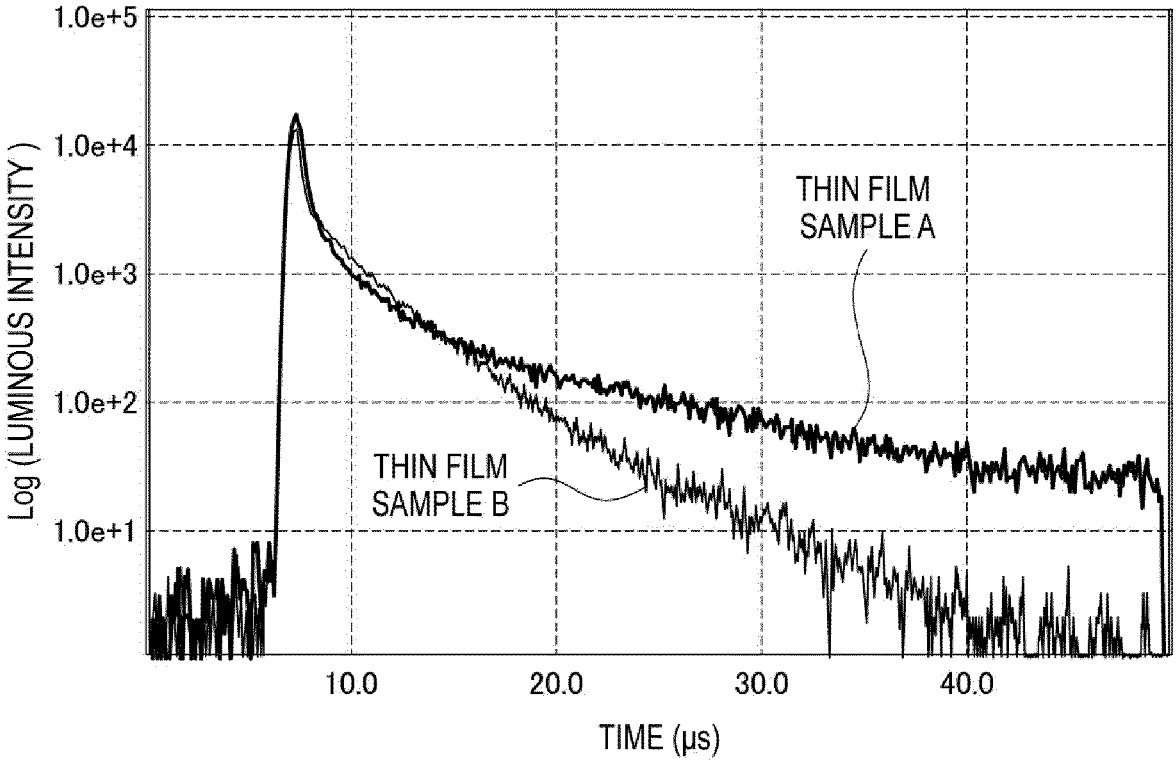
1.0e+5
1.0e+4
1.0e+3
1.0e+2
1.0e+1
Log (LUMINOUS INTENSITY)
THIN FILM SAMPLE A
THIN FILM SAMPLE B
10.0
20.0
30.0
40.0
TIME (μs)

FIG.4

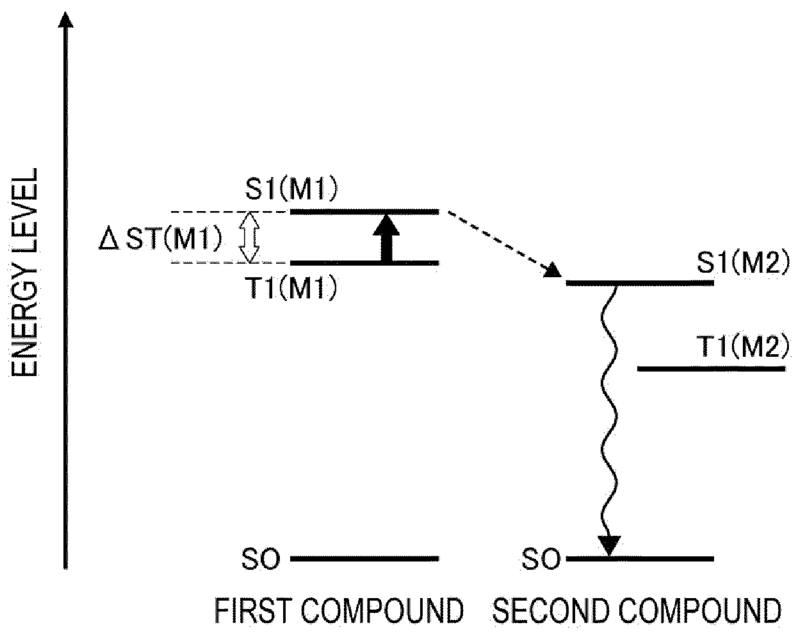
ENERGY LEVEL
S1(M1)
ΔST(M1)
T1(M1)
S1(M2)
T1(M2)
SO
SO
FIRST COMPOUND
SECOND COMPOUND

FIG.5

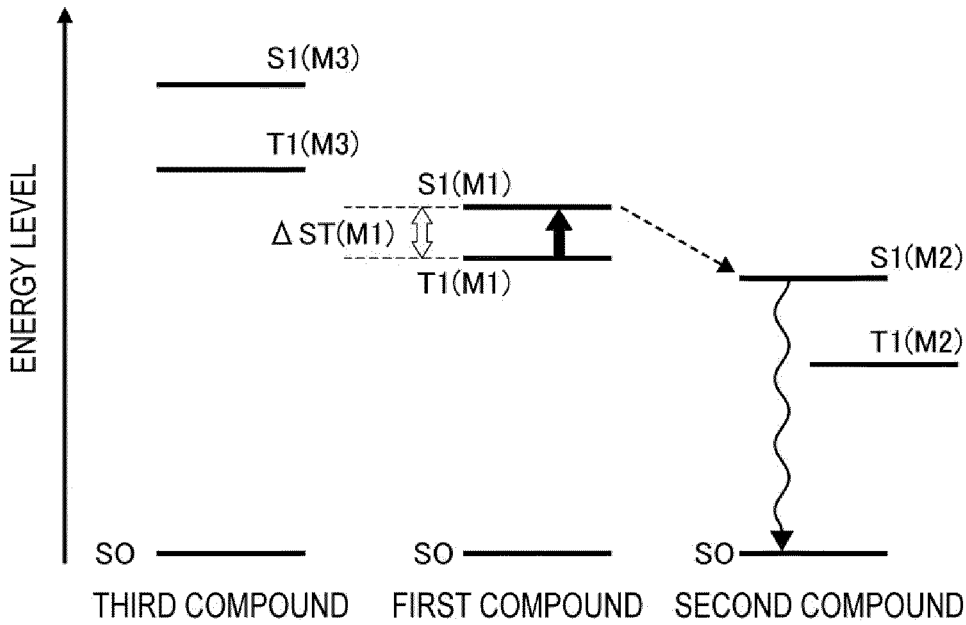
ENERGY LEVEL
S1(M3)
T1(M3)
S1(M1)
ΔST(M1)
T1(M1)
S1(M2)
T1(M2)
SO
SO
SO
THIRD COMPOUND
FIRST COMPOUND
SECOND COMPOUND

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013264550 A1
- EP 2530071 A1
- WO 2013180241 A
- WO 2014092083 A
- WO 2014104346 A
- WO 2012153780 A
- WO 2013038650 A
- JP 2010270103 A
- WO 2011086935 A

### Non-patent literature cited in the description

- Yuki Hando-tai no Debaisu Bussei (Device Physics of Organic Semiconductors). Kodansha, 01 April 2012, 261-268
- **HAJIME NAKANOTANI et al.** High-efficiency organic light-emitting diodes with fluorescent emitters. *NATURE COMMUNICATIONS,* 30 May 2014, vol. 5, 4016
- *Bulletin of the Chemical Society of Japan,* 1978, vol. 1978 (2), 212-216
- Yuki Hando-tai no Debaisu Bussei (Device Physics of Organic Semiconductors). Kodansha, 261-268
- *Nature,* 2012, vol. 492, 234-238
- *Chemical Communications,* 2013, 10385-10387
- *NATURE Photonics,* 2014, 326-332
- *APPLIED PHYSICS LETTERS,* 2012, vol. 101, 093306